# EUROPEAN PATENT APPLICATION

(11) **EP 4 772 506 A1**
(43) Date of publication of application: **08.07.2026**
(21) Application number: 24858655.4
(22) Date of filing: 29.08.2024
(51) Int. Cl.: C07D 405/12, C07D 403/06, C07D 403/14, A61K 31/454, A61K 31/4545, A61K 31/496, A61K 31/501, A61P 15/00

(54) **COMPOUND FOR REGULATING ACTIVITY OF MICRORNA-124**

(30) Priority: 30.08.2023 CN 202311104097
(71) Applicant: Jiangsu Chia Tai Fenghai Pharmaceutical Co., Ltd., Yancheng, Jiangsu 224100 (CN)
(72) Inventor: ZHU, Yongqiang, Yancheng, Jiangsu 224100 (CN); JIANG, Chunhuan, Yancheng, Jiangsu 224100 (CN); TANG, Guqi, Yancheng, Jiangsu 224100 (CN); LI, Chenhui, Yancheng, Jiangsu 224100 (CN); WEN, Tiantian, Yancheng, Jiangsu 224100 (CN); DONG, Zezhang, Yancheng, Jiangsu 224100 (CN); SHI, Jingmiao, Yancheng, Jiangsu 224100 (CN)
(74) Representative: J A Kemp LLP
(86) International application number: PCT/CN2024/115323
(87) International publication number: WO 2025/045123

(57) **Abstract**

Disclosed in the present invention is a compound for regulating the activity of microRNA-124. The compound has a structure as represented by formula (I), and the compound can be used for preventing or treating inflammatory diseases including, but not limited to, inflammatory bowel disease, rheumatoid arthritis, Crohn's disease, ulcerative colitis, multiple sclerosis, Alzheimer's disease, Parkinson's disease, osteoarthritis, atherosclerosis, ankylosing spondylitis, psoriasis, dermatitis, Sjogren's syndrome, bronchitis, asthma, and inflammation associated with colon cancer, and in particular, inflammatory bowel disease, rheumatoid arthritis, Crohn's disease, ulcerative colitis, multiple sclerosis, osteoarthritis, ankylosing spondylitis, psoriasis, Sjogren's syndrome, bronchitis, and inflammation associated with colon cancer.

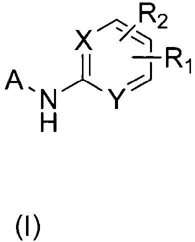

## Description

### Technical Field

The present invention belongs to the field of medicinal chemistry, and relates to a compound for regulating the activity of microRNA-124, and a preparation method thereof, as well as the medical use thereof for treating inflammatory diseases.

### Background Art

RNA interference (RNAi) is a process that effectively silences or suppresses target gene expression, referring to the intracellular mRNA-specific degradation mediated by endogenous or exogenous double-stranded RNA (dsRNA), thereby leading to the silencing of target gene expression and resulting in the corresponding loss of functional phenotype. One of the silencing mechanisms is through inhibition of specific mRNA translation induced by microRNAs.

MicroRNA, also known as "mature microRNA," is small (approximately 18-24 nucleotides in length) non-coding RNA molecules encoded in the genomes of plants and animals. In some cases, highly conserved endogenously expressed microRNAs regulate gene expression by binding to the 3'-untranslated region (3'-UTR) of specific mRNAs. To date, 700 distinct miRNAs have been identified in the human genome, which extensively participate in various physiological or pathological processes such as organismal growth, development, and apoptosis through differential expression patterns. In recent years, it has been discovered that alterations in human miRNA levels are definitively associated with various diseases (such as inflammatory responses, sepsis, ischemia/reperfusion injury, and cancer, among others).

Current research has found that overexpression of a miRNA (namely, miR-124) initiates an anti-inflammatory cascade. miR-124 targets signal transducer and transcription activator 3 (STAT3) to regulate cytokine production in macrophages, reducing the secretion of IL-1b, IL-6, and TNF-a, thereby inhibiting Th17 proliferation. Upregulation of miR-124 in macrophages also reduces MCP1 production, thereby limiting neutrophil recruitment, activating macrophage transdifferentiation from M1 to M2, inactivating inflammatory macrophages, and converting them into microglia-like cells. Abivax's microRNA therapy ABX464, featuring a novel anti-inflammatory mechanism of action, is under development for multiple inflammatory diseases.

Overexpression of microRNA-124 and its anti-inflammatory cascade in inflammatory responses indicate that therapies targeting microRNA-124 can effectively treat and/or prevent inflammatory diseases.

### Summary of the Invention

The present invention provides a compound for regulating the activity of microRNA-124, a pharmaceutical composition, and a preparation method thereof, for medical use in treating inflammatory diseases.

The first aspect of the present invention provides a compound of the following formula (I), or a pharmaceutically acceptable salt thereof, 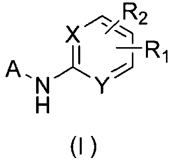
wherein X and Y are independently selected from CH or N;
A is selected from cyano, hydroxyl, carboxyl, optionally substituted C1-C6 alkyl, optionally substituted C2-C6 alkenyl, optionally substituted C2-C6 alkynyl, optionally substituted C1-C6 alkoxy, optionally substituted C3-C6 cycloalkyl, and optionally substituted aromatic ring; the substituents of the optionally substituted C1-C6 alkyl, C2-C6 alkenyl, C2-C6 alkynyl, C1-C6 alkoxy, C3-C6 cycloalkyl, and aromatic ring are independently selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr¹;
HetAr¹ is optionally substituted 4- to 10-membered heterocycle or 5- to 10-membered aromatic ring having 1 to 3 heteroatoms independently selected from N, O, or S. In some embodiments, the substituents of HetAr¹ may be selected from hydrogen, halogen, cyano, hydroxyl, carboxyl, C1-C6 alkyl, C1-C6 haloalkyl, C1-C6 alkoxy, C1-C6 haloalkoxy, C1-C6 alkylthio, C1-C6 haloalkylthio, and C1-C6 hydroxyalkyl;
R₁ and R₂ are independently selected from hydrogen, halogen, hydroxyl, carboxyl, COOR, optionally substituted C1-C6 alkyl, optionally substituted C1-C6 alkoxy, optionally substituted C3-C6 cycloalkyl, and optionally substituted C1-C6 alkylamino; the substituents of the C1-C6 alkyl, C1-C6 alkoxy, C3-C6 cycloalkyl, and C1-C6 alkylamino are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, and C1-C6 haloalkylamino;
alternatively, R₁ and R₂ together with the carbon atom to which they are attached further form optionally substituted saturated 5- to 6-membered heterocycle, wherein the 5- to 6-membered heterocycle optionally contains a heteroatom selected from N and O, and the substituents of the optionally substituted saturated 5- to 6-membered heterocycle are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, and C1-C6 haloalkylamino;
alternatively, R₁ and R₂ together with the carbon atom to which they are attached further form optionally substituted 5- to 6-membered aromatic ring; wherein the substituents are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, and C1-C6 haloalkylamino.

In some other embodiments, the present invention further provides a compound of the following formula (I-1), or a pharmaceutically acceptable salt thereof, 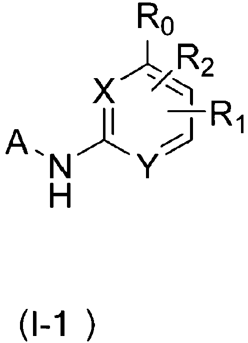
wherein X and Y are independently selected from CH or N;
A is selected from cyano, hydroxyl, carboxyl, optionally substituted C1-C6 alkyl, optionally substituted C2-C6 alkenyl, optionally substituted C2-C6 alkynyl, optionally substituted C1-C6 alkoxy, optionally substituted C3-C6 cycloalkyl, and optionally substituted aromatic ring; the substituents of the optionally substituted C1-C6 alkyl, C2-C6 alkenyl, C2-C6 alkynyl, C1-C6 alkoxy, C3-C6 cycloalkyl, and aromatic ring are independently selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr¹;
HetAr¹ is optionally substituted 4- to 10-membered heterocycle or 5- to 10-membered aromatic ring having 1 to 3 heteroatoms independently selected from N, O, or S; wherein the substituents are selected from hydrogen, halogen, cyano, hydroxyl, carboxyl, C1-C6 alkyl, C1-C6 haloalkyl, C1-C6 alkoxy, C1-C6 haloalkoxy, C1-C6 alkylthio, C1-C6 haloalkylthio, and C1-C6 hydroxyalkyl;
R₀ is H or 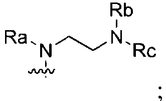
R₁ and R₂ together with the carbon atom to which they are attached further form optionally substituted 5- to 6-membered halogenated aromatic ring; the halogen in the halogenated aromatic ring is independently selected from F, Cl, Br; wherein the substituents are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, C1-C6 haloalkylamino, HetAr⁴, and 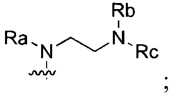
HetAr⁴ is optionally substituted 4- to 10-membered heterocycle or 5- to 10-membered aromatic ring having 1 to 3 heteroatoms independently selected from N, O, or S; wherein the substituents are selected from hydrogen, halogen, cyano, hydroxyl, carboxyl, C1-C6 alkyl, C1-C6 haloalkyl, C1-C6 alkoxy, C1-C6 haloalkoxy, C1-C6 alkylthio, C1-C6 haloalkylthio, and C1-C6 hydroxyalkyl;
Rₐ is selected from hydrogen, halogen, hydroxyl, carboxyl, C1-C6 alkyl, and C1-C6 haloalkyl, and R_{b} and R_{c} are independently selected from hydrogen, halogen, hydroxyl, carboxyl, C1-C6 alkyl, and C1-C6 haloalkyl;
alternatively, R_{b} and R_{c} together with the carbon atom to which they are attached further form optionally substituted saturated 5- to 6-membered heterocycle, wherein the 5- to 6-membered heterocycle optionally contains a heteroatom selected from N and O, and the substituents of the optionally substituted saturated 5- to 6-membered heterocycle are independently selected from hydrogen, halogen, hydroxyl, carboxyl, COOR, - C1-C6 alkyl, C1-C6 haloalkyl, C1-C6 alkoxy, C1-C6 haloalkoxy, C1-C6 alkylamino, and C1-C6 haloalkylamino.

The compound according to the present invention, wherein the following compounds **a-x** are excluded:

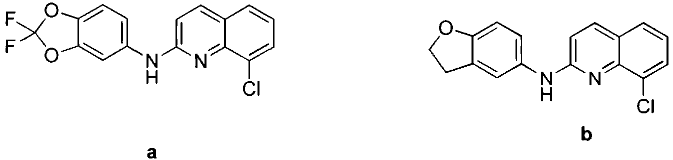

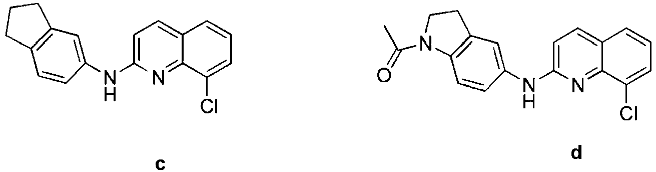

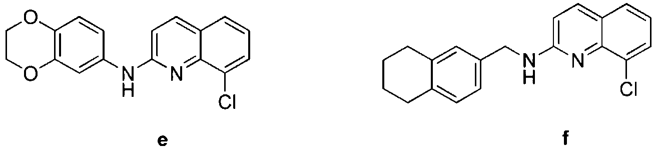

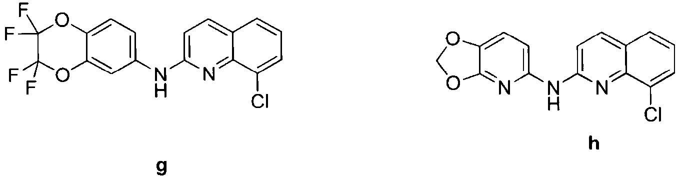

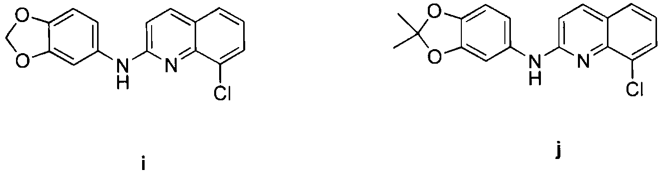

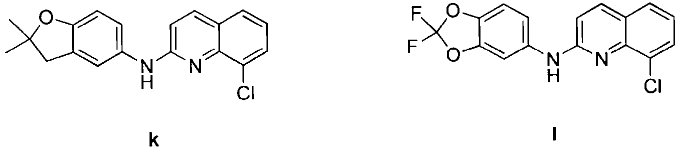

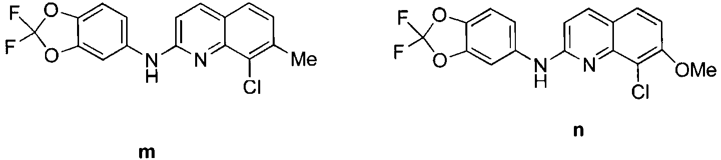

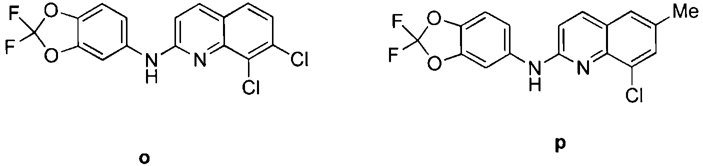

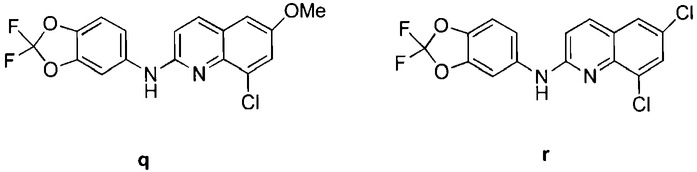

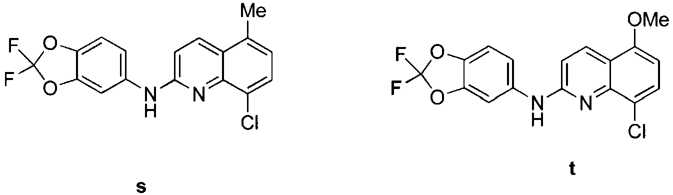

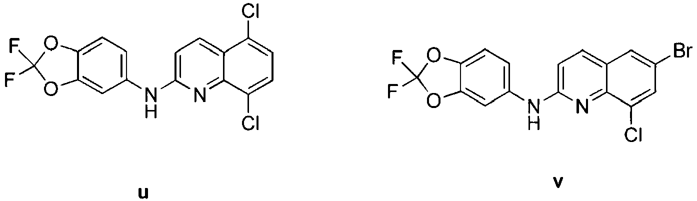

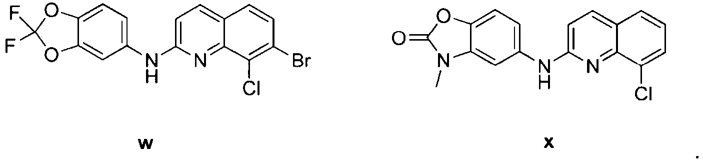

Further, in some embodiments of the present invention, in the compound of formula (I), X is CH; Y is N; A is C2-C6 alkenyl; R1 and R2 together with the carbon atom to which they are attached further form optionally substituted 5- to 6-membered aromatic ring, and are connected to a adjacent benzene ring to form a fused ring; wherein the substituent is halogen; further, R1 and R2 together with the carbon atom to which they are attached form a benzene ring, and are connected to a adjacent benzene ring to form a fused ring; the substituent is halogen.

The present invention further provides a compound of following formula (II) or a pharmaceutically acceptable salt thereof, 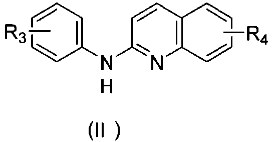
R₃ is selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr²; wherein R is C1-C6 alkyl;
HetAr² is substituted or unsubstituted 4- to 6-membered monocyclic heterocycle having 1 to 3 heteroatoms independently selected from N, O, or S; and is connected to a adjacent benzene ring to form a fused ring; wherein the substituents are selected from hydrogen, halogen, cyano, hydroxyl, carboxyl, C1-C6 alkyl, C1-C6 haloalkyl, C1-C6 alkoxy, C1-C6 haloalkoxy, C1-C6 alkylthio, C1-C6 haloalkylthio, and C1-C6 hydroxyalkyl;
R₄ is selected from hydrogen, halogen, cyano, hydroxyl, carboxyl, COOR, C1-C6 alkyl, C1-C6 haloalkyl, C1-C6 alkoxy, and C1-C6 haloalkoxy; wherein R is C1-C6 alkyl;

The following compounds **A-U** are excluded:

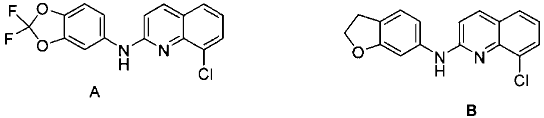

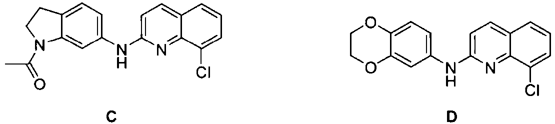

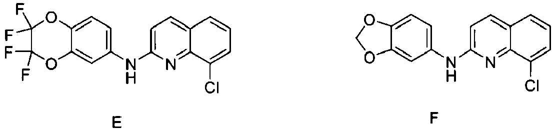

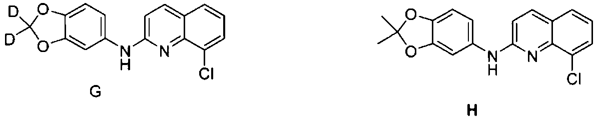

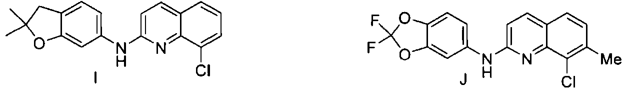

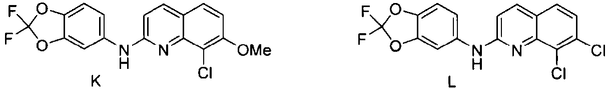

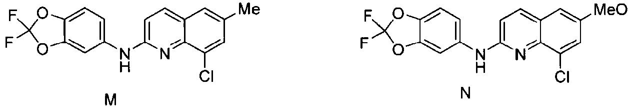

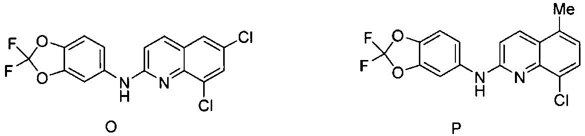

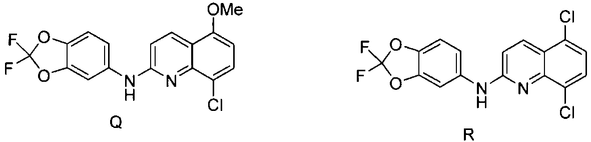

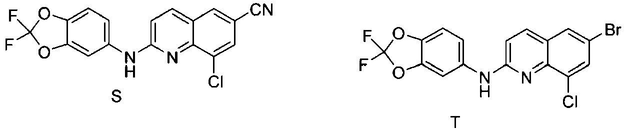

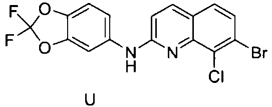

Further, in some embodiments of the present invention, in the compound of formula (II), R₃ is selected from HetAr²; HetAr² is substituted or unsubstituted 4- to 6-membered monocyclic aromatic heterocycle having 1 to 3 heteroatoms independently selected from N, O, or S; and is connected to a adjacent benzene ring to form a fused ring; wherein substituents are selected from hydrogen, halogen, cyano, hydroxyl, carboxyl, C1-C6 alkyl, C1-C6 haloalkyl, C1-C6 alkoxy, C1-C6 haloalkoxy, C1-C6 alkylthio, C1-C6 haloalkylthio, and C1-C6 hydroxyalkyl; further substituents are selected from hydrogen, halogen, cyano, hydroxyl, carboxyl, C1-C3 alkyl, C1-C3 haloalkyl, C1-C3 alkoxy, C1-C3 haloalkoxy, C1-C3 alkylthio, C1-C3 haloalkylthio, and C1-C3 hydroxyalkyl; wherein R₄ is halogen.

Further, in some other embodiments of the present invention, in the compound of formula (II), R₃ is selected from HetAr²; HetAr² is substituted or unsubstituted 4- to 6-membered monocyclic saturated heterocycle containing one O; and is connected to a adjacent benzene ring to form a fused ring; wherein substituents are selected from hydrogen, halogen, cyano, hydroxyl, and carboxyl; wherein R₄ is halogen.

The present invention further provides a compound of following formula (III) or a pharmaceutically acceptable salt 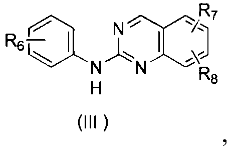
R₆ is selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr³; wherein R is C1-C6 alkyl;
HetAr³ is substituted or unsubstituted 4- to 6-membered monocyclic heterocycle having 1 to 3 heteroatoms independently selected from N, O, or S; and is connected to a adjacent benzene ring to form a fused ring; wherein the substituents are selected from hydrogen, halogen, hydroxyl, and carboxyl;
R₇ and R₈ are independently selected from hydrogen, halogen, hydroxyl, carboxyl, COOR, C1-C6 alkyl, C1-C6 haloalkyl, C1-C6 alkoxy, and C1-C6 haloalkoxy; wherein R is C1-C6 alkyl.

Further in the present invention, in the compound of formula (III),
R₆ in some embodiments is selected from C1-C6 haloalkoxy or C1-C6 haloalkylthio; R₇ and R₈ are independently selected from hydrogen, halogen, C1-C6 haloalkyl, C1-C6 alkoxy; in some more specific embodiments , R₆ is selected from C1-C3 haloalkoxy or C1-C3 haloalkylthio; R₇ and R₈ are independently selected from hydrogen, halogen, C1-C3 haloalkyl, C1-C3 alkoxy; in some embodiments, R₇ and R₈ are not both hydrogen.

Further in the present invention, in the compound of formula (III), R₆ in some other embodiments is selected from HetAr¹, which is substituted or unsubstituted 4- to 6-membered monocyclic heterocycle having 1 to 3 heteroatoms independently selected from N, O, or S; and is connected to a adjacent benzene ring to form a fused ring; wherein the substituents are selected from hydrogen and halogen; R₇ and R₈ are independently selected from hydrogen and halogen; in some embodiments, R₇ and R₈ are not both hydrogen.

The present invention further provides a compound of following formula (III-1) or a pharmaceutically acceptable salt, 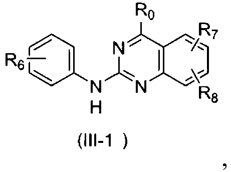
R₆ is selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr³; wherein R is C1-C6 alkyl;
HetAr³ is substituted or unsubstituted 4- to 6-membered monocyclic heterocycle having 1 to 3 heteroatoms independently selected from N, O, or S; and is connected to a adjacent benzene ring to form a fused ring; wherein the substituents are selected from hydrogen, halogen, hydroxyl, and carboxyl;
R₀ is H or 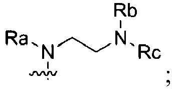 R₇ and R₈ are independently selected from halogen, C1-C6 haloalkylamino, HetAr⁴, and 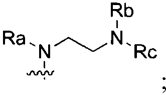 HetAr⁴ is optionally substituted 4- to 10-membered heterocycle or 5- to 10-membered aromatic ring having 1 to 3 heteroatoms independently selected from N, O, or S; Rₐ is selected from hydrogen, halogen, hydroxyl, carboxyl, C1-C6 alkyl, and C1-C6 haloalkyl; R_{b} and R_{c} are independently selected from hydrogen, halogen, hydroxyl, carboxyl, C1-C6 alkyl, and C1-C6 haloalkyl; or R_{b} and R_{c} together with the nitrogen atom to which they are attached further form optionally substituted saturated 5- to 6-membered heterocycle, wherein the 5- to 6-membered heterocycle optionally contains a heteroatom selected from N and O, and the substituents of the optionally substituted saturated 5- to 6-membered heterocycle are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, and C1-C6 haloalkylamino.

In some embodiments, R₆ is selected from C1-C6 haloalkoxy or C1-C6 haloalkylthio; preferably, R₆ is selected from C1-C3 haloalkoxy or C1-C3 haloalkylthio; more preferably, R₆ is trifluoromethyloxy.

In some embodiments, R₇ is selected from F, Cl or Br.

In some embodiments, R₀ is H.

In some embodiments, R₇ is halogen; R₈ is selected from HetAr⁴ or 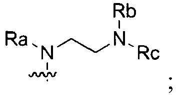 preferably, HetAr⁴ is optionally substituted 4- to 10-membered heterocycle having 1 to 3 heteroatoms independently selected from N, O, or S, and preferred substituents of HetAr⁴ are selected from hydrogen, halogen, hydroxyl, carboxyl, methyl, ethyl, propyl, butyl, and C1-C3 haloalkyl.

In some embodiments, Rₐ is selected from hydrogen, halogen, hydroxyl, carboxyl, methyl, ethyl, propyl, and butyl; R_{b} and R_{c} are independently selected from hydrogen, halogen, hydroxyl, carboxyl, methyl, ethyl, propyl, and butyl; or R_{b} and R_{c} together with the nitrogen atom to which they are attached further form optionally substituted saturated 5- to 6-membered heterocycle containing N and O, wherein the substituents of the 5- to 6-membered heterocycle are independently selected from hydrogen, halogen, hydroxyl, carboxyl, C1-C6 alkyl, C1-C6 haloalkyl, C1-C6 alkoxy, C1-C6 haloalkoxy, C1-C6 alkylamino, and C1-C6 haloalkylamino; preferably, R_{b} and R_{c} are independently selected from methyl, ethyl, propyl, and butyl; or R_{b} and R_{c} together with the nitrogen atom to which they are attached further form saturated 5- to 6-membered heterocycle containing N and O.

The present invention also provides the following compounds **1-7** and **9-51,** or pharmaceutically acceptable salts thereof:

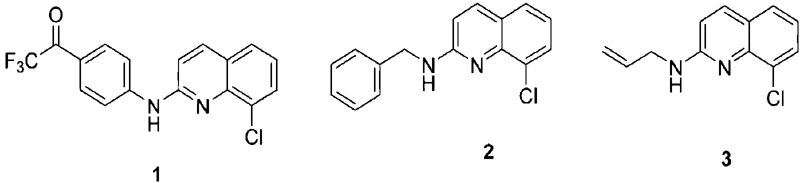

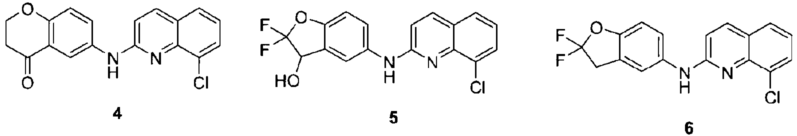

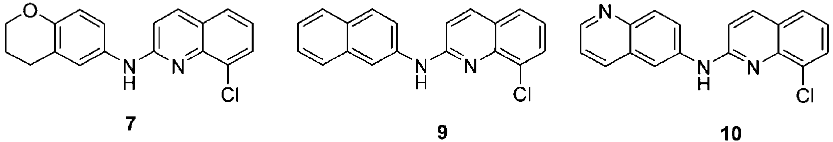

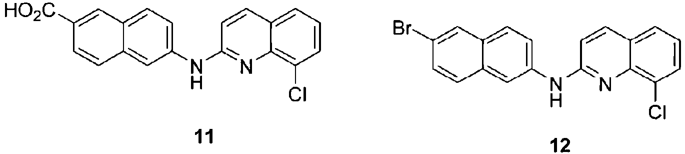

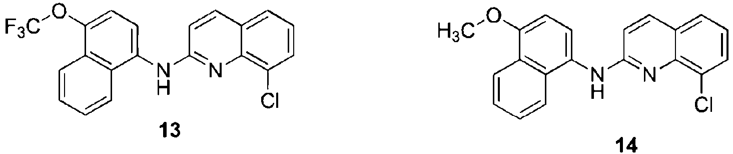

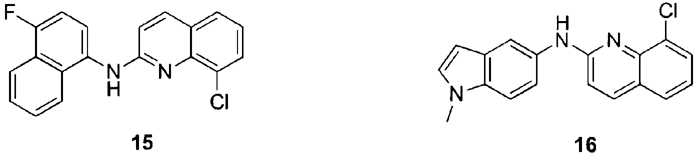

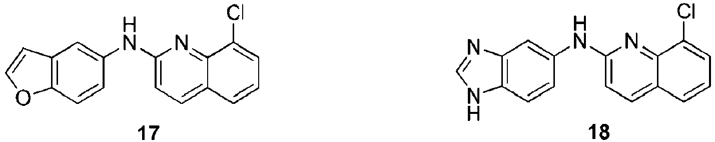

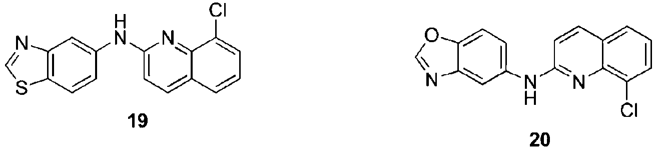

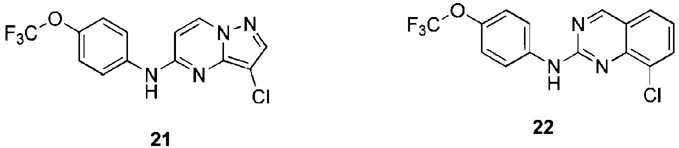

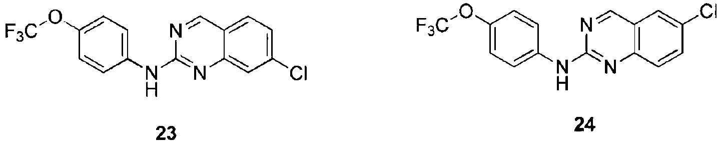

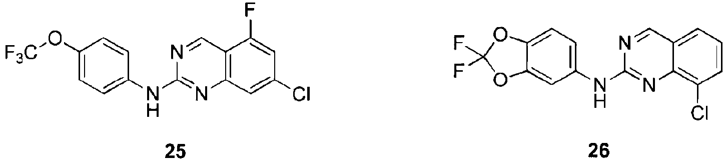

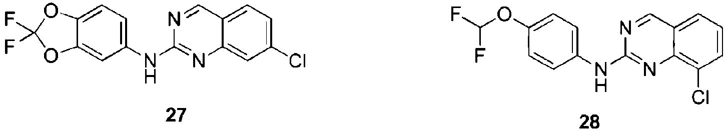

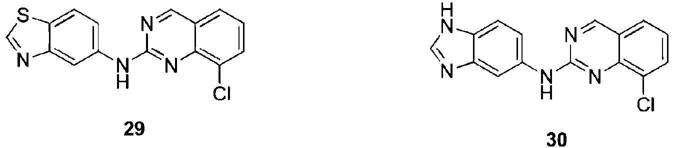

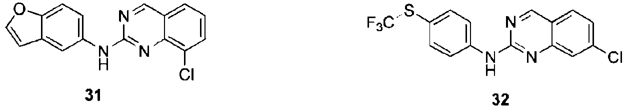

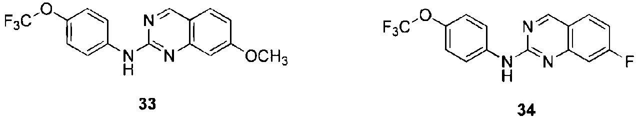

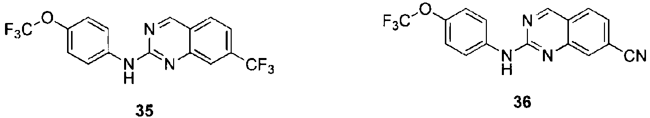

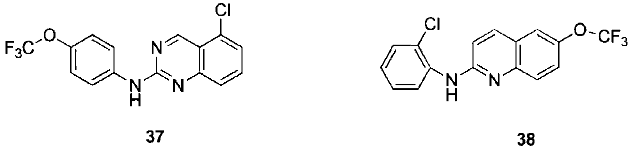

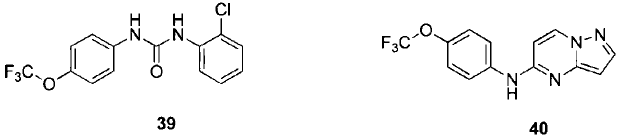

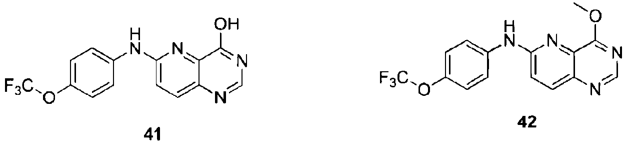

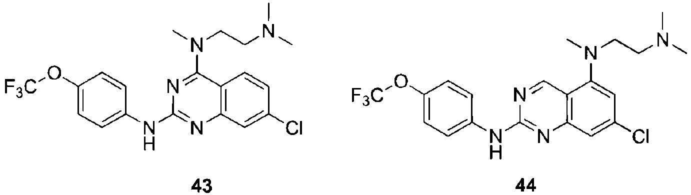

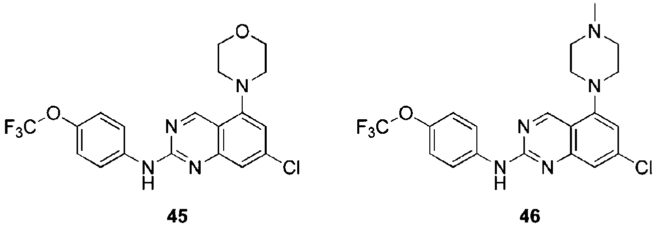

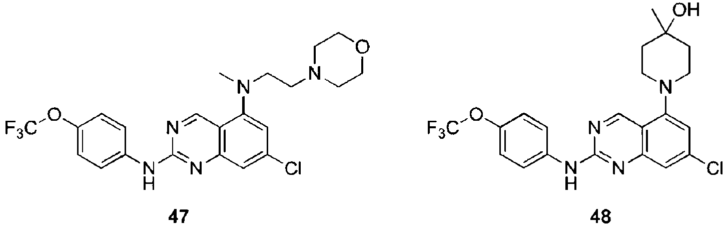

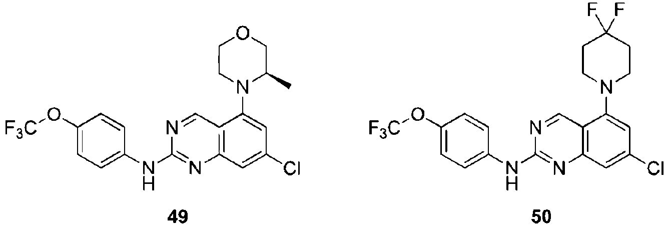

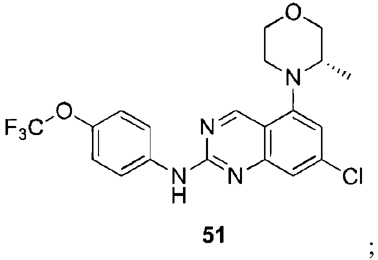

Another aspect of the present invention relates to a method for preparing a compound of formula (I) or a pharmaceutically acceptable salt thereof, the method comprising:

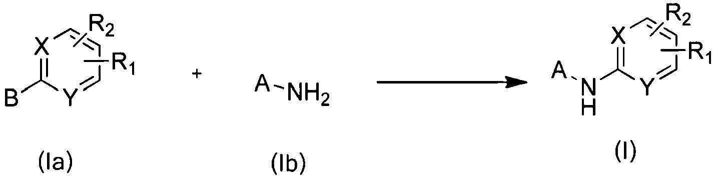

The compound of formula (Ia) or a pharmaceutically acceptable salt thereof and the compound of formula (Ib) or a pharmaceutically acceptable salt thereof undergo nucleophilic substitution to yield the compound of formula (I) or a pharmaceutically acceptable salt thereof;
wherein B is halogen; preferably a chlorine atom; X, Y, R₁, and R₂ are as defined in formula (I).

Another aspect of the present invention relates to a method for preparing a compound of formula (II) or a pharmaceutically acceptable salt thereof, the method comprising:

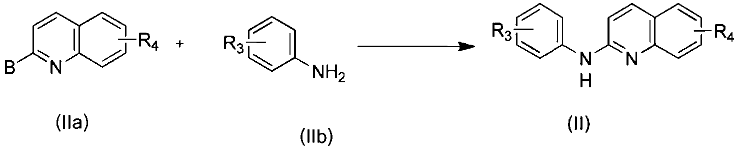

The compound of formula (IIa) or a pharmaceutically acceptable salt thereof and the compound of formula (IIb) or a pharmaceutically acceptable salt thereof undergo nucleophilic substitution to yield the compound of formula (II) or a pharmaceutically acceptable salt thereof;
wherein B is halogen; preferably a chlorine atom; R₃, and R₄ are as defined in formula (II).

Another aspect of the present invention relates to a method for preparing a compound of formula (III) or a pharmaceutically acceptable salt thereof, the method comprising:

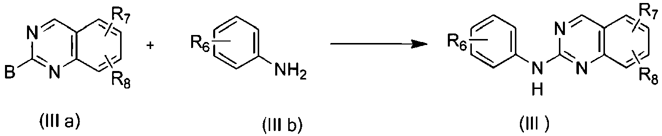

The compound of formula (IIIa) or a pharmaceutically acceptable salt thereof and the compound of formula (IIIb) or a pharmaceutically acceptable salt thereof undergo nucleophilic substitution to yield the compound of formula (III) or a pharmaceutically acceptable salt thereof;
wherein B is halogen; preferably a chlorine atom; R₆, R₇, and R₈ are as defined in formula (III).

The present invention further provides a pharmaceutical composition comprising a compound of the present invention or a pharmaceutically acceptable salt thereof and one or more pharmaceutically acceptable carriers.

The present invention also provides the use of the compound of the present invention or a pharmaceutically acceptable salt thereof as a microRNA-124 regulator.

The present invention also provides the compound of the present invention or a pharmaceutically acceptable salt thereof, for use in preventing or treating inflammatory diseases.

The inflammatory diseases described in the present invention include, but are not limited to, inflammatory bowel disease, rheumatoid arthritis, Crohn's disease, ulcerative colitis, multiple sclerosis, Alzheimer's disease, Parkinson's disease, osteoarthritis, atherosclerosis, ankylosing spondylitis, psoriasis, dermatitis, Sjogren's syndrome, bronchitis, asthma, and inflammation associated with colon cancer, and in particular, inflammatory bowel disease, rheumatoid arthritis, Crohn's disease, ulcerative colitis, multiple sclerosis, osteoarthritis, ankylosing spondylitis, psoriasis, Sjogren's syndrome, bronchitis, and inflammation associated with colon cancer.

The present invention also encompasses methods for synthesizing the compounds of the present invention or pharmaceutically acceptable salts thereof.

In the present application, the term "pharmaceutically acceptable salt" includes pharmaceutically acceptable acid addition salts and pharmaceutically acceptable base addition salts.

The term "alkyl," used alone or as part of a larger group such as "alkoxy" or "alkylamino" and similar terms, means a saturated aliphatic straight or branched monovalent hydrocarbon radical. Unless otherwise specified, alkyl groups generally have 1-6 carbon atoms, i.e., (C1-C6)alkyl. As used herein, (C1-C6)alkyl means a radical having 1 to 6 carbon atoms arranged linearly or in a branched manner. Examples include methyl, ethyl, n-propyl, isopropyl, and the like.

"Alkenyl" means a branched or straight-chain monovalent hydrocarbon radical containing at least one double bond. The alkenyl group may be mono- or polyunsaturated and may exist in E or Z configuration. Unless otherwise specified, the alkenyl group generally has 2-6 carbon atoms, i.e., (C2-C6)alkenyl. For example, "(C2-C6)alkenyl" means a radical having 2-6 carbon atoms arranged linearly or in a branched manner. "Alkynyl" means a branched or straight-chain monovalent hydrocarbon radical containing at least one triple bond. Unless otherwise specified, the alkynyl group generally has 2-6 carbon atoms, i.e., (C2-C6)alkynyl. For example, (C2-C6)alkynyl means a radical having 2 to 6 carbon atoms arranged linearly or in a branched manner.

"Alkoxy" refers to an alkyl radical attached via an oxygen linking atom, represented by -O-alkyl. For example, "(C1-C6)alkoxy" includes methoxy, ethoxy, propoxy, and butoxy.

"Alkylthio" refers to an alkyl radical attached via a sulfur linking atom, represented by -S-alkyl. For example, "(C1-C6)alkylthio" includes methylthio, ethylthio, propylthio, and butylthio.

"Cycloalkyl" refers to a saturated aliphatic cyclic hydrocarbon radical, generally containing 3-6 ring carbon atoms, i.e., (C3-C6)cycloalkyl. (C3-C6)cycloalkyl includes, but is not limited to, cyclopropyl, cyclobutyl, cyclopentyl, and cyclohexyl.

The term "alkylamino" means an alkyl or alkoxy group attached with one or more amino groups as appropriate. Represented by -N-alkyl, -NH-alkyl, or NH2-alkyl. For example, "(C1-C6)alkylamino" includes methoxy, ethoxy, propoxy, and butoxy.

"Monocyclic heterocycle," when used alone or as part of a larger group, means a saturated or unsaturated 4- to 7-membered ring radical, optionally containing one or more double bonds, generally having 4-7 ring atoms selected from carbon and at least one (generally 1 to 4, more generally 1 or 2) heteroatom(s) (e.g., oxygen, nitrogen). "Substituted monocyclic heterocycle" is substituted at any one or more substitutable ring atoms (ring carbon atoms bonded to hydrogen). "Monocyclic saturated heterocycle" means a saturated 4- to 7-membered ring radical containing no double bonds, generally having 4-7 ring atoms selected from carbon and at least one (generally 1 to 4, more generally 1 or 2) heteroatom(s) (e.g., oxygen, nitrogen). The term "monocyclic heterocyclyl" is intended to include all possible isomeric forms.

"Monocyclic aromatic heterocycle," when used alone or as part of a larger group, means an unsaturated 4- to 6-membered ring radical, generally means an aromatic ring group having 4-6 ring atoms selected from carbon and at least one (generally 1 to 4, more generally 1 or 2) heteroatom(s) (e.g., oxygen, nitrogen). Examples of monocyclic 4- to 6-membered heteroaryl include furanyl (e.g., 2-furanyl, 3-furanyl), imidazolyl (e.g., N-imidazolyl, 2-imidazolyl, 4-imidazolyl, 5-imidazolyl), isoxazolyl (e.g., 3-isoxazolyl, 4-isoxazolyl, 5-isoxazolyl), oxadiazolyl (e.g., 2-oxadiazolyl, 5-oxadiazolyl), oxazolyl (e.g., 2-oxazolyl, 4-oxazolyl, 5-oxazolyl), pyrazolyl (e.g., 3-pyrazolyl, 4-pyrazolyl), pyrrolyl (e.g., 1-pyrrolyl, 2-pyrrolyl, 3-pyrrolyl), pyridinyl (e.g., 2-pyridinyl, 3-pyridinyl, 4-pyridinyl), pyrimidinyl (e.g., 2-pyrimidinyl, 4-pyrimidinyl, 5-pyrimidinyl), pyridazinyl (e.g., 3-pyridazinyl), thiazolyl (e.g., 2-thiazolyl, 4-thiazolyl, 5-thiazolyl), isothiazolyl, triazolyl (e.g., 2-triazolyl, 5-triazolyl), tetrazolyl (e.g., tetrazolyl), and thienyl (e.g., 2-thienyl, 3-thienyl).

"Fused ring" means a bicyclic system consisting of two adjacent rings sharing two common atoms.

Certain compounds described herein may exist in various stereoisomeric or tautomeric forms. Stereoisomers are compounds that differ only in their spatial arrangement. When a compound of the present disclosure is named or depicted by structure without indicating stereochemistry, it is understood that the name or structure encompasses all possible stereoisomers, geometric isomers, including substantially pure stereoisomers or geometric isomers and combinations thereof.

### Detailed Description of Embodiments

The present invention is further illustrated below in conjunction with specific examples, based on the general technical knowledge and conventional means in the art. The following examples are merely some preferred examples of the present invention and should not be construed as limiting the invention. For those skilled in the art, several modifications can be made without departing from the scope of the present invention, and these modifications should also be regarded as falling within the protection scope of the present invention.

### Example 1: Synthesis of Compound 1

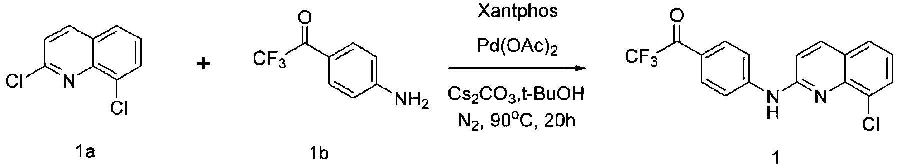

A 25 mL round-bottom flask was charged with nitrogen, and compound **1a** (198 mg, 1.0 mmol, Bide Pharmatech), compound **1b** (189 mg, 1.0 mmol, Bide Pharmatech), Xantphos (29 mg, 0.05 mmol), Pd(OAc)₂ (8.5 mg, 0.05 mmol), cesium carbonate (978 mg, 3.0 mmol), and tert-butanol (10 mL) were sequentially added. The mixture was heated to 100°C and reacted for 20 h. The reaction was stopped, and the mixture was evaporated under reduced pressure. Water (40 mL) was added, and the mixture was extracted with ethyl acetate (3 × 25 mL), washed with saturated brine, and dried over anhydrous sodium sulfate. The organic phases were combined, concentrated and purified by column chromatography (PE/EA = 100:1-10:1, v/v) to afford compound **1** (200 mg).

¹H NMR (400 MHz, DMSO-d6) δ 10.51 (s, 1H), 8.48 - 8.41 (m, 2H), 8.29 (d, J = 8.9 Hz, 1H), 8.09 (d, J = 8.6 Hz, 2H), 7.87 (dd, J = 7.6, 1.4 Hz, 1H), 7.83 (dd, J = 8.1, 1.3 Hz, 1H), 7.39 (t, J = 7.8 Hz, 1H), 7.28 (d, J = 8.9 Hz, 1H).

ESI-MS:m/z 351[M+H]⁺ .

### Example 2: Synthesis of Compound 2

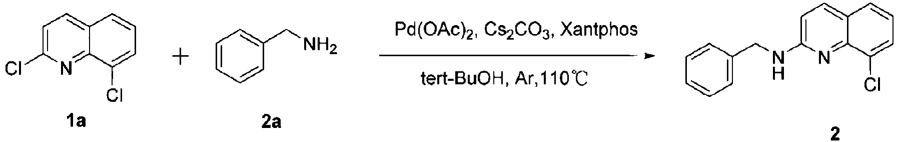

To a 75 mL sealed tube were added compound **1a** (800 mg, 1.0 eq, Bide Pharmatech), compound **2a** (519 mg, 1.2 eq, Energy Chemical), Pd(OAc)₂ (91 mg, 0.1 eq), cesium carbonate (2.6 g, 2.0 eq), and Xantphos (701 mg, 0.3 eq). After adding tert-butanol (10 mL) and mixing thoroughly, the mixture was heated and stirred at 110°C in an oil bath for 20 h under argon protection. The reaction was stopped, methanol (20 mL) was added, the mixture was filtered by suction, and the filter cake was washed with methanol. The filtrate was concentrated and purified by column chromatography (PE:EA = 100:1-4:1, v/v) to afford compound **2** (230 mg).

¹H NMR (400 MHz, DMSO-d6) δ 7.92 (d, J = 9.0 Hz, 1H), 7.86 (t, J = 5.9 Hz, 1H), 7.65 - 7.57 (m, 2H), 7.52 - 7.42 (m, 2H), 7.36 - 7.28 (m, 2H), 7.24 (d, J = 7.3 Hz, 1H), 7.11 (t, J = 7.8 Hz, 1H), 6.88 (d, J = 8.9 Hz, 1H), 4.68 (d, J = 5.9 Hz, 2H) .

ESI-MS:m/z 269.1 [M+H]⁺.

### Example 3: Synthesis of Compound 3

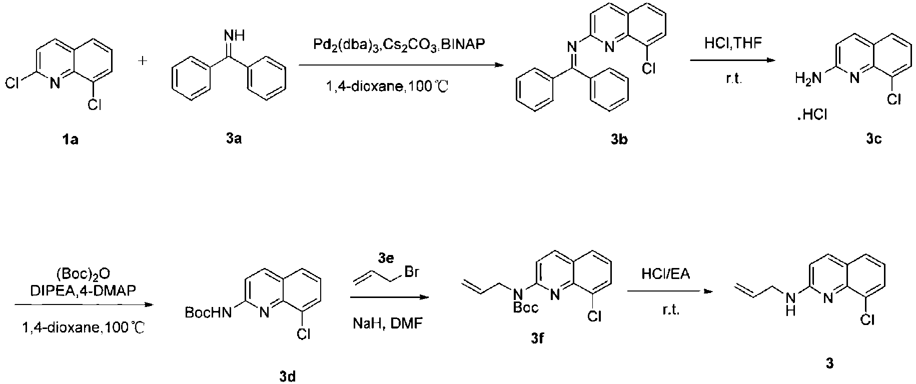

The preparation method of the above-mentioned compound **3b** was as follows:
In a sealed tube, compound **1a** (2 g, 1.0 eq, Bide Pharmatech), compound **3a** (3.67 g, 2.0 eq, Bide Pharmatech), Pd₂(dba)₃ (925 mg, 0.1 eq), BINAP (1.26 g, 0.2 eq), Cs₂CO₃ (9.87 g, 3.0 eq) were added, followed by the addition of 1,4-dioxane (20 mL). The mixture was heated and stirred at 100°C. Upon completion of the reaction, water (100 mL) was added, and the mixture was extracted with ethyl acetate (3 × 50 mL). The organic phases were combined, dried over anhydrous sodium sulfate, concentrated under reduced pressure, and purified by column chromatography (PE/EA = 100:1-3:1, v/v) to afford crude product **3b** (4 g).

The preparation method of the above compound **3c** was as follows:
The crude compound **3b** (4 g) obtained from Preparation Example 1 was dissolved in tetrahydrofuran (10 mL) in a single-necked flask, and concentrated hydrochloric acid was added dropwise, resulting in the precipitation of a white solid at the bottom of the flask. The mixture was filtered by suction, and the filter cake was dried to afford compound **3c** (2 g).

The preparation method of the above compound **3d** was as follows:
In a sealed tube, compound **3c** (1.75 g, 1.0 eq), (Boc)₂O (3.72 g, 2.1 eq, Energy Chemical), DIPEA (3.15 g, 3.0 eq), and 4-DMAP (994 mg, 1.0 eq) were added, followed by the addition of 1,4-dioxane (20 mL). The mixture was heated and stirred at 100°C overnight. Upon completion of the reaction, (100 mL) was added, and the mixture was extracted with ethyl acetate (3 × 50 mL). The organic phases were combined, dried over anhydrous sodium sulfate, concentrated under reduced pressure, and purified by column chromatography (PE/EA = 100:1-3:1, v/v) to afford compound **3d** (740 mg).

The preparation method of the above compound **3f** was as follows:
To a single-necked flask was added compound **3d** (740 mg, 1.0 eq), followed by dissolution in 10 mL DMF, then NaH (159 mg, 1.5 eq) was added, and the mixture was stirred at room temperature for 0.5 h, after which compound **3e** (353 mg, 1.1 eq, Energy Chemical) was added, and stirring was performed at room temperature. Upon completion of the reaction, water (20 mL) was added, and the mixture was extracted with ethyl acetate (3 × 15 mL). The organic phases were combined, dried over anhydrous sodium sulfate and concentrated under reduced pressure to afford compound **3f** (400 mg), which was used directly in the next step without purification.

Synthesis of the above compound **3:**
compound **3f** was added to a single-necked flask, followed by addition of HCl/EA (2 mL, 10 eq), and the mixture was stirred at room temperature overnight. After completion of the reaction, the reaction liquid was evaporated to dryness and purified by column chromatography to afford compound **3** (220 mg).

¹H NMR (400 MHz, DMSO-*d*₆) δ 7.92 (d, *J =* 8.9 Hz, 1H), 7.62 (ddd, *J =* 12.8, 7.8, 1.5 Hz, 2H), 7.48 (t, *J* = 5.6 Hz, 1H), 7.11 (t, *J =* 7.7 Hz, 1H), 6.87 (d, *J* = 8.9 Hz, 1H), 6.02 (ddt, *J* = 17.3, 10.6, 5.5 Hz, 1H), 5.29 (dq, *J* = 17.2, 1.8 Hz, 1H), 5.12 (dq, *J* = 10.2, 1.6 Hz, 1H), 4.13 (tt, *J* = 5.6, 1.6 Hz, 2H).

ESI-MS:m/z 219.1 [M+H]⁺.

### Example 4: Synthesis of Compound 4

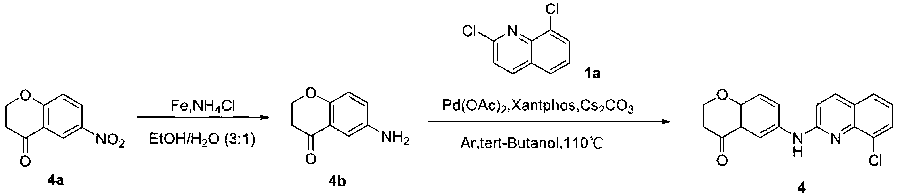

The preparation method of the above compound **3f** was as follows:
To a single-necked flask were added compound **4a** (2 g, 1.0 eq, Bide Pharmatech), reduced iron powder (1.73 g, 3.0 eq), ammonium chloride (498 mg, 0.9 eq), and a mixed solvent (10 mL, ethanol/water = 3:1, v/v). The mixture was heated to reflux at 80°C for approximately 12 h. After completion of the reaction, methanol was added to dissolve the mixture, which was then filtered by suction. The filter cake was washed with methanol, evaporated to dryness, and then separated and purified by column chromatography to afford compound **4b** (1.6 g). Synthesis of the above compound **4:**
In a sealed tube, compound **4b** (200 mg, 1.0 eq), compound **1a** (218 mg, 0.9 eq, Bide Pharmatech), Pd(OAc)₂ (28 mg, 0.1 eq), cesium carbonate (799 mg, 2.0 eq), and Xantphos (213 mg, 0.3 eq) were added, followed by the addition of tert-butanol (10 mL). The mixture was thoroughly mixed, subjected to argon replacement for protection, and the sealed tube was closed. The reaction was heated and stirred at 110°C in an oil bath for 4 h. After completion of the reaction, 20 mL of methanol was added, and the mixture was filtered by suction. The filter cake was washed with methanol. The filtrate was concentrated, and separated and purified by column chromatography (PE:EA=100:1-4:1, v/v) to afford compound **4** (80 mg).

¹H NMR (400 MHz, DMSO-*d*₆) δ 9.76 (s, 1H), 8.73 (d, *J =* 2.7 Hz, 1H), 8.28 (dd, *J* = 9.0, 2.8 Hz, 1H), 8.11 (d, *J* = 8.9 Hz, 1H), 7.74 (dd, *J* = 17.9, 7.7 Hz, 2H), 7.26 (t, *J* = 7.7 Hz, 1H), 7.07 (t, *J =* 9.5 Hz, 2H), 4.52 (t, *J =* 6.4 Hz, 2H), 2.79 (t, *J* = 6.4 Hz, 2H).

ESI-MS:m/z 325.2 [M+H]⁺.

### Example 5: Synthesis of Compound 5

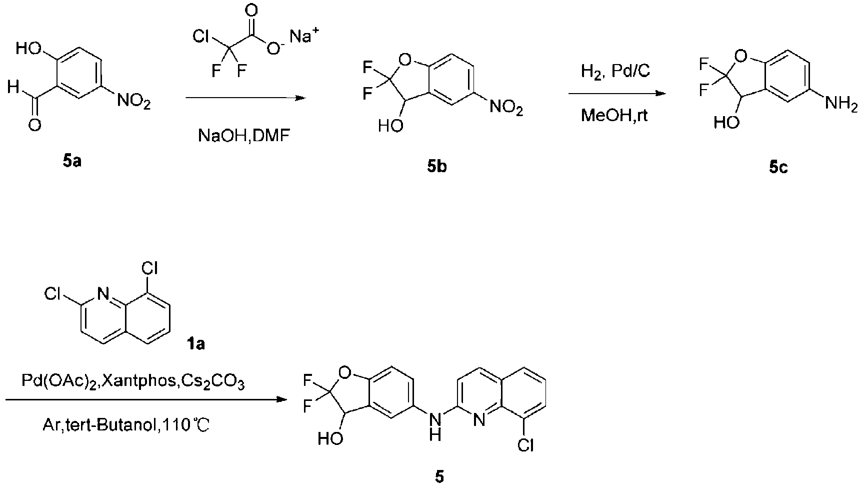

The preparation method of the above compound **5b** was as follows:
A 100 mL round-bottom flask was charged with nitrogen, followed by sequential addition of sodium hydroxide (12 g, 299 mmol) and DMF (50 mL). The mixture was heated to 95°C and reacted for 0.5 h. Then, a solution of compound **5a** (5 g, 29.9 mmol, Bide Pharmatech) and compound sodium 2-chloro-2,2-difluoroacetate (23 g, 149 mmol, Bide Pharmatech) in DMF was added dropwise. After completion of the addition, the reaction was continued for 1.5 h. Upon completion of the reaction, water (100 mL) was added, and the mixture was extracted with ethyl acetate (3 × 50 mL), washed with saturated brine, and dried over anhydrous sodium sulfate. The organic phases were combined and concentrated, and the crude product was purified by silica gel column chromatography (PE:EA = 100:1-15:1, v/v) to afford compound **5b** (5.6 g).

The preparation method of the above compound **5c** was as follows:
To a 50 mL round-bottom flask were successively added compound **5b** (3 g, 13.8 mmol), 10% palladium on carbon (300 mg), and methanol (20 mL). The reaction was carried out under a hydrogen atmosphere at room temperature overnight. After completion of the reaction, the mixture was filtered by suction, and the filter cake was washed with methanol and concentrated to afford compound **5c** (2.56 g).

### Synthesis of Compound 5

A 25 mL round-bottom flask was charged with nitrogen, and compound **1a** (198 mg, 1.0 mmol, Bide Pharmatech), compound **5c** (187 mg, 1.0 mmol), Xantphos (29 mg, 0.05 mmol), Pd(OAc)₂ (8.5 mg, 0.05 mmol), cesium carbonate (978 mg, 3.0 mmol), and tert-butanol (10 mL) were sequentially added. The mixture was heated to 100°C and reacted for 20 h. The reaction was stopped, and the mixture was evaporated to dryness under reduced pressure. Water (40 mL) was added, and the mixture was extracted with ethyl acetate (3 × 25 mL), washed with saturated brine, and dried over anhydrous sodium sulfate. The organic phases were combined and concentrated, and the crude product was purified by silica gel column chromatography (PE/EA = 100:1-10:1, v/v) to afford compound **5** (156 mg).

¹H NMR (400 MHz, DMSO-*d*₆) δ 9.79 (s, 1H), 8.51 (d, *J =* 2.3 Hz, 1H), 8.12 (d, *J =* 8.8 Hz, 1H), 8.04 (dd, *J =* 8.9, 2.5 Hz, 1H), 7.75 (dd, *J* = 16.6, 7.8 Hz, 2H), 7.27 (t, *J* = 7.6 Hz, 1H), 7.10 (t, *J* = 8.9 Hz, 2H), 6.70 (d, *J* = 7.7 Hz, 1H), 5.39 - 5.29 (m, 1H).

ESI-MS:m/z 349.2[M+H]⁺.

### Example 6: Synthesis of Compound 6

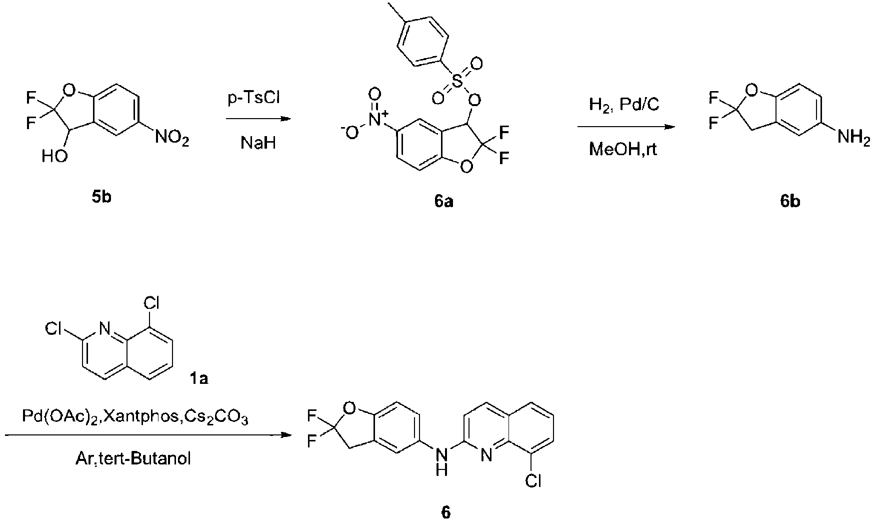

The preparation method of the above compound **6a** was as follows:
To a 50 mL round-bottom flask were added compound **5b** (1.0 g, 4.61 mmol) and anhydrous THF (15 mL), cooled to 0°C, followed by sequential portionwise addition of NaH (221 mg, 5.53 mmol) and p-toluenesulfonyl chloride (1.76 g, 9.21 mmol). After completion of addition, the reaction was continued at 10°C for 3 h. Upon completion of the reaction, the reaction was quenched with saturated ammonium chloride solution, extracted with ethyl acetate (3 × 25 mL), washed with saturated brine, and dried over anhydrous sodium sulfate. The organic phases were combined and concentrated, and the crude product was purified by silica gel column chromatography (PE/EA = 100:1-25:1, v/v) to afford compound **6a** (1.0 g).

The preparation method of the above compound **6b** was as follows:
To a 50 mL round-bottom flask were sequentially added compound **6a** (1 g, 2.69 mmol), 10% palladium on carbon (300 mg), ethyl acetate (15 mL), and tetrahydrofuran (15 mL). The reaction was carried out under hydrogen atmosphere at room temperature overnight. After completion of the reaction, the mixture was filtered under vacuum. The filter cake was washed with methanol and concentrated to afford compound **6c** (400 mg).

### Synthesis of Compound 6

A 25 mL round-bottom flask was charged with nitrogen, and compound **1a** (198 mg, 1.0 mmol, Bide Pharmatech), compound **6b** (171 mg, 1.0 mmol), Xantphos (29 mg, 0.05 mmol), Pd(OAc)₂ (8.5 mg, 0.05 mmol), cesium carbonate (978 mg, 3.0 mmol), and tert-butanol (10 mL) were sequentially added. The mixture was heated to 100°C and reacted for 20 h. The reaction was stopped, and the mixture was evaporated to dryness under reduced pressure. Water (40 mL) was added, and the mixture was extracted with ethyl acetate (3 × 25 mL), washed with saturated brine, and dried over anhydrous sodium sulfate. The organic phases were combined and concentrated, and the crude product was purified by silica gel column chromatography (PE/EA = 100:1-10:1, v/v) to afford compound **6** (90 mg).

¹H NMR (400 MHz, DMSO-*d*₆) δ 9.76 (s, 1H), 8.36 (s, 1H), 8.11 (d, *J =* 8.9 Hz, 1H), 7.97 (d, *J* = 8.8 Hz, 1H), 7.79 - 7.75 (m, 1H), 7.74 - 7.70 (m, 1H), 7.26 (t, *J* = 7.8 Hz, 1H), 7.09 (t, *J* = 8.7 Hz, 2H), 3.87 (t, *J* = 14.6 Hz, 2H) .

ESI-MS:m/z 333.2[M+H]⁺.

### Example 7: Synthesis of Compound 7

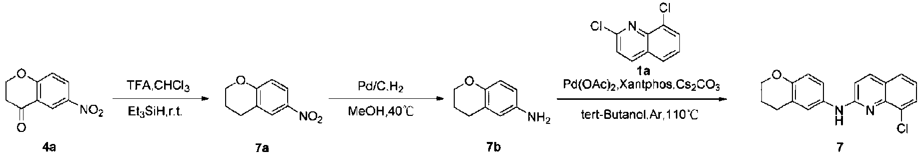

The preparation method of the above compound **7a** was as follows:
To a single-necked flask were sequentially added compound **4a** (1 g, 1.0 eq, Bide Pharmatech), TFA (10 mL), and CHCl₃ (4 mL). After stirring to dissolve, trimethylsilane (1 g, Energy Chemical) was slowly added dropwise, and the mixture was stirred at room temperature overnight. After completion of the reaction, 50 mL of water was added, and the mixture was extracted with dichloromethane (3 × 25 mL), washed with saturated brine, and dried over anhydrous sodium sulfate. The organic phases were combined and concentrated to afford crude compound **7a** (900 mg).

The preparation method of the above compound **7b** was as follows:
To a single-neck flask was added compound **7a** (900 mg, 1.0 eq), the mixture was dissolved in methanol, then 10% palladium on carbon (50 mg) was added, and the system was subjected to hydrogen replacement, heated and reacted at 40°C overnight. After completion of the reaction, the mixture was filtered by suction to remove palladium on carbon, and the filtrate was concentrated to afford compound **7b** (450 mg). Synthesis of Compound **7:**
Compound **7b** (440 mg, 1.0 eq), compound **1a** (292 mg, 0.5 eq, Bide Pharmatech), Pd(OAc)₂ (66 mg, 0.1 eq), cesium carbonate (1.92 g, 2.0 eq), and Xantphos (512 mg, 0.3 eq) were added to a sealed tube, followed by the addition of 3 mL of tert-butanol. The mixture was thoroughly mixed, subjected to argon replacement for protection, and the sealed tube was closed. The reaction was heated and stirred at 110°C in an oil bath for 4 h. After completion of the reaction, 2 times as much as methanol was added, and the mixture was filtered by suction. The filter cake was washed with methanol. After spin-drying the organic phase, the residue was purified by column chromatography (PE/EA: 100:1-3:1, v/v) to afford compound **7** (50 mg).

¹H NMR (400 MHz, DMSO-*d*₆) δ 9.48 (s, 1H), 8.19 (d, *J* = 2.6 Hz, 1H), 8.05 (d, *J* = 8.9 Hz, 1H), 7.73 (dd, *J* = 7.6, 1.4 Hz, 1H), 7.68 (dd, *J* = 8.0, 1.3 Hz, 1H), 7.61 (dd, *J* = 8.7, 2.7 Hz, 1H), 7.22 (t, *J* = 7.8 Hz, 1H), 7.06 (d, *J* = 9.0 Hz, 1H), 6.71 (d, *J* = 8.8 Hz, 1H), 4.11 (t, *J* = 5.1 Hz, 2H), 2.78 (t, *J* = 6.5 Hz, 2H), 1.94 (dq, *J* = 10.4, 6.0 Hz, 2H).

ESI-MS:m/z 311.2[M+H]⁺.

### Example 8: Synthesis of compound 8 (disclosed in Example 1 of PCT/CN2022/095441)

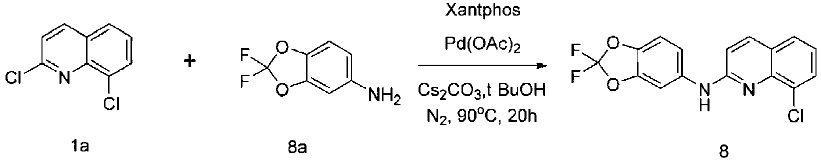

A 25 mL round-bottom flask was charged with nitrogen, and compound **1a** (198 mg, 1.0 mmol, Bide Pharmatech), compound **8a** (173 mg, 1.0 mmol, Bide Pharmatech), Xantphos (29 mg, 0.05 mmol), Pd(OAc)2 (8.5 mg, 0.05 mmol), cesium carbonate (978 mg, 3.0 mmol), and tert-butanol (10 mL) were sequentially added. The mixture was heated to 100°C and reacted for 20 h. The reaction was stopped, and the mixture was evaporated to dryness under reduced pressure. Water (40 mL) was added, and the mixture was extracted with ethyl acetate (3 × 25 mL), washed with saturated brine, and dried over anhydrous sodium sulfate. The organic phases were combined and concentrated, and the crude product was purified by column chromatography (PE/EA = 100:1-10:1, v/v) to afford compound **8** (89 mg).

¹H NMR (400 MHz, DMSO-*d*₆) δ 9.99 (s, 1H), 8.88 (d, *J* = 2.1 Hz, 1H), 8.17 (d, *J* = 8.9 Hz, 1H), 7.81 (dd, *J* = 7.6, 1.4 Hz, 1H), 7.76 (dd, *J* = 8.0, 1.4 Hz, 1H), 7.49 (dd, *J* = 8.8, 2.1 Hz, 1H), 7.38 (d, *J* = 8.8 Hz, 1H), 7.31 (t, *J* = 7.8 Hz, 1H), 7.13 (d, *J* = 8.9 Hz, 1H).

ESI-MS:m/z 335.1 [M+H]⁺.

### Example 9: Synthesis of Compound 9

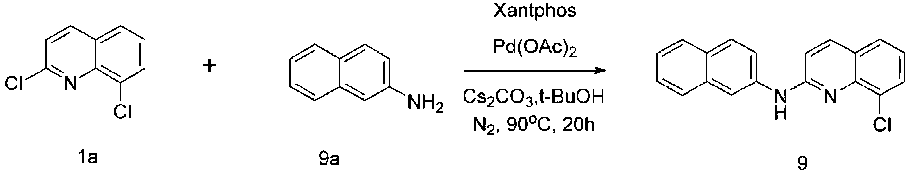

A 25 mL round-bottom flask was charged with nitrogen, and compound **1a** (300 mg, 2.1 mmol, Bide Pharmatech), compound **9a** (300 mg, 1.5 mmol, Energy Chemical), Xantphos (29 mg, 0.05 mmol), Pd(OAc)₂ (8.5 mg, 0.05 mmol), cesium carbonate (978 mg, 3.0 mmol), and tert-butanol (10 mL) were sequentially added. The mixture was heated to 90°C and reacted for 20 h. The reaction was stopped, and the mixture was evaporated to dryness under reduced pressure. Water (40 mL) was added, and the mixture was extracted with ethyl acetate (3 × 25 mL), washed with saturated brine, and dried over anhydrous sodium sulfate. The organic phases were combined and concentrated, and the crude product was subjected to silica gel column chromatography (PE/EA = 100:1-10:1, v/v) to afford compound **9** (210 mg).

¹H NMR (400 MHz, DMSO-*d*₆) δ 9.98 (s, 1H), 9.43 (d, *J* = 2.1 Hz, 1H), 8.18 (d, *J* = 8.9 Hz, 1H), 7.93 - 7.71 (m, 6H), 7.49 (t, *J* = 7.5 Hz, 1H), 7.34 (dt, *J =* 18.0, 7.6 Hz, 2H), 7.23 (d, *J* = 8.9 Hz, 1H).

ESI-MS:m/z 305.1[M+H]⁺ .

### Example 10: Synthesis of Compound 10

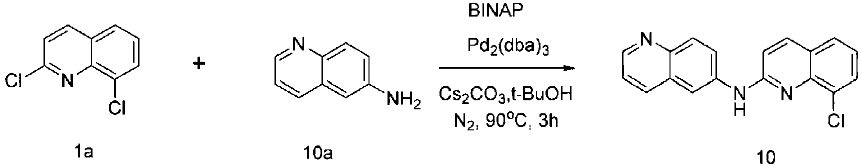

To a sealed tube were added compound **1a** (200 mg, 1 mmol, Bide Pharmatech), compound **10a** (175 mg, 1.2 mmol, Bide Pharmatech), Pd₂(dba)₃ (92.5 mg, 0.1 mmol), BINAP (188.6 mg, 0.3 mmol), and Cs₂CO₃ (658 mg, 2 mmol), followed by the addition of tert-butanol (10 mL) as solvent. The mixture was subjected to Ar replacement and reacted at 90°C for 3 h. After completion of the reaction, the reaction was stopped, and the mixture was evaporated to dryness under reduced pressure. Water (40 mL) was added, and the mixture was extracted with ethyl acetate (3 × 25 mL), washed with saturated brine, and dried over anhydrous sodium sulfate. The organic phases were combined and concentrated, and the crude product was subjected to silica gel column chromatography (PE/EA = 100:1-2:1, v/v) to afford compound **10** (110 mg).

¹H NMR (400 MHz, DMSO-*d*₆) δ 10.13 (s, 1H), 9.48 (s, 1H), 8.74 (d, *J* = 4.2 Hz, 1H), 8.21 (dd, *J* = 8.6, 4.0 Hz, 2H), 7.98 (d, *J =* 3.1 Hz, 2H), 7.85 (d, *J* = 7.6 Hz, 1H), 7.79 (d, *J* = 7.9 Hz, 1H), 7.49 (dd, *J* = 8.3, 4.2 Hz, 1H), 7.34 (t, *J* = 7.7 Hz, 1H), 7.25 (d, *J* = 8.9 Hz, 1H).

ESI-MS:m/z 306.2[M+H]⁺ .

### Example 11: Synthesis of Compound 11

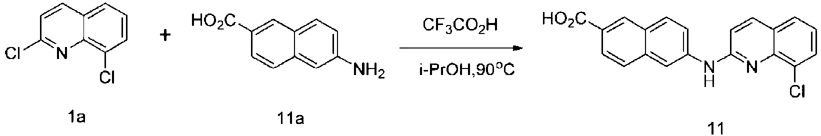

Compound **1a** (200 mg, 1 mmol, Bide Pharmatech), compound **11a** (283.6 mg, 1.5 mmol), and trifluoroacetic acid (138.2 mg, 1.2 mmol, Energy Chemical) were added to a sealed tube, followed by the addition of isopropanol (6 mL) as solvent. The atmosphere was replaced with Ar, and the reaction was carried out at 90°C for 12 h. After completion of the reaction, the reaction liquid was quenched with water, extracted with DCM (20 mL × 4), the combined organic phases were separated, washed with saturated NaCl solution, dried over anhydrous Na₂SO₄, and concentrated under vacuum to afford the crude product; purification by column chromatography (DCM/MeOH = 100:1-10:1, v/v) afforded product **11** (37 mg).

¹H NMR (400 MHz, DMSO-*d*₆) δ 10.17 (s, 1H), 9.51 (s, 1H), 8.51 (s, 1H), 8.22 (d, *J* = 8.7 Hz, 1H), 8.06 (d, *J* = 8.6 Hz, 1H), 7.95 (d, *J* = 8.3 Hz, 1H), 7.83 (dd, *J* = 23.2, 9.0 Hz, 3H), 7.36 (d, *J* = 7.9 Hz, 1H), 7.26 (d, *J* = 8.8 Hz, 1H).

ESI-MS:m/z 349.2[M+H]⁻.

### Example 12: Synthesis of Compound 12

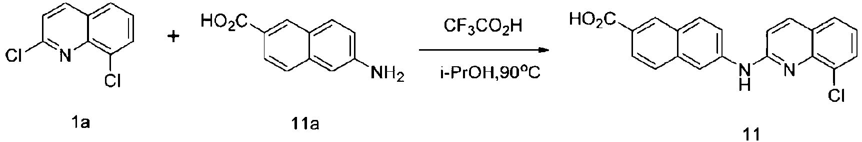

Compound **1a** (200 mg, 1 mmol, Bide Pharmatech), compound **12a** (336 mg, 1.5 mmol, Energy Chemical), and p-toluenesulfonic acid (174 mg, 1 mmol) were added to a sealed tube, followed by the addition of isopropanol (6 mL) as the solvent. The atmosphere was replaced with Ar, and the reaction was carried out at 90°C for 12 h. After completion of the reaction, the reaction liquid was quenched with water, extracted with EA (20mL × 4), the combined organic phases were separated, washed with saturated NaCl solution, dried over anhydrous Na₂SO₄, and concentrated under vacuum to afford the crude product; purification by column chromatography (DCM/MeOH = 100:1-10:1, v/v) to afford product **12** (100 mg).

¹H NMR (400 MHz, DMSO-*d*₆) δ 10.07 (s, 1H), 9.45 (d, *J* = 2.1 Hz, 1H), 8.20 (d, *J* = 8.9 Hz, 1H), 8.13 - 8.07 (m, 1H). 7.88 (d, *J* = 8.9 Hz, 1H), 7.84 (d, *J* = 7.7 Hz, 1H), 7.82 - 7.72 (m, 3H), 7.62 - 7.56 (m, 1H), 7.33 (t, *J* = 7.8 Hz, 1H), 7.23 (d, *J* = 8.9 Hz, 1H) .

ESI-MS:m/z 383.1 [M+H]⁻.

### Example 13: Synthesis of Compound 13

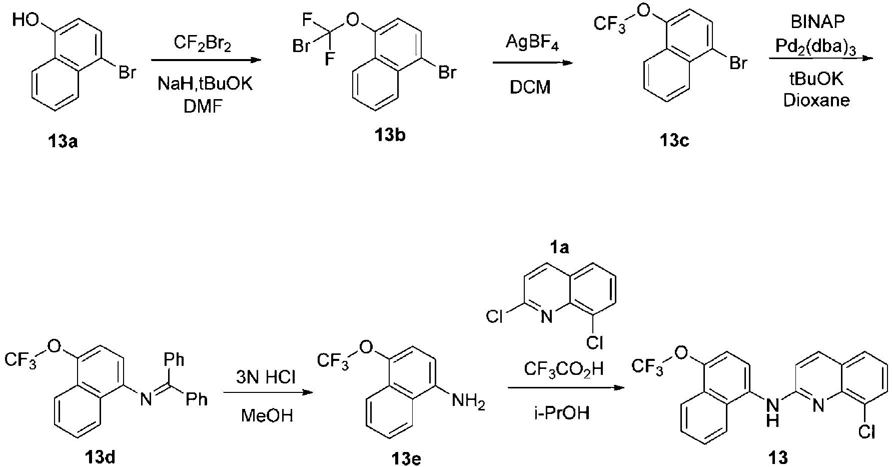

The preparation method of the above compound **13b** was as follows:
Under ice-bath cooling, NaH (1.26 g, 31.5 mmol) was added portionwise to a solution of compound **13a** (2.0 g, 10.5 mmol, Bide Pharmatech) in DMF (20 mL) in a 75 mL sealed tube. After stirring for 10 min, t-BuOK (1.3 g, 11.6 mmol) and CF₂Br₂ (8.8 g, 42.0 mmol, Energy Chemical) were added portionwise to the mixture. The sealed tube was quickly sealed and heated to 70°C to react overnight. After the reaction was completed, the reaction liquid was quenched with water, extracted with EA (20 mL × 4), the combined organic phases were separated, washed with saturated NaCl solution, dried over anhydrous Na₂SO₄, filtered, concentrated, and purified by column chromatography (PE=100%) to afford compound **13b** (1.8 g) as a colorless oil.

The preparation method of the above compound **13c** was as follows:
Under N₂, compound **13b** (4.0 g, 11.4 mmol) was dissolved in anhydrous DCM (60 mL), cooled to -78°C, then AgBF₄ (4.9 g, 25.2 mmol, Energy Chemical) was added, and the reaction liquid was slowly warmed to room temperature and stirred overnight. The NaHCO₃ solution was added to the mixture until pH>8. The mixture was extracted with DCM (30 mL×4), the combined organic phases was separated, washed with saturated NaCl solution, dried over anhydrous Na₂SO₄, filtered, and concentrated to obtain compound **13c** (2.2 g) as a brown oil. The preparation method of the above compound **13d** was as follows:
Compound **13c** (210 mg, 0.72 mmol), benzophenone imine (156 mg, 0.86 mmol, Bide Pharmatech), Pd₂(dba)₃ (16 mg), BINAP (11 mg), and potassium tert-butoxide (112 mg, 1.0 mmol) and 5 mL of anhydrous dioxane were sequentially added to a 25 mL sealed tube, and the mixture was heated to 90°C and reacted for 4 h. After completion of the reaction, the mixture was quenched with water, extracted with EA (20 mL × 4), the combined organic phases were separated, washed with saturated NaCl solution, dried over anhydrous Na₂SO₄, filtered, concentrated, and purified by column chromatography (PE:EA = 100:1-30:1, v/v) to afford compound **13d** (200 mg).

The preparation method of the above compound **13e** was as follows:
Compound **13d** (200 mg, 0.51 mmol) was dissolved in 5 mL of methanol, 1 N HCl (2 mL) was added, and the reaction was carried out at room temperature for 1 h. After completion of the reaction, the mixture was concentrated to afford compound **13e** (79 mg).

### Synthesis of Compound 13:

To a 75 mL sealed tube were sequentially added compound **13e** (270 mg, 1.52 mmol, 1.0 equiv.), isopropanol (10 mL), compound **1a** (357 mg, 1.8 mmol, 1.5 eq., Bide Pharmatech), and CF₃COOH (107 mg, 1.44 mmol, 1.2 eq.). The tube was sealed and heated to 100°C to react for 48 h. After completion of the reaction, the reaction liquid was extracted with ethyl acetate/water. The combined organic phases were separated, washed with saturated NaCl solution, dried over anhydrous Na₂SO₄, filtered, concentrated, and purified by column chromatography (PE:EA = 100:1-97:3, v/v) to afford compound **13** (200 mg).

¹H NMR (400 MHz,DMSO-*d*₆) δ 9.64 (s, 1H), 8.98 (d, *J =* 8.6 Hz, 1H), 8.80 - 8.40 (m, 1H). 8.21 (d, *J =* 9.0 Hz, 1H), 8.18 - 7.93 (m, 1H), 7.95 - 7.61 (m, 4H), 7.62 (dd, *J* = 8.6, 1.7 Hz, 1H), 7.49 (d, *J* = 9.0 Hz, 1H), 7.30 (t, *J* = 7.8 Hz, 1H).

ESI-MS:m/z 389.2[M+H]⁺ .

### Example 14: Synthesis of Compound 14

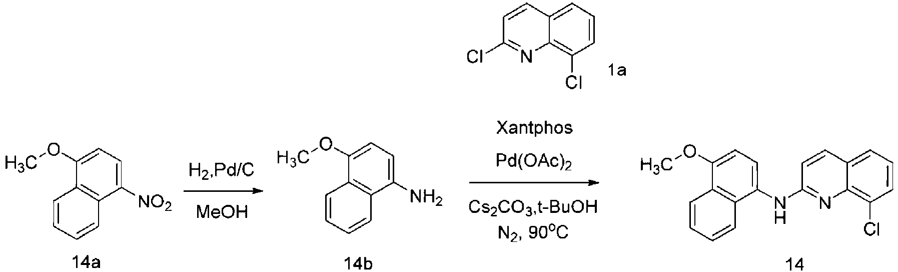

The preparation method of the above compound **14b** was as follows:
To a single-neck flask was added compound **14a** (350 mg, 1.72 mmol, Bide Pharmatech), dissolved in methanol, followed by addition of 10% palladium on carbon (50 mg). The mixture was subjected to hydrogen replacement and stirred at room temperature overnight. After completion of the reaction, the mixture was filtered by suction to remove palladium on carbon, and the filtrate was concentrated to afford compound **14b** (250 mg).

### Synthesis of Compound 14:

To a sealed tube were added compound **1a** (200 mg, 1 mmol, Bide Pharmatech), compound **14b** (210 mg, 1.2 mmol), Pd(OAc)₂ (22.7 mg, 0.1 mmol), Xantphos (175.3 mg, 0.3 mmol), and Cs₂CO₃ (658 mg, 2 mmol). Then, tert-butanol (10 mL) was added as the solvent, and the mixture was replaced with Ar and reacted at 90°C for 5 h. After completion of the reaction, the reaction liquid was quenched with water and extracted with EA (20 mL × 4). The combined organic phases were separated, washed with saturated NaCl solution, dried over anhydrous Na₂SO₄, and concentrated under vacuum to afford the crude product. Purification by column chromatography (PE/EA = 100:1-3:1, v/v) to afford compound **14** (80 mg).

¹H NMR (400 MHz, DMSO-*d*₆) δ 9.31 (s, 1H), 8.32 (d, *J* = 8.4 Hz, 1H), 8.23 (dd, *J* = 7.7, 1.9 Hz, 1H), 8.20 - 8.17 (m, 1H), 8.07 (d, *J* = 8.9 Hz, 1H), 7.73 - 7.67 (m, 2H), 7.57 (td, *J =* 7.8, 1 4 Hz, 2H), 7.22 (t, *J* = 7.7 Hz, 1H), 7.14 (d, *J* = 8.9 Hz, 1H), 7.04 (d, *J* = 8.4 Hz, 1H), 4.01 (s, 3H).

ESI-MS:m/z 335.2[M+H]⁻.

### Example 15: Synthesis of Compound 15

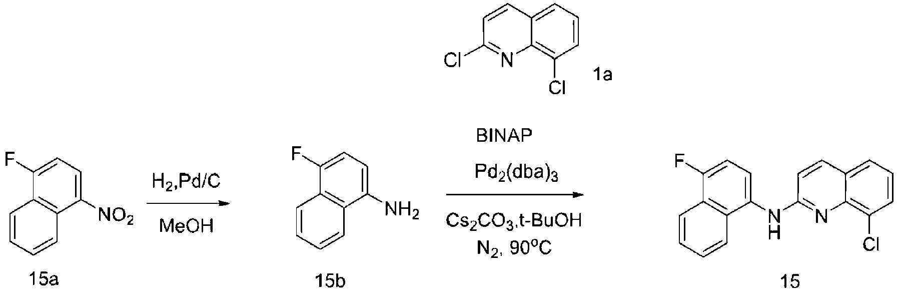

The preparation method of the above compound **15b** was as follows:
To a single-neck flask was added compound **15a** (350 mg, 1.57 mmol, Bide Pharmatech), dissolved in methanol, followed by addition of 10% palladium on carbon (50 mg). The mixture was subjected to hydrogen replacement and stirred at room temperature overnight. After completion of the reaction, the mixture was filtered by suction to remove palladium on carbon, and the filtrate was concentrated to afford compound **14b** (220 mg).

Synthesis of Compound **15:** To a sealed tube were added compound **1a** (200 mg, 1 mmol, Bide Pharmatech), compound **15b** (195 mg, 1.2 mmol), Pd₂(dba)₃ (92.5 mg, 0.1 mmol), BINAP (188.6 mg, 0.3 mmol), and Cs₂CO₃ (658 mg, 2 mmol). Then, tert-butanol (10 mL) was added as the solvent, and the mixture was replaced with Ar and reacted at 90°C for 4 h. After completion of the reaction, water was added to the reaction liquid and the system was extracted with EA (20 mL × 4). The organic phases were combined, washed with saturated NaCl solution, dried over anhydrous Na₂SO₄, and concentrated under vacuum to afford the crude product. Purification by column chromatography (PE/EA = 100:1-3:1, v/v) to afford compound **15** (120 mg).

¹H NMR (400 MHz, DMSO-*d*₆) δ 9.51 (s, 1H), 8.64 (dd, *J* = 8.6, 5.2 Hz, 1H), 8.41 - 8.35 (m, 1H), 8.15 (d, *J* = 9.0 Hz, 1H), 8.13 - 8.06 (m, 1H), 7.74 (dd, *J* = 7.9, 2.2 Hz, 2H), 7.68 (dd, *J* = 6.6, 3.2 Hz, 2H), 7.40 (dd, *J =* 10.5, 8.5 Hz, 1H), 7.34 (d, *J* = 8.9 Hz, 1H), 7.26 (t, *J=* 7.8 Hz, 1H).

ESI-MS:m/z 323.2[M+H]⁻

### Example 16: Synthesis of Compound 16

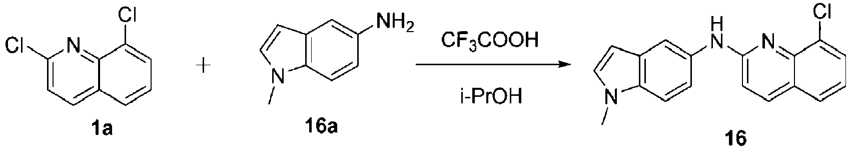

To a sealed tube were added compound **16a** (200 mg, 1.36 mmol, Bide Pharmatech), compound **1a** (140 mg, 0.71 mmol, Bide Pharmatech), and TFA (122 mg, 1.07 mmol). The mixture was dissolved in 5 mL of isopropanol, and the system was heated and reacted at 100°C overnight. After completion of the reaction, 10 mL of water was added, and an appropriate amount of K₂CO₃ was used to adjust the pH to ~8. The mixture was extracted with dichloromethane (20 mL × 3), and the organic phases were combined, washed with saturated NaCl solution, dried over anhydrous Na₂SO₄, and concentrated under vacuum to afford the crude product. Purification by column chromatography (PE/EA = 100:1-6:1, v/v) to afford compound 16 (50 mg).

¹H NMR (400 MHz, DMSO-*d*₆) δ 9.53 (s, 1H), 8.70 (d, *J =* 2.0 Hz, 1H), 8.05 (d, *J =* 8.9 Hz, 1H), 7.74 (dd, *J* = 7.6, 1.3 Hz, 1H), 7.71 - 7.62 (m, 2H), 7.41 (d, *J* = 8.8 Hz, 1H), 7.28 (d, *J* = 3.0 Hz, 1H), 7.22 (t, *J* = 7.7 Hz, 1H), 7.11 (d, *J* = 9.0 Hz, 1H), 6.38 (d, *J* = 3.0 Hz, 1H), 3.79 (s, 3H).

ESI-MS:m/z 308.2[M+H]⁻.

### Example 17: Synthesis of Compound 17

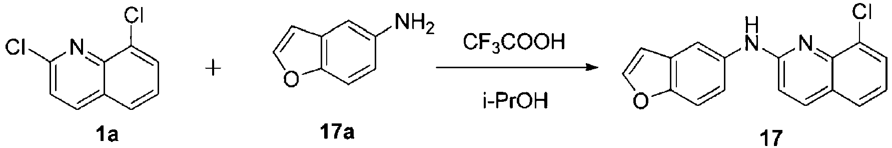

To a sealed tube were added compound **17a** (200 mg, 1.5 mmol, Bide Pharmatech), compound **1a** (149 mg, 0.75 mmol, Bide Pharmatech), and TFA (134mg, 1.18mmol). The mixture was dissolved in 5 mL of isopropanol, and the system was heated and reacted at 100°C overnight. After completion of the reaction, 10 mL of water was added, and an appropriate amount of K₂CO₃ was used to adjust the pH to ~8. The mixture was extracted with dichloromethane (20 mL × 3), and the organic phases were combined, washed with saturated NaCl solution, dried over anhydrous Na₂SO₄, and concentrated under vacuum to afford the crude product. Purification by column chromatography (PE/EA = 100:1-6:1, v/v) to afford compound **17** (200 mg).

¹H NMR (400 MHz, DMSO-*d*₆) δ 9.74 (s, 1H), 8.87 (d, *J* = 2.2 Hz, 1H), 8.11 (d, *J* = 8.9 Hz, 1H), 7.96 (d, *J =* 2.1 Hz, 1H), 7.83 - 7.75 (m, 2H), 7.72 (dd, *J* = 8.0, 1.3 Hz, 1H), 7.56 (d, *J* = 8.9 Hz, 1H), 7.26 (t, *J* = 7.8 Hz, 1H), 7.15 (d, *J* = 8.9 Hz, 1H), 6.99 - 6.92 (m, 1H).

ESI-MS:m/z 295.2[M+H]⁺.

### Example 18: Synthesis of Compound 18

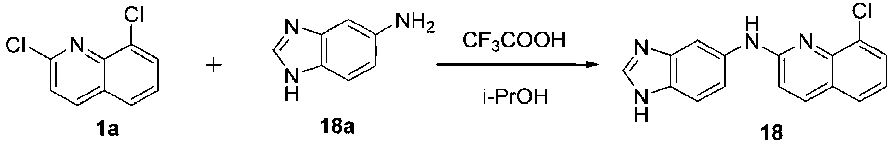

To a 75 mL sealed tube were sequentially added compound **18a** (200 mg, 1.5 mmol, Bide Pharmatech), isopropanol (10 mL), and compound **1a** (446 mg, 2.25 mmol, Bide Pharmatech), followed by CF₃COOH (134 mg, 1.8 mmol). The mixture was heated and reacted at 100°C for 48 h. After completion of the reaction, 30 mL of water was added, resulting in precipitation of a solid. The solid was filtered by suction to afford compound **18** (100 mg).

¹H NMR (400 MHz, CDCl₃) δ 10.30 (s, 1H), 9.49 (d, *J* = 32.3 Hz, 2H), 8.17 (d, *J =* 8.9 Hz, 1H), 7.78 (dt, *J* = 13.2, 7.6 Hz, 4H), 7.36 - 7 16 (m, 2H), 5.71 (s, 1H).

ESI-MS:m/z 295.1[M+H]⁻

### Example 19: Synthesis of Compound 19

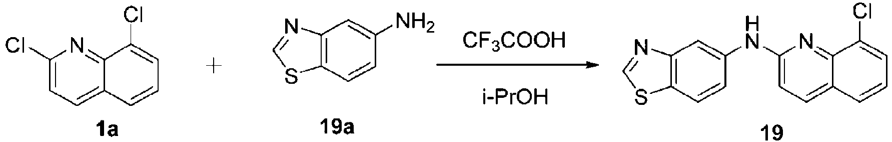

To a 75 mL sealed tube were sequentially added compound **1a** (416 mg, 1.4 mmol), isopropanol (10 mL), and compound **19a** (200 mg, 2.1 mmol, Energy Chemical), followed by CF₃COOH (125 mg, 1.68 mmol). The mixture was heated and reacted at 100°C for 48 hours. After completion of the reaction, water (30 mL) was added, resulting in precipitation of a solid. The solid was filtered by suction to afford compound 19 (200 mg).

¹H NMR (400 MHz, CDCl₃) δ 9.97 (s, 1H), 9.47 (d, *J =* 2.1 Hz, 1H), 9.34 (s, 1H), 8.16 (d, *J* = 8.9 Hz, 1H), 8.07 (d, *J* = 8.7 Hz, 1H), 7.88 (dd, *J* = 8.8, 2.2 Hz, 1H), 7.78 (ddd, *J* = 19.0, 7.8, 1.4 Hz, 2H), 7.30 (t, *J* = 7.8 Hz, 1H), 7.20 (d, *J* = 8.9 Hz, 1H), 5.75 (s, 1H).

ESI-MS:m/z 312.1[M+H]⁺.

### Example 20: Synthesis of Compound 20

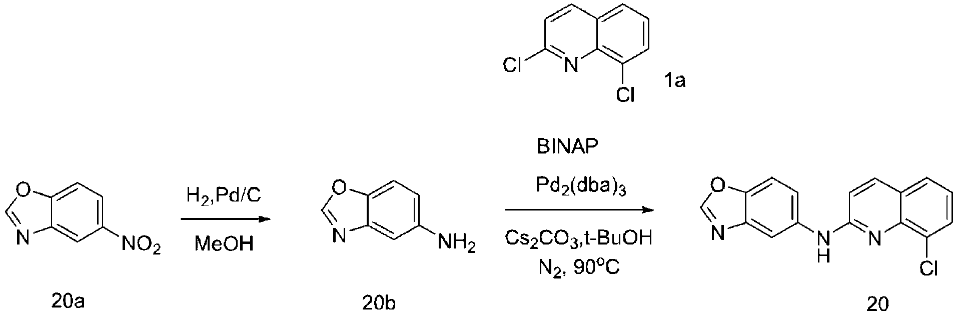

The preparation method of the above compound **20b** was as follows:
To a single-neck flask was added compound **20a** (350 mg, 1.83 mmol, Bide Pharmatech), dissolved in methanol, followed by addition of 10% palladium on carbon (50 mg). The mixture was subjected to hydrogen replacement and stirred at room temperature overnight. After completion of the reaction, the mixture was filtered by suction to remove palladium on carbon, and the filtrate was concentrated to afford compound **20b** (230 mg).

Synthesis of Compound **20:**
To a sealed tube were added compound **1a** (200 mg, 1 mmol, Bide Pharmatech), compound **20a** (162.6 mg, 1.2 mmol), Pd₂(dba)₃ (92.5 mg, 0.1 mmol), BINAP (188.6 mg, 0.3 mmol), and Cs₂CO₃ (658 mg, 2 mmol). Then, tert-butanol (10 mL) was added as the solvent, and the mixture was replaced with Ar and reacted at 90°C for 4 h. After completion of the reaction, the reaction liquid was quenched with water and extracted with EA (20 mL × 4). The combined organic phases were separated, washed with saturated NaCl solution, dried over anhydrous Na₂SO₄, and concentrated under vacuum to afford the crude product. Purification by column chromatography (PE/EA = 100:1-3:1, v/v) to afford compound **20** (100 mg).

¹H NMR (400 MHz, DMSO-*d*₆) δ 9.90 (s, 1H), 9.16 (d, *J* = 2.1 Hz, 1H), 8.68 (s, 1H), 8.15 (d, *J* = 9.0 Hz, 1H), 7.79 (ddd, *J* = 9.0, 6.6, 1.7 Hz, 2H), 7.75 (d, *J* = 9.7 Hz, 2H), 7.29 (t, *J* = 7.8 Hz, 1H), 7.17 (d, *J* = 8.9 Hz, 1H).

ESI-MS:m/z 296.1[M+H]⁺.

### Example 21: Synthesis of Compound 21

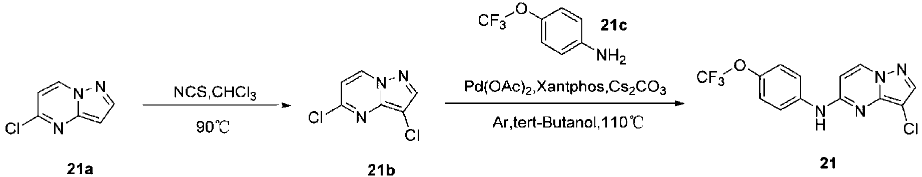

The preparation method of the above compound **21b** was as follows:
To a single-necked flask were added compound **21a** (1 g, 1.0 eq, Bide Pharmatech), NCS (1.04 g, 1.2 eq, Bide Pharmatech), and 10 mL of chloroform. The mixture was heated and reacted at 90°C for 4 h. After completion of the reaction, the reaction liquid was distilled and evaporated to dryness under reduced pressure to afford crude compound **21b** (0.6 g).

### Synthesis of Compound 21:

In a sealed tube, compound **21b** (300 mg, 1.0 eq), compound **21c** (339 mg, 1.2 eq, Bide Pharmatech), Pd(OAc)₂ (36 mg, 0.1 eq), cesium carbonate (1.04 g, 2.0 eq), and Xantphos (277 mg, 0.3 eq) were added, followed by the addition of 5 mL tert-butanol. The mixture was subjected to argon replacement for protection, heated and reacted at 110°C in an oil bath for 10 h. After completion of the reaction, 2 times as much as methanol was added, and the reaction liquid was filtered by suction. The filter cake was washed with methanol. The filtrate was concentrated, and purified by column chromatography (PE:EA=100:1-6:1, v/v) to afford compound 21 (30 mg).

¹H NMR (400 MHz, DMSO-*d*₆) δ 10.08 (s, 1H), 8.71 (d, *J* = 7.5 Hz, 1H), 8.07 (s, 1H), 8.05 - 7.94 (m, 2H), 7.39 (d, *J =* 8.6 Hz, 2H), 6.58 (d, *J* = 7.6 Hz, 1H).

ESI-MS:m/z 329.1[M-H]⁺.

### Example 22: Synthesis of Compound 22

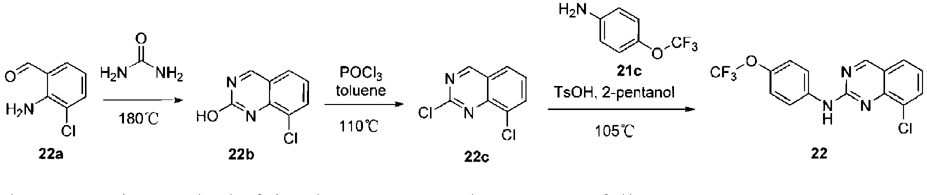

The preparation method of the above compound **22b** was as follows:
In a sealed tube, compound **22a** (2.75 g, 1.0 eq, Bide Pharmatech), urea (10.62 g, 10.0 eq) were added, and the mixture was heated and stirred at 180°C for 4 h. After completion of the reaction, the mixture was cooled to room temperature, and ice water was added with stirring. The mixture was filtered by suction, and the filter cake was washed with ice water. The solid was dissolved in a mixture of 200 mL saturated sodium carbonate and 250 mL water, and extracted with EA until no impurities remained in the aqueous phase. The pH was adjusted to 5-6 with concentrated HCl, stirred for 1 h, and filtered by suction to obtain compound **22b** (2.35 g).

The preparation method of the above compound **22c** was as follows:
To a single-necked flask were added compound **22b** (2.35 g, 1.0 eq), phosphorus oxychloride (3.99 g, 2.0 eq, Energy Chemical), and 20 mL of toluene, and the mixture was heated to reflux at 110°C for 6 h. After completion of the reaction, the reaction liquid was concentrated to dryness to afford compound **22c** (800 mg), which was used directly in the next step without purification.

### Synthesis of Compound 22:

In a sealed tube, compound **22c** (800 mg, 1.0 eq), compound **21c** (854 mg, 1.2 eq, Bide Pharmatech), and p-toluenesulfonic acid (830 mg, 1.0 eq) were added, dissolved in 2-pentanol, and then the mixture was heated and reacted at 105°C for 5 h. After completion of the reaction, 2-pentanol was evaporated to dryness, water (30 mL) was added, and the mixture was extracted with EA (20 mL × 4). The organic phases were combined, washed with saturated NaCl solution, dried over anhydrous Na₂SO₄, and concentrated under vacuum to afford the crude product. Purification by column chromatography (PE/EA = 100:1-5:1, v/v) to afford 20 mg of compound **22.**

¹H NMR (400 MHz, DMSO-*d*₆) δ 10.41 (s, 1H), 9.42 (s, 1H), 8.26 (d, *J =* 8.7 Hz, 2H), 8.03 (d, *J* = 7.6 Hz, 1H), 7.95 (d, *J* = 7.9 Hz, 1H), 7.38 (d, *J* = 9.1 Hz, 3H), 7.20 (t, *J* = 7.9 Hz, 1H) .

ESI-MS:m/z 340.1[M+H]⁺.

### Example 23: Synthesis of Compound 23

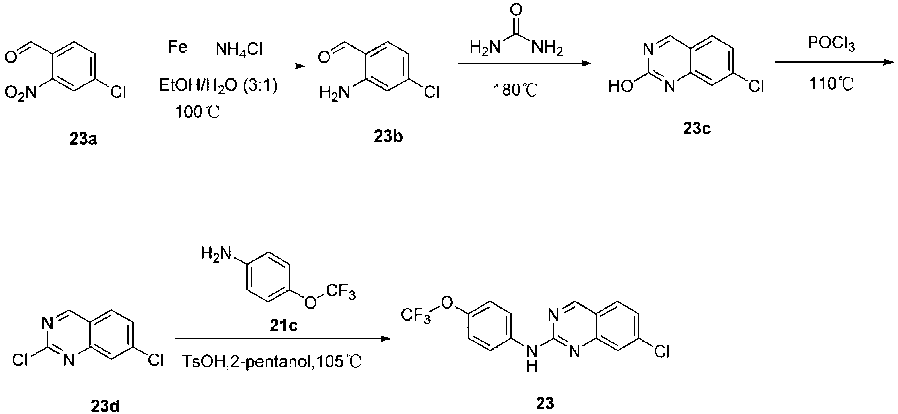

The preparation method of the above compound **23b** was as follows:
To a single-necked flask were added compound **23a** (2 g, 1.0 eq, Bide Pharmatech), reduced iron powder (1.8 g, 3.0 eq), NH₄Cl (1.8 g, 3.0 eq), and 40 mL of ethanol/water (3:1), and the mixture was refluxed at 100°C overnight. After filtration to remove the iron powder, the ethanol was evaporated to dryness, and the mixture was extracted with water and EA. The organic phase was evaporated to dryness to afford compound **23b** (1.2 g).

The preparation method of the above compound **23c** was as follows:
Compound **23b** (1.2 g, 1.0 eq) and urea (4.24 g, 10.0 eq, Bide Pharmatech) were added to a sealed tube, and the mixture was heated and stirred at 180°C for 4 h. After completion of the reaction, the mixture was cooled to room temperature, and ice water was added with stirring. The mixture was filtered by suction, and the filter cake was washed with ice water. The moisture in the filter cake was evaporated to dryness to afford compound **23c** (1.2 g).

The preparation method of the above compound **23d** was as follows:
Compound 3 (1.1g, 1.0eq) and 10 mL POCl₃ were added to a single-neck flask, and the mixture was stirred under reflux at 110°C for 4h. After completion of the reaction, most of POCl₃ was removed by distillation under reduced pressure, and the residual POCl₃ was quenched with ice water. The solid impurities were removed by filtration under suction, and the mixture was extracted with EA. The organic phase was evaporated to dryness to afford compound **23d** (400 mg).

### Synthesis of Compound 23:

In a sealed tube, compound **23d** (400 mg, 1.0 eq), compound **21c** (392 mg, 1.1 eq, Bide Pharmatech), and TsOH (415 mg, 1.2 eq) were added. The mixture was dissolved in 2-pentanol and heated to reflux at 105°C for 4 h. After completion of the reaction, 2-pentanol was removed by distillation under reduced pressure, water (30 mL) was added, and the mixture was extracted with EA (20 mL × 4). The organic phases were combined, washed with saturated NaCl solution, dried over anhydrous Na₂SO₄, and concentrated under vacuum to afford the crude product. Purification by column chromatography (PE/EA = 100:1-4:1, v/v) to afford compound **23** (55 mg).

¹H NMR (400 MHz, CDCl₃) δ 9.08 (s, 1H), 7.89 - 7.84 (m, 2H), 7.79 (d, *J* = 1.9 Hz, 1H), 7.70 (d, *J* = 8.5 Hz, 1H), 7.47 (s, 1H), 7.34 (dd, *J* = 8.5, 1.9 Hz, 1H), 7.28 (d, *J =* 4.7 Hz, 2H).

ESI-MS:m/z 340.1[M+H]⁻.

### Example 24: Synthesis of Compound 24

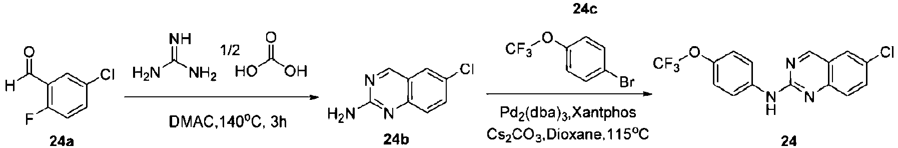

The preparation method of the above compound **24b** was as follows:
Compound 5-chloro-2-fluorobenzaldehyde (24a, 3.02 g, 19.1 mmol, Bide Pharmatech) was dissolved in DMAC (10 mL), 4 times as much as guanidine carbonate (4 eg, Bide Pharmatech) was added, and the mixture was reacted at 140°C for 3 hours. TLC monitoring indicated no remaining starting material, heating was stopped, and the mixture was cooled to room temperature, followed by addition of 100 mL of water and cooling at 0°C overnight. The mixture was filtered by suction and oven-dried to afford the yellow solid compound **24b** (2.2 g).

### Synthesis of Compound 24:

To a round-bottom flask were sequentially added **24b** (180 mg, 1.0 mmol), dioxane (75 mL), **24c** (240 mg, 1.0 mmol, Bide Pharmatech), Pd₂(dba)₃ (46 mg, 0.05 mmol), cesium carbonate (480 mg, 1.5 mmol), and Xantphos (29 mg, 0.05 mmol). The mixture was then heated to 115°C under nitrogen protection and reacted for 6 h. The reaction was stopped, cooled to room temperature, and the solvent was evaporated under reduced pressure. To the residue was added water (20 mL), extracted 3 times with ethyl acetate (3 x 20 mL), the organic phases were combined, then washed with saturated brine, dried over anhydrous sodium sulfate, and then concentrated. The crude product was subjected to silica gel column chromatography (PE/EA = 4:1-2:1, v/v) to afford the yellow solid compound **24** (120 mg).

¹H NMR (400 MHz, DMSO-*d*₆) δ 10.21 (s, 1H), 9.32 (s, 1H), 8.16 - 8.02 (m, 3H), 7.87 - 7.79 (m, 1H), 7.70 (d, *J* = 9.0 Hz, 1H), 7.34 (d, *J* = 8.6 Hz, 2H) .

ESI-MS:m/z 340.1[M+H]⁺.

### Example 25: Synthesis of Compound 25

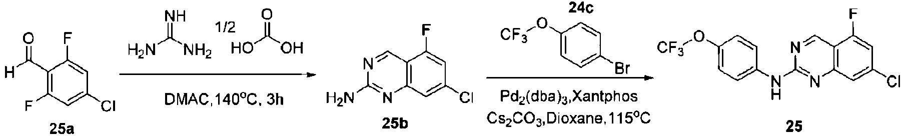

The preparation method of the above compound **25b** was as follows:
Compound **25a** (3.0 g, 17.0 mmol, Bide Pharmatech) was dissolved in DMAC (10 mL), 4 times as much as guanidine carbonate (4 eg, Bide Pharmatech) was added, and the mixture was reacted at 140°C for 3 hours. TLC monitoring indicated no remaining starting material, heating was stopped, and the mixture was cooled to room temperature, followed by addition of 100 mL of water and cooling at 0°C overnight. The mixture was filtered by suction, oven-dried to afford compound **25b** (2.3 g), a yellow solid, which was used directly in the next step without purification.

### Synthesis of Compound 25:

To a round-bottom flask were sequentially added **25b** (198 mg, 1.0 mmol), dioxane (75 mL), **24c** (240 mg, 1.0 mmol), Pd₂(dba)₃ (46 mg, 0.05 mmol), cesium carbonate (480 mg, 1.5 mmol), and Xantphos (29 mg, 0.05 mmol). The mixture was then heated to 115°C under nitrogen protection and reacted for 6 h. The reaction was stopped, cooled to room temperature, and the solvent was evaporated under reduced pressure. To the residue was added water (20 mL), extracted 3 times with ethyl acetate (3 x 20 mL), the organic phases were combined, then washed with saturated brine, dried over anhydrous sodium sulfate, and then concentrated. The crude product was subjected to silica gel column chromatography (PE/EA = 4:1-2:1, v/v) to afford the yellow solid compound **25** (110 mg).

¹H NMR (400 MHz, DMSO-*d*₆) δ 9.30 (s, 1H), 7.82 (d, *J* = 8.6 Hz, 2H), 7.56 (s, 1H), 7.45 (s, 1H), 7.23 (s. 1H), 6.99 (d, *J =* 8.8 Hz, 1H).

ES1-MS:m/z 358.1[M+H]⁻.

### Example 26: Synthesis of Compound 26

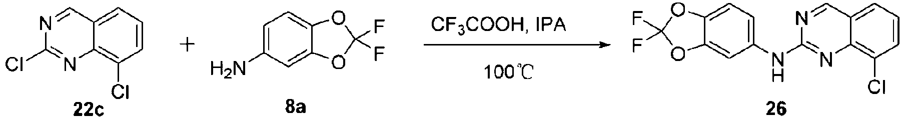

In a sealed tube, compound **22c** (400 mg, 1.0 eq), compound **8a** (418 mg, 1.2 eq, Bide Pharmatech), and TFA (179 mg, 1.2 eq) were added. The mixture was dissolved in 5 mL of isopropanol, and the system was heated and stirred at 100°C overnight. After completion of the reaction, 10 mL of water was added, and an appropriate amount of K₂CO₃ was used to adjust the pH to ~8. The mixture was extracted with dichloromethane (20 mL × 3), and the organic phases were combined, washed with saturated NaCl solution, dried over anhydrous Na₂SO₄, and concentrated under vacuum to afford the crude product. Purification by column chromatography (PE/EA = 100:1-3:1, v/v) to afford compound **26** (70 mg).

¹H NMR (400 MHz, DMSO-*d*₆) δ 10.48 (s, 1H), 9.41 (s, 1H), 8.62 (s, 1H), 8.04 (dd, *J* = 7.6, 1.3 Hz, 1H), 7.95 (dd, *J =* 8.0, 1.3 Hz, 1H), 7.65 (dd, *J =* 8.9, 2.1 Hz, 1H), 7.41 (dd, *J =* 8.4, 6.5 Hz, 2H) .

ESI-MS:m/z 336.1[M+H]⁻.

### Example 27: Synthesis of Compound 27

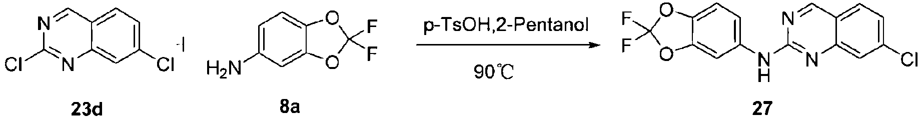

Compound **23d** (200 mg, 1 mmol), compound **8a** (191.4 mg, 1.1 mmol, Bide Pharmatech), and p-toluenesulfonic acid (207.6 mg, 1.2 mmol) were added to a sealed tube, followed by the addition of sec-amyl alcohol (6 mL) as the solvent. The atmosphere was replaced with Ar, and the reaction was carried out at 90°C for 2 h. After completion of the reaction, water (30 mL) was added to the reaction liquid, which was then extracted with EA (20 mL × 4). The organic phases were combined, washed with saturated NaCl solution, dried over anhydrous Na₂SO₄, and concentrated under vacuum to afford the crude product. Purification by column chromatography (PE/EA = 100:1-3:1, v/v) to afford compound **27** (30 mg).

¹H NMR (400 MHz, DMSO-*d*₆) δ 10.26 (s, 1H), 9.35 (s, 1H), 8.30 (d, *J* = 2.1 Hz, 1H), 7.98 (d, *J* = 8.5 Hz, 1H), 7.80 (d, *J* = 2.0 Hz, 1H), 7.57 (dd, *J* = 8.8, 2.1 Hz, 1H), 7.43 (dd, *J* = 8.5, 2.0 Hz, 1H), 7.36 (d, *J* = 8.8 Hz, 1H).

ESI-MS:m/z 336.1[M+H]⁻.

### Example 28: Synthesis of Compound 28

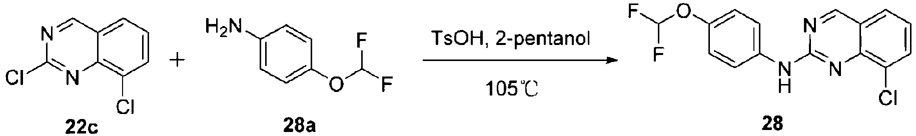

In a sealed tube, compound **22c** (400 mg, 1.0 eq), compound **28a** (384 mg, 1.2 eq, Bide Pharmatech), and p-toluenesulfonic acid (346 mg, 1.0 eq) were added, dissolved in 2-pentanol, and then the mixture was heated and stirred at 105°C for 10 h. After completion of the reaction, 2-pentanol was evaporated to dryness, 10 mL of water was added, and an appropriate amount of K₂CO₃ was used to adjust the pH to ~8. The mixture was extracted with dichloromethane (20 mL × 3), and the organic phases were combined, washed with saturated NaCl solution, dried over anhydrous Na₂SO₄, and concentrated under vacuum to afford the crude product. Purification by column chromatography (PE/EA = 100:1-10:1, v/v) to afford compound **28** (88 mg).

¹H NMR (400 MHz, DMSO-*d*₆) δ 10.29 (s, 1H), 9.39 (s, 1H), 8.19 (d, *J* = 8.6 Hz, 2H), 8.01 (d, *J* = 7.6 Hz, 2H), 7.93 (d, *J* = 7.9 Hz, 1H), 7.40 - 7.34 (m, 1H), 7.28 - 7.10 (m, 3H) .

ESI-MS:m/z 322.2[M+H]⁺.

### Example 29: Synthesis of Compound 29

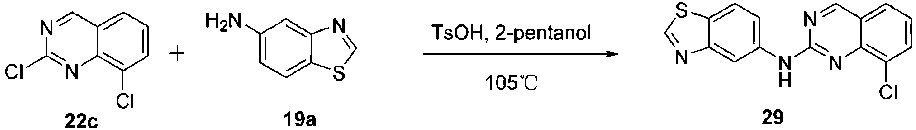

Compound **22c** (500 mg, 1.0 eq), compound **19a** (94 mg, 0.25 eq, Energy Chemical), and p-toluenesulfonic acid (233 mg, 1.2 eq) were added to a sealed tube, dissolved in 2-pentanol, and then the mixture was heated and stirred at 105°C for 10 h. After completion of the reaction, 2-pentanol was evaporated to dryness, 10 mL of water was added, and an appropriate amount of K₂CO₃ was used to adjust the pH to ~8. The mixture was extracted with dichloromethane (20 mL × 3), and the organic phases were combined, washed with saturated NaCl solution, dried over anhydrous Na₂SO₄, and concentrated under vacuum to afford the crude product. Purification by column chromatography (PE/EA = 100:1-10:1, v/v) to afford compound **29** (88 mg).

¹H NMR (400 MHz, DMSO-*d*₆) δ 10.46 (s, 1H), 9.43 (s, 1H), 9.37 (s, 1H), 9.29 (s, 2H), 8.09 (d, *J* = 8.7 Hz, 1H), 8.03 (dd, *J =* 13.6, 8.2 Hz, 2H), 7.96 (d, *J =* 7.9 Hz, 1H), 7.41 (t, *J =* 7.8 Hz, 1H).

ESI-MS:m/z 313.1 [M+H]⁻.

### Example 30: Synthesis of Compound 30

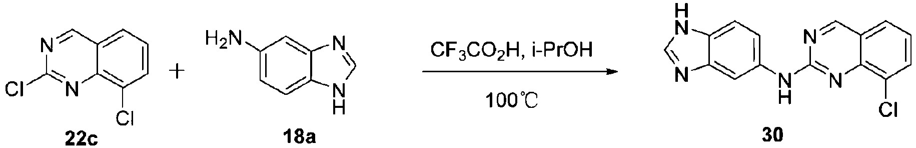

In a sealed tube, compound **22c** (500 mg, 1.0 eq), compound **18a** (84 mg, 0.25 eq, Bide Pharmatech), and TFA (223 mg, 1.2 eq) were added. The mixture was dissolved in 5 mL of isopropanol, and the system was heated and stirred at 100°C overnight. After completion of the reaction, the reaction liquid was concentrated, and 10 mL of water was added. An appropriate amount of K₂CO₃ was used to adjust the pH to ~8. The mixture was extracted with dichloromethane (20 mL × 3), and the organic phases were combined, washed with saturated NaCl solution, dried over anhydrous Na₂SO₄, and concentrated under vacuum to afford the crude product. Purification by column chromatography (PE/EA = 100:1-3:1, v/v) to afford compound **30** (65 mg).

¹H NMR (400 MHz, DMSO-*d*₆) δ 12.43 (s, 1H), 10.19 (s, 1H), 9.35 (s, 1H), 8.96 - 8.74 (m, 1H), 8.13 (s, 1H), 8.00 (d, *J* = 7.5 Hz, 1H), 7.91 (d, *J* = 7.9 Hz, 1H), 7.64 (s, 1H), 7.55 (s, 1H), 7.35 (t, *J* = 7.7 Hz, 1H).

ESI-MS:m/z 296.1[M+H]⁺.

### Example 31: Synthesis of Compound 31

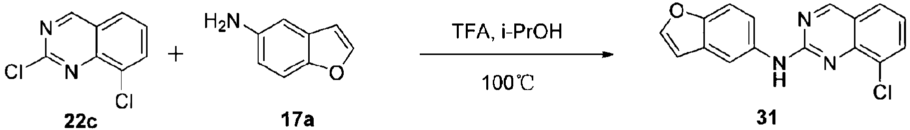

In a sealed tube, compound **22c** (500 mg, 1.0 eq), compound **17a** (84 mg, 0.25 eq, Bide Pharmatech), and TFA (223 mg, 1.2 eq) were added. The mixture was dissolved in 3 mL of isopropanol, and the system was heated and reacted at 100°C overnight. After completion of the reaction, the reaction liquid was concentrated, and 10 mL of water was added. An appropriate amount of K₂CO₃ was used to adjust the pH to ~8. The mixture was extracted with dichloromethane (20 mL × 3), and the organic phases were combined, washed with saturated NaCl solution, dried over anhydrous Na₂SO₄, and concentrated under vacuum to afford the crude product. Purification by column chromatography (PE/EA = 100:1-10:1, v/v) to afford compound **31** (100 mg).

¹H NMR (400 MHz, DMSO-*d*₆) δ 10.21 (s, 1H), 9.36 (d, *J* = 1.8 Hz, 1H), 8.68 (s, 1H), 8.03 - 7.95 (m, 2H), 7.94 - 7.85 (m, 2H), 7.57 (d, *J* = 8.9 Hz, 1H), 7.36 (t, *J* = 7.8 Hz, 1H), 7.03 - 6.92 (m, 1H).

ESI-MS:m/z 296.1[M+H]⁺.

### Example 32: Synthesis of Compound 32

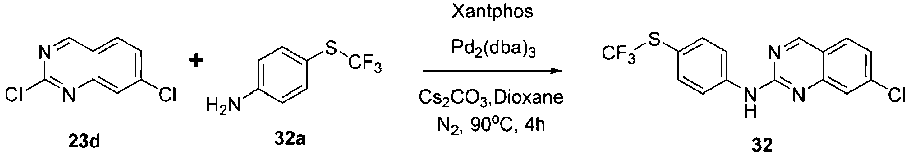

To a sealed tube were added compound **23d** (240 mg, 1.34 mmol), compound **32a** (344 mg, 1.34 mmol, Bide Pharmatech), Pd₂(dba)₃ (61.2 mg, 0.067 mmol), Xantphos (77.3 mg, 0.134 mmol), and Cs₂CO₃ (653 mg, 2 mmol). 1,4-dioxane (10 mL) was added as the solvent, and the atmosphere was replaced with nitrogen. The reaction was carried out at 90°C for 4 h. After completion of the reaction, water (30 mL) was added to the reaction liquid, and the mixture was extracted with DCM (20 mL × 4). The organic phases were combined, washed with saturated NaCl solution, dried over anhydrous Na₂SO₄, and concentrated under vacuum to afford the crude product. Purification by column chromatography (PE/EA = 100:1-3:1, v/v) to afford compound 32 (100 mg).

¹H NMR (400 MHz, DMSO-*d*₆) δ 10.43 (s, 1H), 9.42 - 9.38 (m, 1H), 8.18 - 8.13 (m, 2H), 8.01 (d. *J* = 8.6 Hz, 1H), 7.80 (d, *J* = 2.0 Hz, 1H), 7.69 - 7.65 (m, 2H), 7.47 (dd, *J* = 8.6, 2.0 Hz, 1H).

ESI-MS:m/z 356.1[M+H]⁺ .

### Example 33: Synthesis of Compound 33

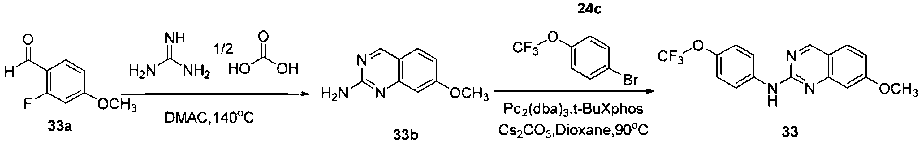

The preparation method of the above compound **33b** was as follows:
In a sealed tube, compound **33a** (3.8 g, 1.0 eq, Energy Chemical), guanidine carbonate (17.77 g, 4.0 eq, Bide Pharmatech), and 40 mL DMAC were added, and the mixture was heated and reacted at 140°C for 4 h. After TLC confirmed the completion of the reaction, the mixture was cooled to room temperature, poured into ice water, and stirred in an ice-water bath for 20 min. The mixture was filtered by suction, and the filter cake was oven-dried to afford compound **33b** (3.0 g).

### Synthesis of Compound 33:

In a sealed tube, compound **33b** (200 mg, 1.0 eq), compound **24c** (276 mg, 1.0 eq, Bide Pharmatech), Pd₂(dba)₃ (104 mg, 0.1 eq), tBuXphos (146 mg, 0.3 eq), and cesium carbonate (744 mg, 2.0 eq) were added, followed by the addition of 5 mL of dioxane. The mixture was subjected to argon replacement for protection, and heated to 90°C to react overnight. After completion of the reaction, 30 mL of water was added to the reaction liquid, and extraction was performed with EA (20 mL × 4). The organic phases were combined, washed with saturated NaCl solution, dried over anhydrous Na₂SO₄, and concentrated. The residue was purified by column chromatography (PE/EA = 100:1-9:1, v/v). The concentrated solid was slurried to afford compound 33 (300 mg).

¹H NMR (400 MHz, DMSO-*d*₆) δ 9.99 (s, 1H), 9.15 (s, 1H), 8.17 - 8.04 (m, 2H), 7.83 (d, *J* = 8.8 Hz, 1H), 7.40 - 7.29 (m, 2H), 7.13 - 6.95 (m, 2H), 3.94 (s, 3H).

ESI-MS:m/z 336.0[M+H]⁺.

### Example 34: Synthesis of Compound 34

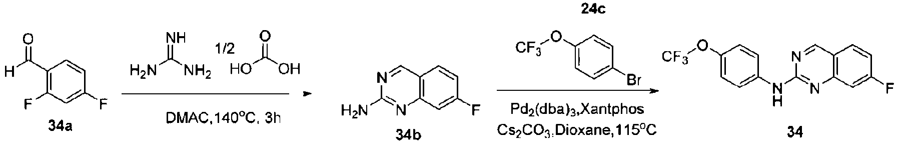

The preparation method of the above compound **34b** was as follows:
Compound **34a** (5 g, 35.19 mmol, Bide Pharmatech) and guanidine carbonate (25.36 g, 140.75 mmol, Bide Pharmatech) were added to a sealed tube, followed by the addition of 30 mL DMAC as solvent, and the reaction was carried out at 140°C for 6 h. After completion of the reaction, the reaction liquid was added dropwise to ice water, stirred to precipitate a solid, and the filter cake was filtered by suction and oven-dried to afford crude product compound **34b** (3.1 g).

### Synthesis of Compound 34:

To a sealed tube were added compound **34b** (600 mg, 3.68 mmol), compound **24c** (886 mg, 3.68 mmol, Bide Pharmatech), Pd₂(dba)₃ (168.4 mg, 0.184 mmol), Xantphos (212.8 mg, 0.368 mmol), and Cs₂CO₃ (1797 mg, 5.52 mmol). 1,4-dioxane (15 mL) was added as the solvent, and the atmosphere was replaced with Ar. The reaction was carried out at 100°C for 6 h. After completion of the reaction, water (30 mL) was added to the reaction liquid, and the mixture was extracted with EA (20 mL × 4). The organic phases were combined, washed with saturated NaCl solution, dried over anhydrous Na₂SO₄, and concentrated under vacuum to afford the crude product. Purification by column chromatography (DCM/McOH = 100:1-10:1, v/v) to afford compound **34** (100 mg).

¹H NMR (400 MHz, DMSO-*d*₆) δ 10.18 (s, 1H), 9.33 (s, 1H), 8.06 (t, *J* = 7.1 Hz, 3H), 7.45 - 7.28 (m, 4H) .

ESI-MS:m/z 324.4[M+H]⁻ .

### Example 35: Synthesis of Compound 35

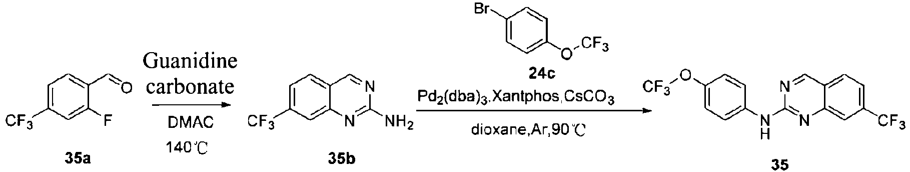

The preparation method of the above compound **35b** was as follows:
In a sealed tube, compound **35a** (1 g, 1.0 eq, Energy Chemical), guanidine carbonate (3.76 g, 4.0 eq, Bide Pharmatech), and 10 mL DMAC were added, and the mixture was stirred and reacted at 140°C for 4 h. After completion of the reaction, the mixture was cooled to room temperature, poured into ice water, and stirred in an ice-water bath for 20 min. The mixture was filtered by suction and oven-dried to afford 940 mg of compound **35b** (940 mg).

### Synthesis of Compound 35:

Compound **35b** (940 mg, 1.0 eq), compound **24c** (1.17 g, 1.1 eq, Bide Pharmatech), Pd₂(dba)₃ (404 mg, 0.1 eq), Xantphos (765 mg, 0.3 eq), and cesium carbonate (2.87 g, 2.0 eq) were added to a sealed tube, followed by the addition of 10 mL of dioxane. The mixture was subjected to argon replacement for protection, and the reaction was carried out at 90°C overnight. After completion of the reaction, the dioxane was evaporated to dryness. Water (30 mL) was added, and the mixture was extracted with ethyl acetate (20 mL × 3). The organic phases were combined, washed with saturated NaCl solution, dried over anhydrous Na₂SO₄, and concentrated under vacuum to afford the crude product. Purification by column chromatography (PE/EA = 100:1-9:1, v/v) and the product was slurried to afford compound **35** (200 mg).

¹H NMR (400 MHz, DMSO-*d*₆) δ 10.36 (s, 1H), 9.50 (d, *J* = 0.9 Hz, 1H), 8.19 (d, *J* = 8.3 Hz, 1H), 8.16 - 8.09 (m, 2H), 8.05 - 7.97 (m, 1H), 7.67 (dd, *J* = 8.4, 1.8 Hz, 1H), 7.39 - 7.32 (m, 2H) .

ESI-MS:m/z 374.0[M+H]⁺ .

### Example 36: Synthesis of Compound 36

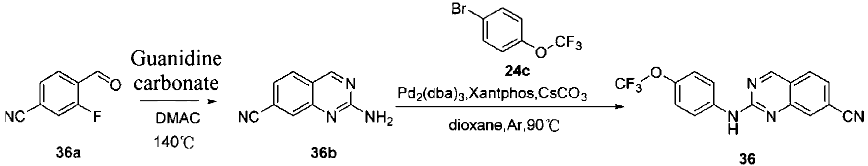

The preparation method of the above compound **36b** was as follows:
Compound **36a** (2 g, 13.41 mmol, Bide Pharmatech) and guanidine carbonate (9.67 g, 53.65 mmol, Bide Pharmatech) were added to a sealed tube, followed by the addition of DMAC (20 mL) as solvent, and the reaction was carried out at 140°C for 6 h. After completion of the reaction, the reaction liquid was added dropwise to ice water, stirred to precipitate a solid, and the filter cake was filtered by suction and oven-dried to afford crude product compound **36b** (1.7 g).

ESI-MS:m/z 171.2[M+H]⁺.

### Synthesis of Compound 36:

To a sealed tube were added compound **36b** (400 mg, 2.35 mmol), compound **24c** (566 mg, 2.35 mmol, Bide Pharmatech), Pd₂(dba)₃ (108 mg, 0.12 mmol), Xantphos (136 mg, 0.24 mmol), and Cs₂CO₃ (1149 mg, 3.53 mmol). 1,4-dioxane (15 mL) was added as the solvent, and the atmosphere was replaced with Ar. The reaction was carried out at 100°C for 6 h. After completion of the reaction, water (30 mL) was added to the reaction liquid, and the mixture was extracted with EA (20 mL × 4). The combined organic extracts were separated, washed with saturated NaCl solution, dried over anhydrous Na₂SO₄, and concentrated under vacuum to afford the crude product. Purification by column chromatography (PE/DCM = 100:1-2:1, v/v) to afford compound **36** (100 mg).

¹H NMR (400 MHz, DMSO-*d*₆) δ 10.37 (s, 1H), 9.48 (s, 1H), 8.22 - 8.18 (m, 1H), 8.11 (dd, *J* = 14.4, 8.6 Hz, 3H), 7.72 (s, 1H), 7.35 (d, *J =* 8.7 Hz, 2H) .

ESI-MS:m/z 331.2[M+H]⁻ .

### Example 37: Synthesis of Compound 37

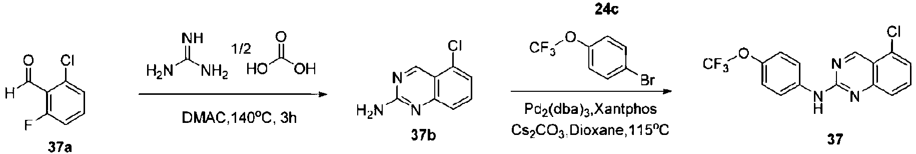

The preparation method of the above compound **37b** was as follows:
Compound **37a** (3.0 g, 18.9 mmol, Bide Pharmatech) was dissolved in DMAC (10 mL), 4 times as much as guanidine carbonate was added, and the mixture was reacted at 140°C for 3 hours. TLC monitoring indicated no remaining starting material, heating was stopped, and the mixture was cooled to room temperature, followed by addition of 100 mL of water and cooling at 0°C overnight. The mixture was filtered by suction, oven-dried to afford compound **37b** (2.1 g), a yellow solid, which was used directly in the next step without purification. Synthesis of Compound **37:**
To a round-bottom flask were sequentially added **37b** (179 mg, 1.0 mmol), dioxane (75 mL), **24c** (240 mg, 1.0 mmol, Bide Pharmatech), Pd₂(dba)₃ (46 mg, 0.05 mmol), cesium carbonate (480 mg, 1.5 mmol), and Xantphos (29 mg, 0.05 mmol). The mixture was then heated to 115°C under nitrogen protection and reacted for 6 h. The reaction was stopped, cooled to room temperature, and the solvent was evaporated under reduced pressure. To the residue was added water (20 mL), extracted 3 times with ethyl acetate (3 x 20 mL), the organic phases were combined, then washed with saturated brine, dried over anhydrous sodium sulfate, and then concentrated. The crude product was subjected to silica gel column chromatography (PE/EA = 4:1-2:1, v/v) to afford the yellow solid compound **37** (140 mg).

¹H NMR (400 MHz, DMSO-*d*₆) δ 10.34 (s, 1H), 9.50 (s, 1H), 8.09 (d, *J* = 8.6 Hz, 2H), 7.81 (t, *J* = 8.0 Hz, 1H), 7.67 (d, *J* = 8.5 Hz, 1H), 7.51 (d, *J* = 7.5 Hz, 1H), 7.36 (d, *J* = 8.6 Hz, 2H).

ESI-MS:m/z 340.7[M+H]⁺.

### Example 38: Synthesis of Compound 38

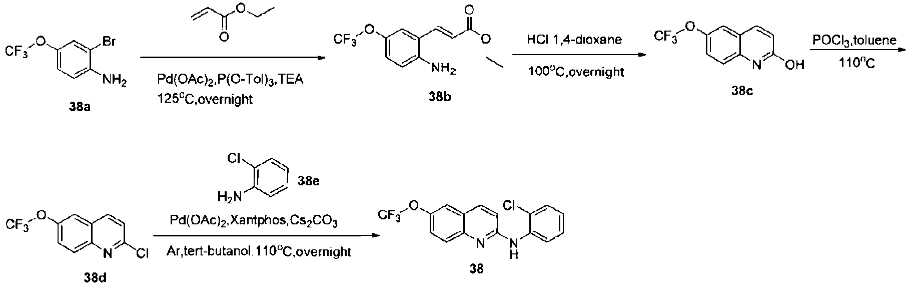

The preparation method of the above compound **38b** was as follows:
Compound **38a** (2 g, 1.0 eq, Bide Pharmatech), compound ethyl acrylate (939 mg, 1.2 eq, Energy Chemical), palladium acetate (88 mg, 0.05 eq), tri(o-tolyl)phosphine (238 mg, 0.1 eq), and triethylamine (7.8 mL) were added to a sealed tube, and the mixture was heated to 125°C and reacted overnight. After completion of the reaction, 50 mL of water was added, and the mixture was extracted with dichloromethane (3 x 30 mL). The organic phases were combined, washed with saturated brine, dried over anhydrous sodium sulfate, and then concentrated. The crude product was purified by silica gel column chromatography (DCM/MeOH=100:1-10:1, v/v) to afford compound **38b** (1.8 g).

The preparation method of the above compound **38c** was as follows:
In a sealed tube, compound **38b** (1.8 g, 1.0 eq) was added, dissolved in 10 mL of 1,4-dioxane, followed by dropwise addition of 0.5 mL of concentrated hydrochloric acid, and the mixture was heated to 100°C and reacted overnight. After completion of the reaction, 30 mL of water was added, the pH was adjusted to greater than 7 with saturated aqueous NaHCO₃ solution, and the mixture was extracted 3 times with ethyl acetate (3 × 20 mL). The organic phases were combined, washed with saturated brine, dried over anhydrous sodium sulfate, and then concentrated. The crude product was slurried with hexane:ethyl acetate (10:1), and after suction filtration, compound 38c (1.0 g) was obtained.

The preparation method of the above compound **38d** was as follows:
To a 50 mL single-neck flask was added compound **38c** (1 g, 1.0 eq), followed by addition of 10 mL toluene and then phosphorus oxychloride (1.34 g, 2.0 eq, Energy Chemical). The mixture was heated to 100°C and reacted overnight. After completion of the reaction, phosphorus oxychloride and toluene were removed as much as possible by distillation under reduced pressure, 50 mL of water was added, and the mixture was extracted with dichloromethane (3 x 30 mL). The organic phases were combined, washed with saturated brine, dried over anhydrous sodium sulfate, and then concentrated to afford compound **38d** (670 mg).

### Synthesis of Compound 38:

In a sealed tube, compound **38d** (670 mg, 1.0 eq), compound **38e** (414 mg, 1.2 eq, Energy Chemical), Pd(OAc)₂ (61 mg, 0.1 eq), cesium carbonate (1.76 g, 2.0 eq), and Xantphos (470 mg, 0.3 eq) were added, followed by the addition of 10 mL tert-butanol. The mixture was thoroughly mixed, subjected to argon replacement for protection, and the sealed tube was closed. The system was heated and reacted at 110°C in an oil bath for 20 h. After completion of the reaction, 2 times as much as methanol was added, and the mixture was filtered by suction. The filter cake was washed with cold methanol. The filtrate was concentrated, and the residue was purified by column chromatography (PE:EA = 100:1-19:1, v/v), then slurried with petroleum ether to afford compound **38** (82 mg).

¹H NMR (400 MHz, DMSO-*d*₆) δ 8.92 (s, 1H), 8.31 (dd, J = 8.2, 1.6 Hz, 1H), 8.18 (d, *J =* 9.0 Hz, 1H), 7.81 (d, *J* = 2.7 Hz, 1H), 7.68 (d, *J* = 9.1 Hz, 1H), 7.57 - 7.47 (m, 2H), 7.42 - 7.31 (m, 2H), 7.13 (td, *J* = 7.7, 1.6 Hz, 1H).

ESI-MS:m/z 339.1[M+H]⁻.

### Example 39: Synthesis of Compound 39

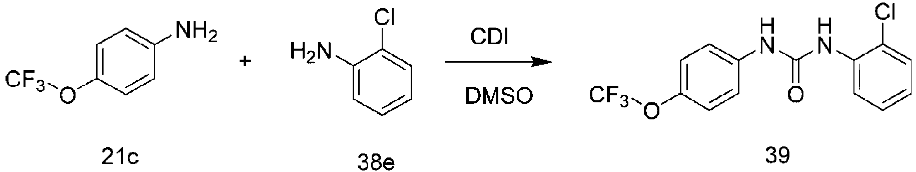

To a 25 mL single-necked flask were sequentially added compound **38e** (255 mg, Energy Chemical), DMSO (2 mL), and CDI (389 mg), and the mixture was stirred at room temperature. Compound **21c** (354 mg, Bide Pharmatech) was dissolved in DMSO (2 mL) and added dropwise to the reaction liquid, followed by reaction for 0.5 h. After completion of the reaction, the mixture was quenched with 1N HCl (16 mL) and extracted with ethyl acetate (15 mL × 3). The organic phases were combined, washed with saturated brine (10 mL) and dried over anhydrous sodium sulfate. The mixture was filtered by suction, and the filtrate was desolvated under vacuum to afford the crude product. The crude product was purified by column chromatography (eluent: PE/EA=10/1, v/v), to afford compound **39** (115 mg) as a white solid.

¹H NMR (400MHz, DMSO-*d6*) *δ*(ppm) 9.58 (s, 1H), 8.33 (s, 1H), 8.15 (dd, J = 8.3, 1.6 Hz, 1H), 7.67 - 7.52 (m, 2H), 7.47 (dd, J = 8.0, 1.5 Hz, 1H), 7.36 - 7.23 (m, 3H), 7.05 (td, J = 7.7, 1.6 Hz, 1H).

ESI-MS:m/z 331.1[M+H]⁻.

### Example 40: Synthesis of Compound 40

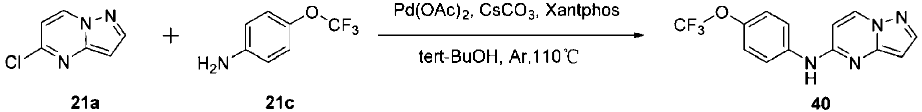

To a 75 mL sealed tube were added compound **21a** (1 g, 1.0 eq, Bide Pharmatech), compound 21c (1.38 g, 1.2 eq, Bide Pharmatech), Pd(OAc)₂ (73 mg, 0.05 eq), cesium carbonate (4.24 g, 2.0 eq), and Xantphos (1.38 g, 0.15 eq), followed by the addition of tert-butanol (12 mL). The mixture was thoroughly mixed, subjected to argon replacement for protection, and the sealed tube was closed. The system was heated to 110°C and reacted for 4 h. After completion of the reaction, methanol (20 mL) was added, and the mixture was filtered by suction. The filter cake was washed with methanol. The filtrate was concentrated, and then purified by column chromatography (PE:EA=100:1-2:1, v/v) to afford compound **40** (210 mg).

¹H NMR (400 MHz, CDCl₃) δ 8.39 (dd, *J* = 7.6, 0.9 Hz, 1H), 7.96 (d, *J* = 2.2 Hz, 1H), 7.70 - 7.60 (m, 2H), 7.27 (d, *J* = 1.2 Hz, 1H), 7.25 (q, *J* = 3.3 Hz, 2H), 6.88 (s, 1H), 6.32 - 6.23 (m, 2H) .

ESI-MS:m/z 295.1[M+H]⁻ .

### Example 41: Synthesis of Compound 41

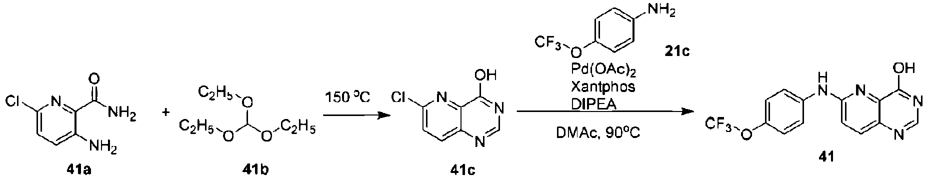

The preparation method of the above compound **41c** was as follows:
To a 75 mL sealed tube were successively added compound **41a** (1.00 g, Jiangsu Aikon) and compound **41b** (8.64 g, Energy Chemical), the mixture was heated to 150°C and reacted for 2 hours, precipitating a large amount of off-white solid. Cooled to 0°C, stirred for 15 min, and filtered by suction. The filter cake was rinsed with petroleum ether (100 mL) and filtered by suction to dryness. The filter cake was collected and dried under air flow at 50°C for 1 h to afford 0.95 g of the beige solid compound **41c** (400 mg).

### Synthesis of Compound 41:

To a 15 mL sealed tube were successively added compound **41c** (400 mg), DMAC (4 mL), compound **21c** (782 mg), Pd(OAc)₂ (50 mg), Xantphos (383 mg), and DIPEA (885 mg). The mixture was protected under argon and heated to 90°C to reacted for 15 h. Upon completion of the reaction, the mixture was diluted with ethyl acetate (30 mL), followed by addition of water (30 mL). The mixture was filtered by suction to remove solids. The filtrate was separated, the organic phase was retained, and the aqueous phase was extracted with ethyl acetate (15 mL × 3). The organic phases were combined. The organic phase was washed with saturated brine (10 mL) and dried over anhydrous sodium sulfate. The mixture was filtered by suction, the filtrate was desolvated under vacuum, and the residue was purified by column chromatography (eluent: EA/MeOH=30:1, v/v) to afford compound **41** as a beige solid (195 mg).

¹H-NMR (400 MHz. DMSO-*d*₆) *δ* (ppm): 12.55 - 12.17 (m, 1H), 9.80 (s, 1H), 8.09 (dd, *J* = 9.0, 1.7 Hz, 2H), 7.96 (d, *J* = 3.3 Hz, 1H), 7.88 (d, *J* = 8.9 Hz, 1H), 7.30 (dd, *J* = 14.7, 8.8 Hz, 3H).

ESI-MS:m/z 323.14[M+H]⁺.

### Example 42: Synthesis of Compound 42

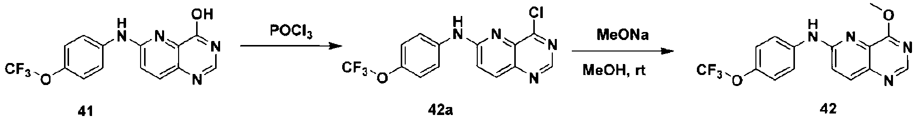

The preparation method of the above compound 42a was as follows:
To a 25 mL single-necked flask were sequentially added compound **41** (110 mg) and POCl₃ (2.6 g), heated to 90°C and reacted for 6 hours to obtain an orange-red solution, and the reaction was stopped. The reaction liquid was desolvated under vacuum to afford a dark red liquid, to which ether (20 mL) was added to precipitate a dark red solid. The solid was filtered by suction to afford dark red solid compound **42a** (100 mg).

### Synthesis of Compound 42:

To a 10 mL single-necked flask were sequentially added compound **42a** (100 mg), MeOH (1 mL), and MeONa (16 mg), and the reaction was performed at room temperature for 1 h. After completion of the reaction, the mixture was desolvated under vacuum, and the residue was purified by column chromatography (eluent: PE/EA = 1:2, v/v) to afford compound **42** as a yellow solid (65 mg).

¹H-NMR (400 MHz, DMSO- *d*₆) δ (ppm): 10.01 (s, 1H), 8.62 (s, 1H), 8.15 - 8.09 (m, 2H), 8.06 (d, J = 9.1 Hz, 1H), 7.43 (d, J = 9.1 Hz, 1H), 7.35 (d, J = 8.7 Hz, 3H), 4.13 (s, 3H) .

ESI-MS:m/z 323.14[M+H]⁺ .

### Example 43: Synthesis of Compound 43

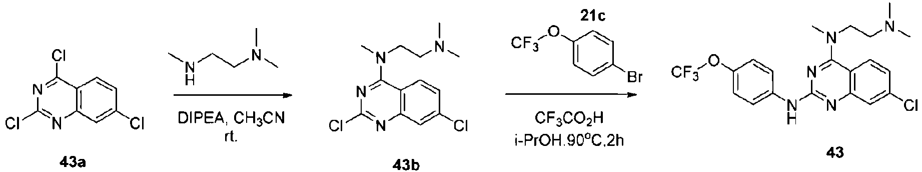

The preparation method of the above compound **43b** was as follows:
Compound **43a** (1.0 g, 4.28 mmol) was dissolved in acetonitrile (10 mL), and N,N,N'-trimethylethylenediamine (525 mg, 5.14 mmol) and DIPEA (1.66 g, 12.8 mmol) were added. The reaction was performed at room temperature for 12 h. TLC monitoring showed no remaining starting material. Water (30 mL) was added, and the mixture was extracted 3 times with ethyl acetate (3 × 20 mL). The organic phases were combined, then washed with saturated brine, dried over anhydrous sodium sulfate, and then concentrated. The residue was slurried with PE (3 mL) and isopropyl ether (5 mL), filtered by suction, and oven-dried to afford compound **43b** (890 mg) as a yellow solid.

### Synthesis of Compound 43:

Compound **43b** (870 mg, 2.9 mmol) and **21c** (620 mg, 3.5 mmol) were dissolved in isopropanol (10 mL), followed by addition of trifluoroacetic acid (1.66 g, 14.6 mmol). The mixture was then heated to 90°C under nitrogen protection and reacted for 2 h. The reaction was stopped, cooled to room temperature, and left to stand overnight. The mixture was filtered by suction, and the filter cake was washed with cold isopropanol. After drying, the yellow solid compound **43** (320 mg) was afforded.

¹H NMR (400 MHz, DMSO-*d*₆) δ 11.24 (s, 1H), 8.26 (d, *J =* 9.0 Hz, 1H), 7.79 (d, *J =* 8.5 Hz, 2H), 7.62 (d, *J =* 2.2 Hz, 1H), 7.42 (d, *J* = 8.5 Hz, 2H), 7.40 - 7.33 (m, 1H), 4.14 (t, *J* = 6.8 Hz, 2H), 3.58 (s. 3H), 3.42 (p, *J =* 5.5 Hz, 2H), 2.71 (d, *J =* 3.2 Hz, 6H) .

ESI-MS:m/z 440.1[M+H]⁻ .

### Example 44: Synthesis of Compound 44

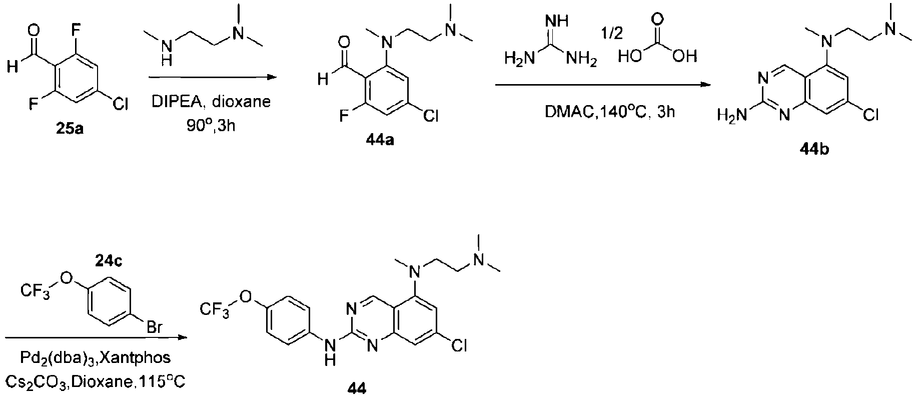

The preparation method of the above compound **44a** was as follows:
Compound **25a** (1.45 g, 8.21 mmol, Bide Pharmatech) was dissolved in 1,4-dioxane (20 mL), followed by addition of N,N,N'-trimethylethylenediamine (923 mg, 9.03 mmol, Energy Chemical) and DIPEA (2.12 g, 16.4 mmol, Sinopharm). The mixture was heated to 90°C and reacted for 3 h. TLC monitoring indicated no remaining starting material. Water (60 mL) was added, and the mixture was extracted 3 times with ethyl acetate (3 × 30 mL). The organic phases were combined, then washed with saturated brine, dried over anhydrous sodium sulfate, and then concentrated. The residue was purified by column chromatography (DCM/MeOH = 10:1, v/v) to afford compound **44a** (1.63 g).

### Synthesis of the above compound 44b:

Compound **44a** (1.6 g, 5.36 mmol) and guanidine carbonate (3.86 g, 21.4 mmol, Bide Pharmatech) were added to a sealed tube, followed by the addition of 15 mL of DMAC as the solvent, and the reaction was carried out at 140°C for 3 h. After completion of the reaction, the reaction liquid was added dropwise to ice water, stirred to precipitate a solid, and the filter cake was filtered by suction and oven-dried to afford crude product compound **44b** (0.98 g).

### Synthesis of Compound 44:

Compound **44b** (960 mg, 3.44 mmol), compound **24c** (826 mg, 3.44 mmol), Pd₂(dba)₃ (157 mg, 0.17 mmol), Xantphos (98 mg, 0.17 mmol), and Cs₂CO₃ (1.6 g, 5.16 mmol) were added to a sealed tube, followed by the addition of 1,4-dioxane (15 mL) as the solvent. The reaction was carried out at 115°C under argon protection for 6 h. After completion of the reaction, water (30 mL) was added to the reaction liquid, and the mixture was extracted with EA (20 mL × 4). The organic phases were combined, washed with saturated NaCl solution and dried over anhydrous Na₂SO₄, then concentrated under vacuum to afford the crude product. Purification by column chromatography (DCM/MeOH = 100:1-10:1, v/v) to afford compound **44** (200 mg).

¹H NMR (400 MHz, DMSO-*d*₆) δ 10.19 (s, 1H), 9.54 (s, 1H), 8.10 - 8.03 (m, 2H), 7.39 - 7.28 (m, 3H), 7.01 (d, *J* = 1.9 Hz, 1H), 3.53 (t, *J* = 6.4 Hz, 2H), 3.40 - 3.39 (m, 2H), 2.94 (s, 3H), 2.78 (d, *J* = 4.8 Hz, 6H) .

ESI-MS:m/z 440.1[M+H]⁻ .

### Example 45: Synthesis of Compound 45

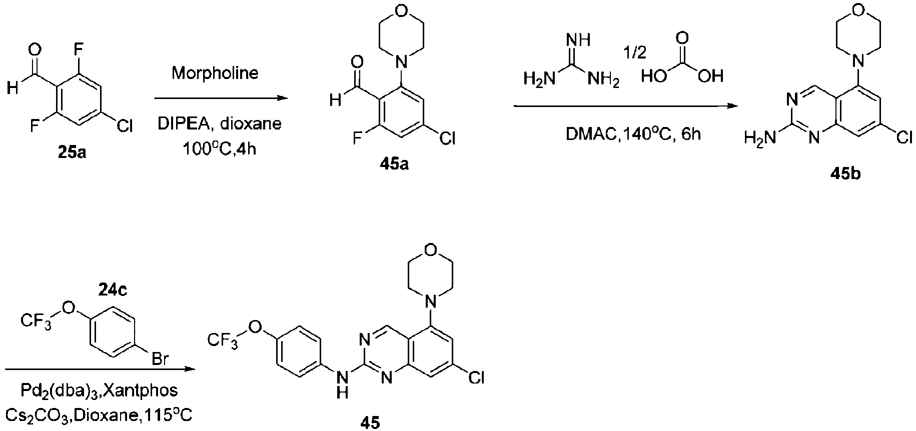

The preparation method of the above compound **45a** was as follows:
Compound **25a** (2.0 g, 11.3 mmol) was dissolved in 1,4-dioxane (20 mL), followed by addition of morpholine (1.09 g, 12.5 mmol) and DIPEA (2.93 g, 22.7 mmol). The mixture was heated to 100°C and reacted for 4 h. TLC monitoring indicated no remaining starting material. Water (60 mL) was added, and the mixture was extracted 3 times with ethyl acetate (3 x 30 mL). The organic phases were combined, then washed with saturated brine, dried over anhydrous sodium sulfate, and then concentrated to afford the yellow oily compound **45a** (1.52 g).

### Synthesis of the above compound 45b:

Compound **45a** (1.5 g, 6.17 mmol) and guanidine carbonate (4.4 g, 24.4 mmol) were added to a sealed tube, followed by the addition of 20 mL DMAC as the solvent, and the reaction was carried out at 140°C for 6 h. After completion of the reaction, 60 mL water was added, and the mixture was extracted 3 times with ethyl acetate (3 x 30 mL). The organic phases were combined, then washed with saturated brine, dried over sodium sulfate, and then concentrated. The residue was slurried with PE (10 mL) and EA (1 mL), filtered by suction, and oven-dried to afford compound **45b** (1.1 g) as a yellow solid.

### Synthesis of Compound 45:

Compound **45b** (1.05 g, 3.97 mmol), compound **24c** (953 mg, 3.97 mmol), Pd₂(dba)₃ (185 mg, 0.20 mmol), Xantphos (117 mg, 0.20 mmol), and Cs₂CO₃ (1.94 g, 5.97 mmol) were added to a sealed tube, followed by the addition of 1,4-dioxane (20 mL) as the solvent. The reaction was carried out at 115°C under argon protection for 6 h. After completion of the reaction, water (60 mL) was added to the reaction liquid, and extraction was performed with EA (30 mL × 4). The organic phases were combined, washed with saturated NaCl solution, dried over anhydrous Na₂SO₄, and concentrated under vacuum to afford the crude product. The crude product was slurried with PE (10 mL) and EA (2 mL), and the resulting solid was filtered by suction. The filter cake was further purified by column chromatography (DCM/MeOH = 100:1-10:1, v/v) to afford compound **45** (350 mg).

¹H NMR (400 MHz, DMSO-*d*₆) δ 10.16 (s, 1H), 9.32 (s, 1H), 8.05 (d, *J* = 8.6 Hz, 2H), 7.34 (d, *J* = 4.2 Hz, 2H), 7.31 (s, 1H), 6.87 (s, 1H), 3.86 (t, *J* = 4.4 Hz, 4H), 3.12 (t, *J* = 4.5 Hz, 4H) .

ESI-MS:m/z 425.1 [M+H]⁻.

### Example 46: Synthesis of Compound 46

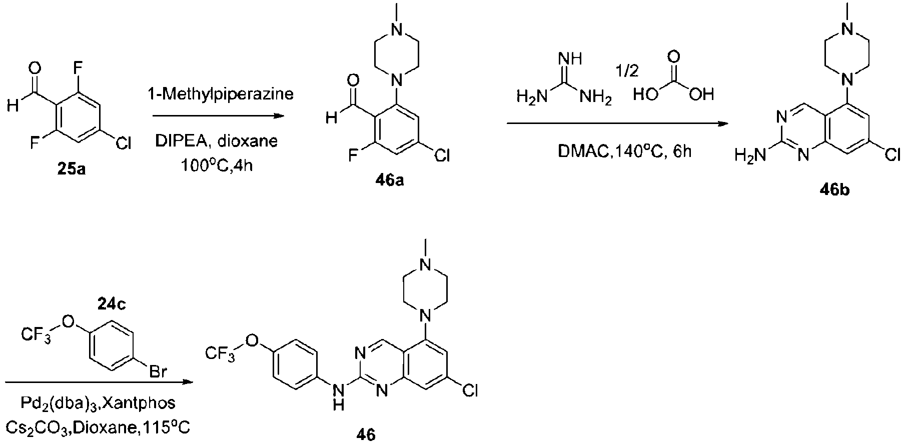

The preparation method of the above compound **46a** was as follows:
Compound **25a** (2.0 g, 11.3 mmol) was dissolved in 1,4-dioxane (20 mL), followed by addition of methylpiperazine (1.25 g, 12.5 mmol) and DIPEA (2.93 g, 22.7 mmol). The mixture was heated to 100°C and reacted for 4 h. TLC monitoring indicated no remaining starting material. Water (60 mL) was added, and the mixture was extracted 3 times with ethyl acetate (3 x 30 mL). The organic phases were combined, then washed with saturated brine, dried over anhydrous sodium sulfate, and then concentrated to afford the yellow oily compound **46a** (1.50 g).

### Synthesis of the above compound 45b:

Compound **46a** (1.5 g, 6.17 mmol) and guanidine carbonate (4.5 g, 24.9 mmol) were added to a sealed tube, followed by the addition of 20 mL DMAC as the solvent, and the reaction was carried out at 140°C for 6 h. After completion of the reaction, 60 mL water was added, and the mixture was extracted 3 times with ethyl acetate (3 x 30 mL). The organic phases were combined, then washed with saturated brine, dried over sodium sulfate, and then concentrated. The residue was slurried with PE (10mL), EA (1 mL) and i-PrOH (1 mL), filtered by suction, and oven-dried to afford compound **46b** (1.0 g) as a yellow solid.

### Synthesis of Compound 46:

To a sealed tube were added compound **46b** (1.0 g, 3.61 mmol), compound **24c** (960 mg, 3.99 mmol), Pd₂(dba)₃ (185 mg, 0.20 mmol), Xantphos (117 mg, 0.20 mmol), and Cs₂CO₃ (1.94 g, 5.97 mmol). 1,4-dioxane (20 mL) was added as the solvent, and the reaction was carried out at 115°C under argon protection for 6 h. After completion of the reaction, water (60 mL) was added to the reaction liquid, followed by extraction with EA (30 mL × 4). The organic phases were combined, washed with saturated NaCl solution and dried over anhydrous Na₂SO₄, then concentrated under vacuum to afford the crude product. The crude product was slurried with a mixed solvent of PE (10 mL), EA (2 mL), DCM (1 mL), and i-PrOH (1 mL), filtered by suction, and oven-dried to afford compound **46** (410 mg) as a yellow solid.

¹H NMR (400 MHz, DMSO-*d*₆) δ 10.21 (s, 1H), 9.35 (s, 1H), 8.06 (d, *J* = 8.8 Hz, 2H), 7.42 (d, *J* = 1.7 Hz, 1H), 7.33 (d, *J* = 8.6 Hz, 2H), 7.00 (d, *J* = 1.9 Hz, 1H), 3.59 (dd, *J* = 12.4, 5.2 Hz, 4H), 3.49 - 3.41 (m, 2H), 3.19 (t, *J* = 12.0 Hz, 2H), 2.92 (s, 3H) .

ESI-MS:m/z 438.1[M+H]⁺.

### Example 47: Synthesis of Compound 47

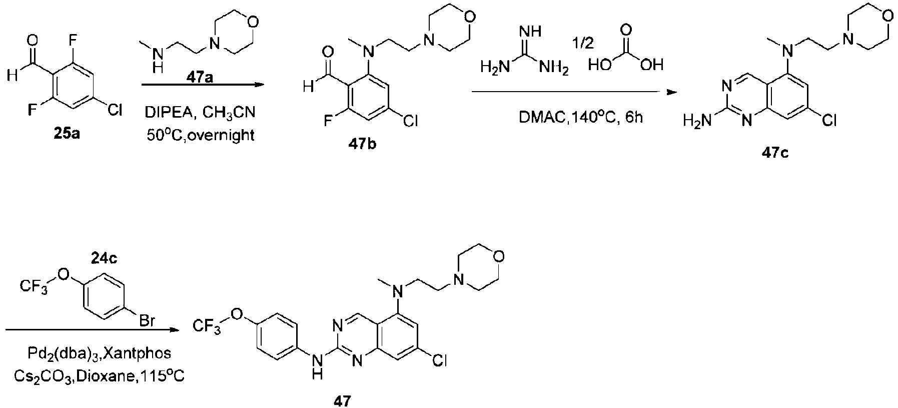

The preparation method of the above compound **47b** was as follows:
Compound **25a** (1.46 g, 8.27 mmol) was dissolved in acetonitrile (20 mL), and then compound **47a** (1.25 g, 8.67 mmol) and DIPEA (2.14 g, 16.6 mmol) were added. The mixture was heated to 50°C and reacted overnight. TLC monitoring indicated no remaining starting material. The solvent was removed by distillation under reduced pressure, and the residue was purified by column chromatography (PE/EA=100:1-3:1, v/v) to afford compound **47b** (1.90 g).

### Synthesis of the above compound 47c:

Compound **47b** (1.9 g, 6.32 mmol) and guanidine carbonate (4.5 g, 24.9 mmol) were added to a sealed tube, followed by the addition of 20 mL DMAC as the solvent, and the reaction was carried out at 140°C for 6 h. After completion of the reaction, 100 mL water was added, and the mixture was extracted 3 times with ethyl acetate (3 × 50 mL). The organic phases were combined, then washed with saturated brine, dried over anhydrous sodium sulfate, and then concentrated. Purification by column chromatography (PE/EA = 3:1 to EA/MeOH = 20:1, v/v) to afford the compound **47c** (1.7 g) as a yellow solid.

### Synthesis of Compound 47:

To a sealed tube were added compound **47c** (1.7 g, 5.28 mmol), compound **24c** (1.4 g, 5.83 mmol), Pd₂(dba)₃ (242 mg, 0.26 mmol), Xantphos (458 mg, 0.79 mmol), and Cs₂CO₃ (3.4 g, 10.5 mmol), followed by the addition of 1,4-dioxane (20 mL) as the solvent. The mixture was replaced with Ar and reacted at 115°C for 3 h. After completion of the reaction, water (60 mL) was added to the reaction liquid, and the mixture was extracted with EA (30 mL × 4). The organic phases were combined, washed with saturated NaCl solution, dried over anhydrous Na₂SO₄, and concentrated under vacuum to afford the crude product. Purification by column chromatography (PE/EA = 100:1-1:1, v/v) to afford 2.1 g of crude product, which was then slurried with a mixed solvent of PE (10 mL), EA (10 mL), and MeOH (0.3 mL). The resulting solid was filtered by suction, and oven-dried to afford compound **47** (1.4 g) as a yellow solid.

¹H NMR (400 MHz, DMSO-*d*₆) δ 10.11 (s, 1H), 9.56 (s, 1H), 8.12 - 8.00 (m, 2H), 7.32 (d, *J* = 8.7 Hz, 2H), 7.22 (d, *J* = 1.8 Hz, 1H), 6.84 (d, *J* = 1.9 Hz, 1H), 3.52 (t, *J* = 4.6 Hz, 4H), 3.34 (d, *J* = 6.5 Hz, 3H), 2.92 (s, 3H), 2.61 (t, *J* = 6.2 Hz, 2H), 2.38 (t, *J* = 4.6 Hz, 4H).

ESI-MS:m/z 482.4[M+H]⁺.

### Example 48: Synthesis of Compound 48

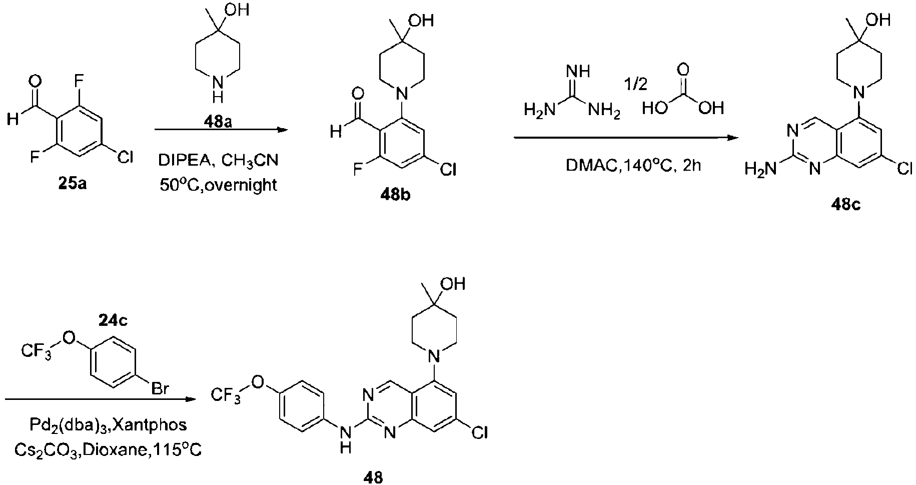

The preparation method of the above compound **48b** was as follows:
Compound **25a** (4.7 g, 26.6 mmol) was dissolved in acetonitrile (30 mL), and compounds **48a** (3.3 g, 28.6 mmol) and DIPEA (6.9 g, 53.5 mmol) were added. The mixture was heated to 50°C and reacted overnight. TLC monitoring indicated no remaining starting material. The solvent was removed by distillation under reduced pressure, and purification by column chromatography (DCM) to afford the compound **48b** (5.8 g) as a yellow solid.

### Synthesis of the above compound 48c:

Compound **48b** (5.8 g, 21.3 mmol) and guanidine carbonate (15.4 g, 85.5 mmol) were added to a sealed tube, followed by the addition of 70 mL DMAC as solvent, and the reaction was carried out at 140°C for 2 h. After completion of the reaction, 200 mL water was added, and the mixture was extracted 3 times with ethyl acetate (3 × 100 mL). The organic phases were combined, then washed with saturated brine, dried over anhydrous sodium sulfate, and then concentrated. Purification by column chromatography (PE/EA = 100:1-8:1, v/v) to afford the compound **48c** (5 g) as a yellow solid.

### Synthesis of Compound 48:

Compound **48c** (1.3 g, 4.44 mmol), compound **24c** (1.2 g, 5.0 mmol, Bide Pharmatech), Pd₂(dba)₃ (203 mg, 0.22 mmol), Xantphos (381 mg, 0.66 mmol), and Cs₂CO₃ (2.89 g, 8.89 mmol) were added to a sealed tube, followed by the addition of 1,4-dioxane (20 mL) as the solvent. The reaction was carried out at 115°C under argon protection for 3 h. After completion of the reaction, water (60 mL) was added to the reaction liquid, and the mixture was extracted with EA (30 mL × 4). The organic phases were combined, washed with saturated NaCl solution, dried over anhydrous Na₂SO₄, and concentrated under vacuum to afford the crude product. Purification by column chromatography (PE/EA = 100:1-3:1, v/v) to afford compound **48** (1.0 g).

¹H NMR (400 MHz, DMSO-*d*₆) δ 10.16 (s, 1H), 9.22 (s, 1H), 8.15 - 8.00 (m, 2H), 7.36 - 7.30 (m, 3H), 7.28 (d, *J* = 1.7 Hz, 1H), 4.39 (s, 1H), 3.14 (td, *J* = 12.0, 8.2 Hz, 4H), 1.90 - 1.61 (m, 4H), 1.23 (s, 3H).

ESI-MS:m/z 453.2[M+H]⁺.

### Example 49: Synthesis of Compound 49

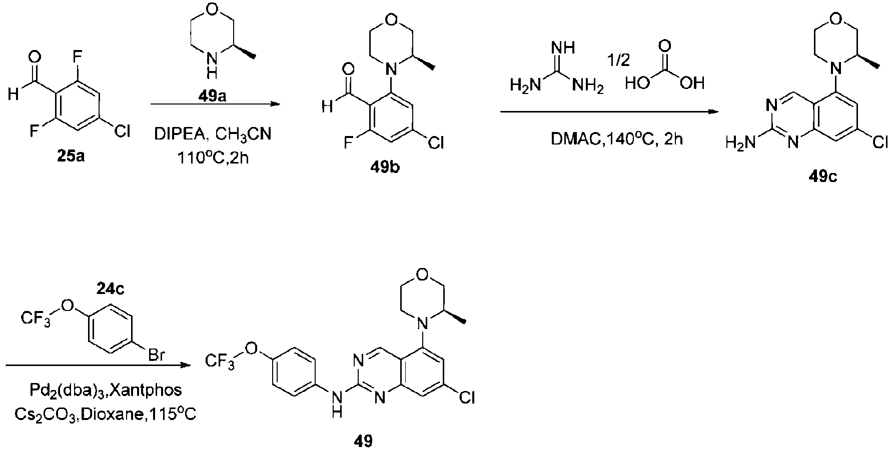

The preparation method of the above compound **49b** was as follows:
Compound **25a** (1.58 g, 8.95 mmol) was dissolved in acetonitrile (30 mL), and compound **49a** (1.0 g, 9.88 mmol) and DIPEA (2.3 g, 17.8 mmol) were added. The mixture was heated to 110°C and reacted for 2 h. TLC monitoring indicated no remaining starting material. The solvent was removed by distillation under reduced pressure, and the residue was purified by column chromatography (PE/EA=100:1-8:1, v/v) to afford compound **49b** (1.7 g) as a yellow solid.

### Synthesis of the above compound 49c:

Compound **49b** (1.7 g, 6.59 mmol) and guanidine carbonate (4.7 g, 26.1 mmol) were added to a sealed tube, followed by the addition of 20 mL DMAC as solvent, and the reaction was carried out at 140°C for 2 h. After completion of the reaction, 100 mL of water was added, and the mixture was extracted 3 times with ethyl acetate (3 x 60 mL). The organic phases were combined, then washed with saturated brine, dried over anhydrous sodium sulfate, and then concentrated to afford the crude solid compound **49c** (2.0 g).

### Synthesis of Compound 49:

Compound **49c** (2.0 g, 7.18 mmol), compound **24c** (1.8 g, 7.50 mmol), Pd₂(dba)₃ (296 mg, 0.32 mmol), Xantphos (560 mg, 0.97 mmol), and Cs₂CO₃ (4.2 g, 12.9 mmol) were added to a sealed tube, followed by the addition of 1,4-dioxane (40 mL) as the solvent. The mixture was replaced with Ar and reacted at 115°C for 4 h. After completion of the reaction, water (100 mL) was added to the reaction liquid, which was then extracted with EA (3 × 60 mL). The organic phases ere combined, washed with saturated NaCl solution, dried over anhydrous Na₂SO₄, and concentrated under vacuum to afford the crude product. Purification by column chromatography (PE/EA = 100:1-10:1, v/v) to afford compound **49** (1.2 g).

¹H NMR (400 MHz, DMSO-*d*₆) δ 10.21 (s, 1H), 9.43 (s, 1H), 8.06 (d, *J =* 8.8 Hz, 2H), 7.43 (d, *J* = 1.7 Hz, 1H), 7.33 (d, *J* = 8.6 Hz, 2H), 7.07 (d, *J* = 1.9 Hz, 1H), 4.02 - 3.70 (m, 3H), 3.48 (dd, *J* = 10.7, 5.1 Hz, 2H), 3.30 - 3.18 (m, 1H), 2.84 (ddd, *J* = 11.4, 6.9, 2.9 Hz, 1H), 0.85 (d, *J* = 5.6 Hz, 3H) .

ESI-MS:m/z 439.2[M+H]⁻.

### Example 50: Synthesis of Compound 50

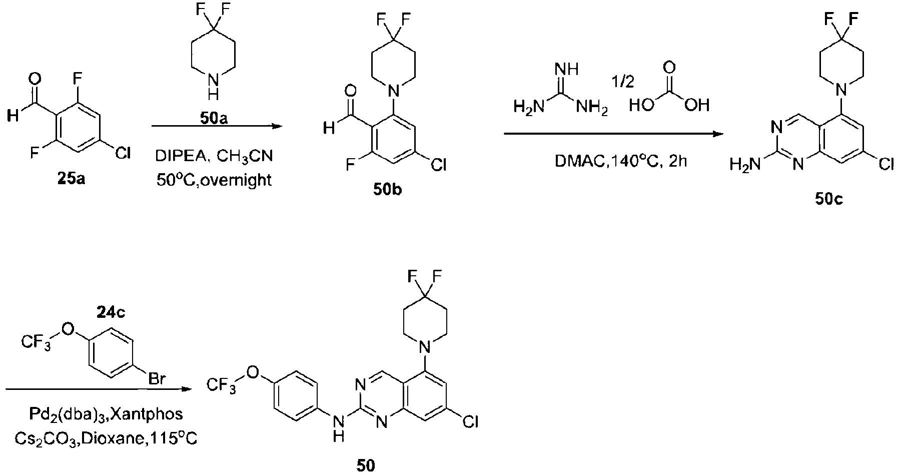

The preparation method of the above compound **50b** was as follows:
Compound **25a** (1.3 g, 7.36 mmol) was dissolved in acetonitrile (15 mL), and compound **50a** (1.0 g, 8.26 mmol) and DIPEA (1.9 g, 14.7 mmol) were added. The mixture was heated to 50°C and reacted overnight. TLC monitoring indicated no remaining starting material. The solvent was removed by distillation under reduced pressure, and the residue was purified by column chromatography (PE/EA=100:1-8:1, v/v) to afford compound **50b** (1.7 g) as a yellow solid.

### Synthesis of the above compound 50c:

Compound **50b** (1.7 g, 6.12 mmol) and guanidine carbonate (4.4 g, 24.4 mmol) were added to a sealed tube, 15 mL of DMAC was added as solvent, and the reaction was carried out at 140°C for 2 h. After completion of the reaction, 100 mL of water was added, and the mixture was extracted 3 times with ethyl acetate (3 x 100 mL). The organic phases were combined, then washed with saturated brine, dried over anhydrous sodium sulfate, and then concentrated to afford the crude compound **50c** (2.2 g).

### Synthesis of Compound 50:

Compound **50c** (2.0 g, 6.69 mmol), compound **24c** (1.8 g, 7.50 mmol), Pd₂(dba)₃ (306 mg, 0.33 mmol), Xantphos (581 mg, 1.0 mmol), and Cs₂CO₃ (4.4 g, 13.5 mmol) were added to a sealed tube, followed by the addition of 1,4-dioxane (30 mL) as the solvent. The reaction was carried out at 115°C under argon protection for 4 h. After completion of the reaction, water (60 mL) was added to the reaction liquid, and the mixture was extracted with EA (4 × 50 mL). The organic phases were combined, washed with saturated NaCl solution, dried over anhydrous Na₂SO₄, and concentrated under vacuum to afford the crude product. Purification by column chromatography (PE/EA = 100:1-10:1, v/v) to afford compound **50** (1.6 g).

¹H NMR (400 MHz, DMSO-*d*₆) δ 10.18 (s, 1H), 9.35 (s, 1H), 8.05 (d, *J =* 9.0 Hz, 2H), 7.40 - 7.28 (m, 3H), 6.95 (d, *J* = 1.9 Hz, 1H), 3.24 (t, *J* = 5.7 Hz, 4H), 2.28 (tt, *J =* 13.6, 5.0 Hz, 4H).

ESI-MS:m/z 459.2[M+H]⁺.

### Example 51: Synthesis of Compound 51

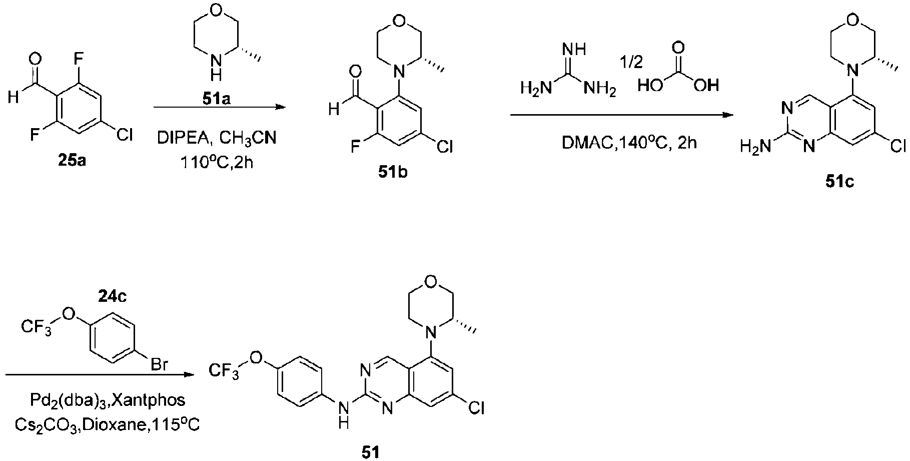

The preparation method of the above compound **51b** was as follows:
Compound **25a** (1.58 g, 8.95 mmol) was dissolved in acetonitrile (30 mL), and compound **49a** (1.0 g, 9.88 mmol) and DIPEA (2.3 g, 17.8 mmol) were added. The mixture was heated to 110°C and reacted for 2 h. TLC monitoring indicated no remaining starting material. The solvent was removed by distillation under reduced pressure, and the residue was purified by column chromatography (PE/EA=100:1-8:1, v/v) to afford solid compound **51b** (2.0 g).

### Synthesis of the above compound 51c:

Compound **51b** (2.0 g, 7.76 mmol) and guanidine carbonate (5.6 g, 31.1 mmol) were added to a sealed tube, followed by the addition of 20 mL DMAC as solvent, and the reaction was carried out at 140°C for 2 h. After completion of the reaction, 100 mL of water was added, and the mixture was extracted with ethyl acetate (3 x 80 mL). The organic phases were combined, then washed with saturated brine, dried over anhydrous sodium sulfate, and then concentrated to afford the crude compound **51c** (2.2 g).

### Synthesis of Compound 51:

Compound **51c** (2.2 g, 7.89 mmol), compound **24c** (2.1 g, 8.75 mmol), Pd₂(dba)₃ (361 mg, 0.39 mmol), Xantphos (685 mg, 1.18 mmol), and Cs₂CO₃ (5.1 g, 15.6 mmol) were added to a sealed tube, followed by the addition of 1,4-dioxane (40 mL) as the solvent. The reaction was carried out at 115°C under argon protection for 5 h. After completion of the reaction, water (100 mL) was added to the reaction liquid, and the mixture was extracted with EA (3 × 80 mL). The organic phases were combined, washed with saturated NaCl solution, dried over anhydrous Na₂SO₄, and concentrated under vacuum to afford the crude product. Purification by column chromatography (PE/EA = 100:1-10:1, v/v) to afford compound **51** (1.3 g).

¹H NMR (400 MHz, DMSO-*d*₆) δ 10.20 (s, 1H), 9.43 (s, 1H), 8.10 - 8.03 (m, 2H), 7.43 (d, *J* = 1.7 Hz, 1H), 7.33 (d, *J* = 8.7 IIz, 2H), 7.07 (d, *J* = 1.8 Hz, 1H), 3.97 (d, *J* = 8.4 Hz, 1H), 3.88 (ddd, *J =* 10.2, 6.9, 2.7 Hz, 1H), 3.77 (ddd, *J* = 10.9, 5.7, 2.9 Hz, 1H), 3.55 - 3.45 (m, 2H), 3.25 (dd, *J* = 11.1, 4.0 Hz, 1H), 2.84 (ddd, *J* = 11.5, 6.9, 2.9 Hz, 1H), 0.85 (d, *J =* 5.6 Hz, 3H).

ESI-MS:m/z 439.2[M+H]⁺.

### Biological Assay

### Test 1: Effect of the compound on miR124 upregulation

The effect of test compounds on miR124 expression levels was evaluated in human peripheral blood mononuclear cells (PBMCs). PBMC cells were thawed at 37°C, centrifuged at 400g for 10 min, and resuspended in RPMI1640 complete medium (containing 10% FBS), followed by incubation at 37°C + 5% CO2 for 24 h. Screening was performed using 6-well plates; cells were seeded at a density of 2.0×10⁶ cells/4 mL in 6-well plates and activated with RPMI1640 complete medium containing PHA-L (5 ug/mL) and IL-2 (40 U/mL) for 48 h. Test compounds (final concentration: 5 µM) were added for treatment at 37°C, 5% CO2 for 6 days, with medium replacement on day 3. The control group was treated with an equal volume of DMSO under the same conditions.

RNA Extraction: On day 6, cells were collected in a 15 mL centrifuge tube and centrifuged at 400g for 10 min, followed by supernatant removal. 500 µL Trizol was added to the cell pellet and thoroughly pipetted, then transferred to a 1 mL centrifuge tube (RNase-free) for lysis at room temperature for 15 min. Each tube was supplemented with 100 µL chloroform (Trizol:chloroform = 5:1 volume ratio), mixed thoroughly, and left at room temperature for approximately 15 min. Centrifugation was performed at 4°C and 12000g for 15 min, revealing distinct layers (from bottom to top: pink lower organic phase, white intermediate protein phase, and colorless upper aqueous phase). The upper RNA layer was aspirated into a clean 1.5 mL centrifuge tube (RNase-free), an equal volume of isopropanol was added, and left at room temperature for 10 min to precipitate RNA. Centrifugation at 4°C and 12000g for 10 min yielded a small white pellet at the tube bottom. 500 µL of 75% ethanol was added to wash the precipitate, let stand at room temperature for 5 min; centrifuged at 4°C and 7600g for 5 min; after the precipitate becomes transparent, RNase-free H2O was added to dissolve the precipitate, and the RNA sample concentration was measured.

Reverse transcription: the reverse transcription system for the above RNA is configured as detailed in Table 1:

**Table 1. RNA reverse transcription system**

| | |
|---|---|
| RNase-free ddH₂O | To 10 µL |
| 4 × gDNA wiper Mix | 2 µL |
| Template RNA | Total RNA: 1µg |

| The above mixture was heated at 42°C for 2 minutes | |
|---|---|
| The above mixture | 10 µL |
| Stem-loop primer (2 µM) | 1 µL |
| 10 × RT Mix | 2 µL |
| IIiScript II Enzyme Mix | 2 µL |
| RNase-free ddH₂O | To 20 µL |

The cDNA reaction cycling conditions: 25°C, 5 min; 50°C, 15 min; 85°C, 5 min.

Fluorescent quantitative PCR reaction: miRNA quantification was performed using SYBR Green I intercalating fluorescence method, with simultaneous detection of the housekeeping gene U6 transcript level as internal reference. The fluorescent quantitative PCR amplification reaction system was configured as detailed in Table 2:

**Table 2. Fluorescent quantitative PCR amplification system**

| | | | | |
|---|---|---|---|---|
| 2 × miRNA Universal SYBR qPCR Master Mix | | | 10 µL | |
| Specific Primer (10 µM) | | | 0.4 µL | |
| mQ Primer R (10 µM) | | | 0.4 µL | |
| Template DNA/cDNA² | | | 5 µL | |
| ddH₂O | | | To 20 µL | |

| PCR amplification cycle conditions | | | | |
|---|---|---|---|---|
| Stage 1 | Pre-denaturation | Reps: 1 | 95°C | 5min |
| Stage 1 | Cyclic reaction | Reps: 40 | 95°C | 10 sec |
| | | | 60°C | 30 sec |
| Stage 1 | Dissolution curve | Reps: 1 | 95°C | 15 sec |
| | | | 60°C | 60 sec |
| | | | 95°C | 15 sec |

Note: 1 represents the stem-loop primer GTCGTATCCAGTGCAGGGTCCGAGGTATTCG CACTGGATACGAC; 2 indicates cDNA template diluted 5-fold; 2 indicates cDNA template dil uted 5-fold.
(1) Using the protocol described above, the average fold change in miR-124 expression (compared to DMSO) was evaluated by relative quantification using PBMCs from different donors. The in vitro results are detailed in Table 3 below:

**Table 3. In vitro upregulation expression levels of miR124 in PBMCs**

| Molecule | Average fold change compared to DMSO treatment | | Number of donors |
|---|---|---|---|
| | Average value | SD | |
| DMSO | 1.00 | 0.00 | 3 |
| ABX464 | 6.91 | 4.35 | 3 |
| Compound 1 | 1.28 | -- | 1 |
| Compound 2 | 1.04 | -- | 1 |
| Compound 3 | 4.20 | -- | 1 |
| Compound 4 | 1.56 | -- | 1 |
| Compound 5 | 6.57 | 0.61 | 2 |
| Compound 6 | 7.18 | 0.65 | 2 |
| Compound 7 | 8.52 | 0.38 | 3 |
| Compound 8 (disclosed in Example 1 of PCT/CN2022/095441) | 1.69 | -- | 1 |
| Compound 9 | 6.00 | 3.59 | 3 |
| Compound 10 | 7.26 | 2.07 | 3 |
| | | | |
| Compound 12 | 3.87 | -- | 1 |
| Compound 13 | 4.38 | 0.59 | 3 |
| Compound 14 | 4.61 | -- | 1 |
| Compound 15 | 3.88 | -- | 1 |
| Compound 16 | 5.96 | 1.44 | 3 |
| Compound 17 | 9.49 | 0.46 | 3 |
| Compound 18 | 2.16 | -- | 1 |
| Compound 19 | 5.42 | 0.91 | 3 |
| Compound 20 | 3.36 | 0.54 | 2 |
| Compound 21 | 5.26 | -- | 1 |
| | | | |
| Compound 23 | 6.54 | 3.84 | 3 |
| Compound 24 | 5.07 | 1.20 | 2 |
| Compound 25 | 6.09 | 0.87 | 2 |
| Compound 26 | 9.34 | 0.45 | 2 |
| Compound 27 | 6.52 | 1.88 | 3 |
| Compound 28 | 8.66 | 0.56 | 2 |
| Compound 29 | 4.90 | -- | 1 |
| Compound 30 | 3.52 | -- | 1 |
| Compound 31 | 6.58 | 0.88 | 3 |
| Compound 32 | 6.68 | 1.61 | 3 |
| Compound 33 | 17.40 | -- | 1 |
| Compound 34 | 18.94 | -- | 1 |
| Compound 35 | 14.80 | -- | 1 |
| Compound 36 | 11.30 | -- | 1 |
| Compound 37 | 24.24 | -- | 1 |
| Compound 38 | 0.90 | -- | 1 |
| Compound 39 | 0.47 | -- | 1 |
| Compound 40 | 0.49 | -- | 1 |
| Compound 41 | 0.77 | -- | 1 |
| Compound 42 | 0.90 | -- | 1 |
| Compound 43 | 0.68 | -- | 1 |
| Compound 44 | 4.89 | 3.82 | 2 |
| Compound 45 | 8.29 | 3.22 | 2 |
| Compound 46 | 4.46 | -- | 1 |
| Compound 47 | 4.29 | -- | 1 |
| Compound 48 | 4.63 | -- | 1 |
| Compound 49 | 0.14 | -- | 1 |
| Compound 50 | 2.89 | -- | 1 |
| Compound 51 | 0.15 | -- | 1 |

| | | | |
|---|---|---|---|
| Note: The screening results of different batches in the table were normalized. | | | |

### Test 2: Preventive and therapeutic effects of the compound on ulcerative colitis (UC) in mice

The preventive and therapeutic effects of the positive drug ABX464 and Compound 23 of the present disclosure on dextran sulfate sodium (DSS)-induced ulcerative colitis were evaluated using a DSS-induced ulcerative colitis (UC) model.

Female C57BL/6 mice (20-22 g) were randomly divided into 7 groups according to body weight after adaptive feeding, as shown in Table 4. On day 1, the mice were switched to 3% DSS feeding. After 10 days of DSS feeding, they were switched back to normal drinking water until day 13. From day 1 to day 13, the mice were continuously administered the corresponding solvents and drugs by gavage. Body weight changes were recorded daily from day 1 to day 13. On day 13, after recording body weight, the mice were dissected to measure colon length. The preventive and therapeutic effects of the test drug on ulcerative colitis in mice were evaluated based on relative body weight change rate and colon length.

**Table 4. Experimental animal grouping and model induction period**

| **Group** | **Modeling** | **Number** | **Administration dose** |
|---|---|---|---|
| Control group | 13-day water | 12 | Solvent (qd) |
| DSS model group | 10 days 3% DSS + 3 days water | 12 | Solvent (qd) |
| Positive drug group | 10 days 3% DSS + 3 days water | 13 | ABX46450mg/ kg(qd) |
| Test drug group 1 | 10 days 3% DSS + 3 days water | 12 | Compound **23** of the present disclosure 6.25 mg/kg (qd) |
| Test drug group 2 | 10 days 3% DSS + 3 days water | 13 | Compound **23** of the present disclosure 12.5 mg/kg (qd) |
| Test drug group 3 | 10 days 3% DSS + 3 days water | 13 | Compound **23** of the present disclosure 25 mg/kg (qd) |
| Test drug group 4 | 10 days 3% DSS + 3 days water | 13 | Compound **23** of the present disclosure 50 mg/kg (qd) |

| | | | |
|---|---|---|---|
| Note: Solvent 10% DMSO + 10% castor oil + 80% saline | | | |

(1) Body weight experimental results showed: compared with the normal control group, the DSS model group began to exhibit significant weight loss from day 8 (p<0.0001), with the extent of weight loss progressively increasing, reaching a 14% reduction by day 10 (p<0.0001); compared with the DSS model group, the test drug group **4** at 50 mg/kg dose did not show sustained weight loss from day 8, with a weight loss of 3.09% by day 9 (p=0.0004) in the test drug group at 50 mg/kg; compared with the DSS model group, all administration groups showed significant weight recovery by day 10, with weight loss of 6.02% (p=0.0001) for the ABX464 positive drug group, and 7.52% (p=0.0046), 8.99% (p=0.0498), 7.96% (p=0.0087), and 3.8% (p<0.0001) for the test drug groups at 6.25, 12.5, 25, and 50 mg/kg doses, respectively. The extent of weight recovery at the experimental endpoint was as follows: the test drug group **4** at 50 mg/kg > ABX464 at 50 mg/kg, while the low- and medium-dose test drug groups showed comparable weight recovery effects to the positive drug ABX464 (details in Table 5). These results indicate that the compound **23** of the present disclosure exhibits a favorable restorative effect on weight loss in DSS-induced ulcerative colitis mice.
(2) Colon length results showed that compared with the normal control group, the DSS model group exhibited significantly shortened colon length (p<0.0001), being only 72.3% of the control group; compared with the DSS model group, all administration groups demonstrated significantly increased colon length, wherein the positive drug group and the test drug groups at different doses of 6.25, 12.5, 25, and 50 mg/kg showed colon lengths of 96.5% (p<0.0001), 89.79% (p=0.0002), 98.1% (p<0.0001), 90.9% (p<0.0001), and 103.3% (p<0.0001) of the control group, respectively (details in Table 3). The above results indicate that the compound **23** of the present disclosure has a good therapeutic effect on colon shortening in DSS-induced ulcerative colitis mice.

**Table 5. Percentage change in relative body weight of C57BL/6 mice (%)**

| Adminis tration cycle (days) | Control group | Model group | ABX464 50mg/kg | Compound **23** | | | |
|---|---|---|---|---|---|---|---|
| | | | | 6.25mg/kg | 12.5mg/kg | 25mg/kg | 50mg/kg |
| 1 | 100.0± 0.00 | 100±0.00 | 100±0.00 | 100±0.00 | 100±0.00 | 100±0.00 | 100±0.00 |
| 2 | 100.9± 0.59 | 103.0± 0.57 | 101.6± 0.45 | 101.5± 0.43 | 99.9±0.63 | 100.5± 0.37 | 99.1±0.65 |
| 3 | 100.9± 0.66 | 104.5± 1.00 | 101.9± 0.47 | 102.4± 0.78 | 100.8± 0.67 | 101.1± 0.59 | 100.2±0.78 |
| 4 | 103.0± 0.63 | 104.6± 1.19 | 101.9± 0.62 | 101.9± 1.02 | 99.9+0.87 | 100.8± 0.44 | 100.0±0.74 |
| 5 | 103.4± 0.73 | 103.7± 0.74 | 101.1± 0.82 | 101.7± 0.98 | 100.5± 0.88 | 99.8±0.61 | 100.7±1.01 |
| 6 | 102.8± 0.72 | 101.8± 0.82 | 98.5±0.79 | 100±0.97 | 97.4±0.87 | 97.5±0.81 | 98.0±1.15 |
| 7 | 102.6± 0.74 | 98.4±1.22 | 95.6±1.09 | 96.4±1.18 | 95.0±1.12 | 95.3±0.98 | 96.9±1.03 |
| 8 | 103.1± 0.50 | 93.2± 1.68#### | 93.5± 1.35 | 94.4±1.50 | 92.7±1.28 | 93.7±1.43 | 96.7±1.20 |
| 9 | 104.8± 0.60 | 89.9± 1.79#### | 93.0±1.33 | 93.4±1.75 | 92.7±1.55 | 92.6±2.25 | 96.9± 1.41*** |
| 10 | 103.4± 0.69 | 86.4± 2.13#### | 94.0± 1.27*** | 92.5± 2.02** | 91.0± 1.78* | 92.0± 2.38** | 96.2± 1.38**** |
| 11 | 102.9± 0.87 | 87.8± 2.48#### | 92.4± 2.22** | 92.4±2.22 | 91.9±1.91 | 94.6± 1.73** | 96.6± 1.45**** |
| 12 | 102.3± 0.72 | 89.6± 1.63#### | 92.7±2.19 | 92.7±2.19 | 92.1±1.17 | 93.5±1.57 | 96.5± 1.62** |
| 13 | 102.2± 0.68 | 90.5± 2.04^{####} | 92.0±2.52 | 92.0±2.52 | 92.1±1.17 | 95.3±1.63 | 96.5± 1.62** |

| | | | | | | | |
|---|---|---|---|---|---|---|---|
| Note: Data in the table are expressed as mean ± SEM; * indicates P<0.05 compared with the model group animals, ** indicates P<0.01 compared with the model group animals, *** indicates P<0.001 compared with the model group animals, **** indicates P<0.0001 compared with the model group animals, #### indicates P<0.0001 compared with the control group animals. | | | | | | | |

**Table 6. C57BL/6 mouse colon length**

| Grouping | Colon length | | Relative colon length (%) |
|---|---|---|---|
| | Mean | SEM | |
| Control group | 8.00 | 0.153 | 100 |
| Model group | 5.94 | 0.179 | 74.2**** |
| ABX46450mg/ kg | 7.72 | 0.153 | 96.5#### |
| Compound **23** 6.25 mg/kg | 7.18 | 0.194 | 89.8### |
| Compound **23** 12.5 mg/kg | 7.85 | 0.314 | 98.1#### |
| Compound **23** 25 mg/kg | 7.28 | 0.135 | 90.9#### |
| Compound **23** 50 mg/kg | 8.27 | 0.132 | 103.4*####* |

| | | | |
|---|---|---|---|
| Note: **** indicates P<0.0001 compared with the control group animals, ### indicates P<0.001 compared with the model group animals, #### indicates P<0.0001 compared with the model group animals. | | | |

### Test 3: Pharmacokinetic study

(1) SD male rats were used as test animals. The drug concentrations in plasma at different time points after gavage administration of ABX464 and compounds **23, 44,** and **45,** as well as after intravenous administration of test compounds 44 and 45, were determined using liquid chromatography-tandem mass spectrometry (LC-MS/MS). The pharmacokinetic characteristics of compounds **23, 44,** and **45** in rats were studied.

SD male rats (200-220 g) were administered with ABX464 and compound **23** at a dose of 20.0 mg/kg by gavage, and compounds 44 and 45 at doses of 25 mg/kg and 1 mg/kg by gavage and intravenous administration, respectively, with 3 animals per group. The administration solvent was a physiological saline solution containing 10% DMSO + 10% polyoxyl castor oil (Cremophor EL). Fasting was maintained for approximately 12 hours prior to administration, and free feeding was allowed 4 hours post-administration, with no water restriction. Before administration and 5 min, 15 min, 30 min, 1 h, 2 h, 4 h, 6 h, 8 h, and 24 h after administration, 0.2 mL of blood was collected from the jugular vein and placed in an EDTA-K2 anticoagulant tube containing a label. After gently inverting the tube to fully mix the anticoagulant EDTA-K2 with the blood, the tube was immediately placed on wet ice. The plasma was separated by centrifugation immediately after blood collection under the following centrifugation conditions: 4°C, 3500 rpm, and 5 min. The upper plasma layer was transferred and stored at -80°C until sample analysis. Quantitative analysis of the concentration of compound **23** in plasma was performed using LC-MS/MS. The pharmacokinetic parameters were calculated from the sample analysis results using WinNonlin software.

**Table 7. Pharmacokinetic parameters**

| Parameter | Unit | Compound **23** | Compound **44** | | Compound **45** | | ABX464 |
|---|---|---|---|---|---|---|---|
| | | p.o. 20 mg/kg | i.v. 1mg/kg | p.o. 25 mg/kg | i.v. 1mg/kg | p.o. 25 mg/kg | p.o. 20 mg/kg |
| T_{1/2} | h | 2.65 | 15.1 | 8.7 | 2.2 | 5.4 | 0.86 |
| Tₘₐₓ | h | 2 | - | 6 | - | 2 | 2 |
| Cₘₐₓ | ng/mL | 1853.3 | - | 389 | - | 2547 | 727.0 |
| MRT_{inf} | h | 2.2 | 8.9 | 8.2 | 1.5 | 3.2 | 2.73 |
| AUC₀₋ₜ | h*ng/mL | 5059.4 | 650 | 5573 | 1178 | 8400 | 2309.01 |
| F | % | - | - | 34.3 | - | 27.6 | - |

, pharmacokinetic results showed: After gavage administration of 20 mg/kg test compound and ABX464 to SD male rats, the Cₘₐₓ values of compound **23** and ABX464 were 1853.3 ng/mL and 727.0 ng/mL, respectively, and the AUC₀₋ₜ values were 5059.4 h*ng/mL and 2309.01 h*ng/mL, respectively, with terminal elimination half-lives of 2.65 h and 0.86 h, respectively. SD rats were administered by gavage with test compounds **44** and **45** at 25 mg/kg, respectively. The Cmax values of compounds **44** and **45** were 389 ng/mL and 2547 ng/mL, respectively, AUC0-t values were 5573 h*ng/mL and 8400 h*ng/mL, respectively, terminal elimination half-lives were 8.7 h and 5.4 h, respectively, and absolute bioavailability values were 34.3% and 27.6%, respectively. As shown in Table 7, at the same dose, compared with ABX464, compound **23** exhibited a longer elimination half-life, higher systemic exposure (Cₘₐₓ and AUC₀₋ₜ), and favorable pharmacokinetic properties; compared with the positive compound ABX464, compound **45** showed a longer half-life and higher systemic exposure (Cmax and AUC0-t); compound **44** exhibited a relatively long half-life, and the test compounds demonstrated favorable pharmacokinetic properties.

(2) Using beagle dogs as test animals, the drug concentration in plasma at different time points after gavage and intravenous administration of test compound **45** was determined by liquid chromatography-tandem mass spectrometry (LC-MS/MS). The pharmacokinetic characteristics of test compound **45** was investigated in beagle dogs.

Beagle dogs, male (9-12 kg), 3 dogs per group, were administered with the test drug **45** at doses of 6 mg/kg (by gavage) and 1 mg/kg (intravenous), respectively, with the vehicle being a physiological saline solution containing 10% DMSO + 10% polyoxyl castor oil (Cremophor EL). The gavage group was fasted for no less than 12 hours before administration, with free access to water. After 4 h of administration, the dogs were fed uniformly. Before administration and 5 min, 15 min, 30 min, 1 h, 2 h, 4 h, 8 h, 10 h, and 24 h after administration, 1 mL of blood was collected from the jugular vein and placed in an EDTA-2K anticoagulant tube with a label. After gently inverting the tube to fully mix the anticoagulant EDTA-2K with the blood, the tube was immediately placed on wet ice. The plasma was separated by centrifugation 1 h after blood collection under the following centrifugation conditions: 4°C, 6800g, and 6 min. The upper plasma layer was transferred and stored at -20°C until sample analysis. The concentration of the test compound in dog plasma was determined using the LC-MS/MS method. Pharmacokinetic parameters were fitted using WinNonlin software, and absolute bioavailability was calculated. The results are shown in Table 8.

**Table 6. Pharmacokinetic parameters of compounds in beagle dogs**

| Parameter | Unit | Compound **45** | |
|---|---|---|---|
| | | i.v. 1mg/kg | p.o. 6 mg/kg |
| T_{1/2} | h | 8.88 | 7.18 |
| Tₘₐₓ | h | - | 2 |
| Cₘₐₓ | ng/mL | - | 204 |
| MRT_{inf} | h | 3.56 | 5.9 |
| AUC₀₋ₜ | h*ng/mL | 1565 | 990 |
| F | % | - | 10.7 |

The pharmacokinetic results showed that after gavage administration of 6 mg/kg of the test compound 45 to beagle dogs, the Cmax and AUC0-t of compound 45 were 204 ng/mL and 990 h*ng/mL, respectively, with a terminal elimination half-life of 7.18 h and an absolute bioavailability of 10.7%.

## Claims

1. A compound of formula (I), or a pharmaceutically acceptable salt thereof, 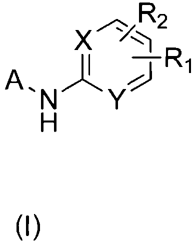
wherein X and Y are independently selected from CH or N;
A is selected from cyano, hydroxyl, carboxyl, optionally substituted C1-C6 alkyl, optionally substituted C2-C6 alkenyl, optionally substituted C2-C6 alkynyl, optionally substituted C1-C6 alkoxy, optionally substituted C3-C6 cycloalkyl, and optionally substituted aromatic ring; substituents of the optionally substituted C1-C6 alkyl, C2-C6 alkenyl, C2-C6 alkynyl, C1-C6 alkoxy, C3-C6 cycloalkyl, and aromatic ring are independently selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr¹;
HetAr¹ is optionally substituted 4- to 10-membered heterocycle or 5- to 10-membered aromatic ring having 1 to 3 heteroatoms independently selected from N, O, or S;
R₁ and R₂ are independently selected from hydrogen, halogen, hydroxyl, carboxyl, COOR, optionally substituted C1-C6 alkyl, optionally substituted C1-C6 alkoxy, optionally substituted C3-C6 cycloalkyl, and optionally substituted C1-C6 alkylamino; substituents of the C1-C6 alkyl, C1-C6 alkoxy, C3-C6 cycloalkyl, and C1-C6 alkylamino are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, and C1-C6 haloalkylamino;
alternatively, R₁ and R₂ together with the carbon atom to which they are attached further form optionally substituted saturated 5- to 6-membered heterocycle, wherein the 5- to 6-membered heterocycle optionally contains a heteroatom selected from N and O, and substituents of the optionally substituted saturated 5- to 6-membered heterocycle are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, and C1-C6 haloalkylamino;
alternatively, R₁ and R₂ together with the carbon atom to which they are attached further form optionally substituted 5- to 6-membered aromatic ring; wherein substituents are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, and C1-C6 haloalkylamino;
the following compounds **a-x** are excluded: 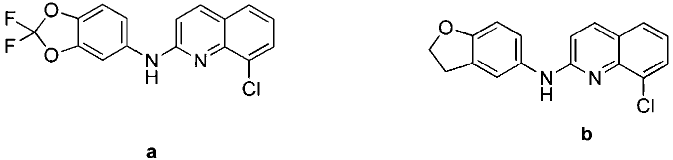 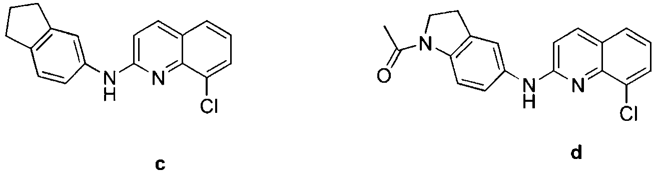 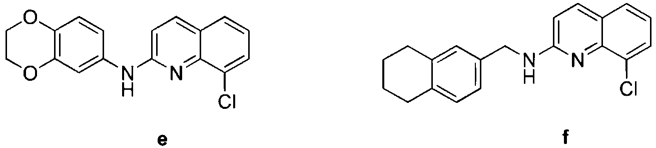 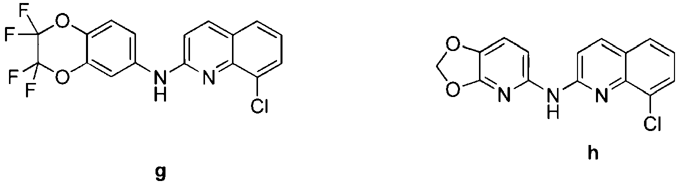 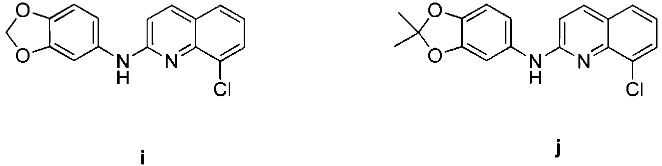 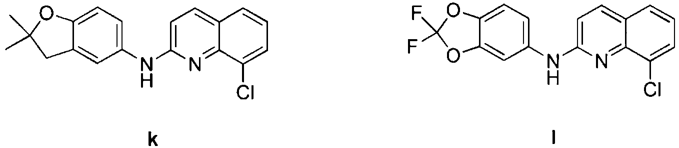 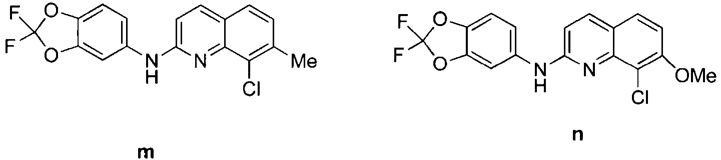 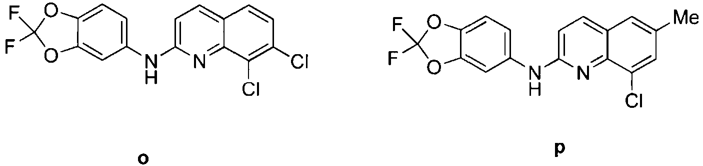 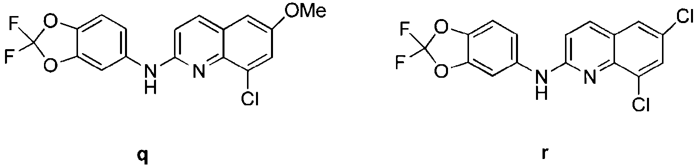 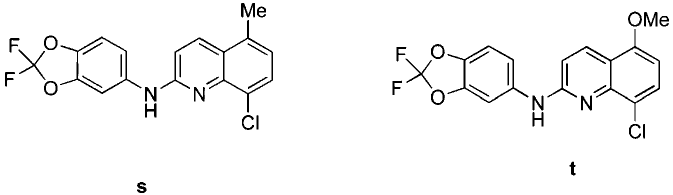 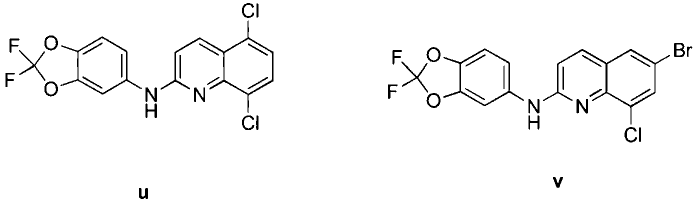 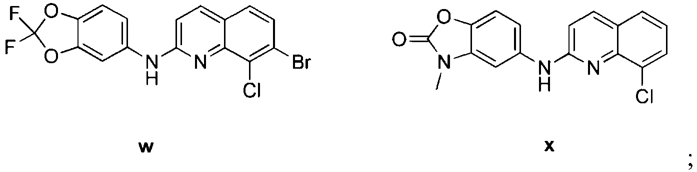
preferably, in the compound of formula (I), X is CH; Y is N; A is C2-C6 alkenyl; R1 and R2 together with the carbon atom to which they are attached further form optionally substituted 5- to 6-membered aromatic ring, and are connected to a adjacent benzene ring to form a fused ring; wherein a substituent is halogen; more preferably, R1 and R2 together with the carbon atom to which they are attached form a benzene ring, and are connected to a adjacent benzene ring to form a fused ring; wherein a substituent is halogen.

2. A compound of formula (I-1), or a pharmaceutically acceptable salt thereof, 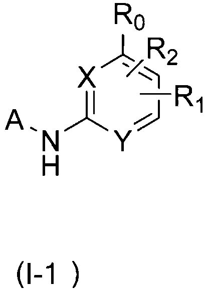
wherein X and Y are independently selected from CH or N;
A is selected from cyano, hydroxyl, carboxyl, optionally substituted C1-C6 alkyl, optionally substituted C2-C6 alkenyl, optionally substituted C2-C6 alkynyl, optionally substituted C1-C6 alkoxy, optionally substituted C3-C6 cycloalkyl, and optionally substituted aromatic ring; substituents of the optionally substituted C1-C6 alkyl, C2-C6 alkenyl, C2-C6 alkynyl, C1-C6 alkoxy, C3-C6 cycloalkyl, and aromatic ring are independently selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr¹;
HetAr¹ is optionally substituted 4- to 10-membered heterocycle or 5- to 10-membered aromatic ring having 1 to 3 heteroatoms independently selected from N, O, or S; wherein substituents are selected from hydrogen, halogen, cyano, hydroxyl, carboxyl, C1-C6 alkyl, C1-C6 haloalkyl, C1-C6 alkoxy, C1-C6 haloalkoxy, C1-C6 alkylthio, C1-C6 haloalkylthio, and C1-C6 hydroxyalkyl;
R₀ is H or 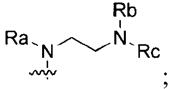
R₁ and R₂ together with the carbon atom to which they are attached further form optionally substituted 5- to 6-membered halogenated aromatic ring; the halogen in the halogenated aromatic ring is independently selected from F, Cl, Br; wherein substituents are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, C1-C6 haloalkylamino, HetAr⁴, and 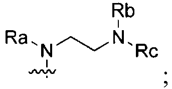
HetAr⁴ is optionally substituted 4- to 10-membered heterocycle or 5- to 10-membered aromatic ring having 1 to 3 heteroatoms independently selected from N, O, or S; wherein substituents are selected from hydrogen, halogen, cyano, hydroxyl, carboxyl, C1-C6 alkyl, C1-C6 haloalkyl, C1-C6 alkoxy, C1-C6 haloalkoxy, C1-C6 alkylthio, C1-C6 haloalkylthio, and C1-C6 hydroxyalkyl;
Rₐ is selected from hydrogen, halogen, hydroxyl, carboxyl, C1-C6 alkyl, C1-C6 haloalkyl, and R_{b} and R_{c} are independently selected from hydrogen, halogen, hydroxyl, carboxyl, C1-C6 alkyl, and C1-C6 haloalkyl;
alternatively, R_{b} and R_{c} together with the nitrogen atom to which they are attached further form optionally substituted saturated 5- to 6-membered heterocycle, wherein the 5- to 6-membered heterocycle optionally contains a heteroatom selected from N and O, and substituents of the optionally substituted saturated 5- to 6-membered heterocycle are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, and C1-C6 haloalkylamino;
the following compounds **a-x** are excluded:

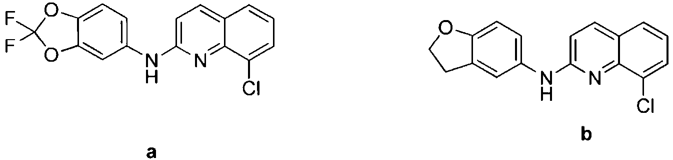

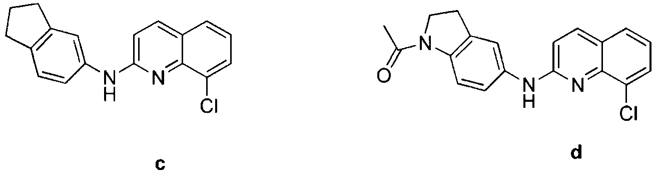

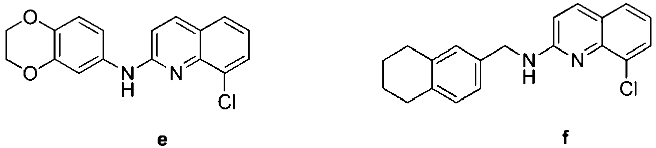

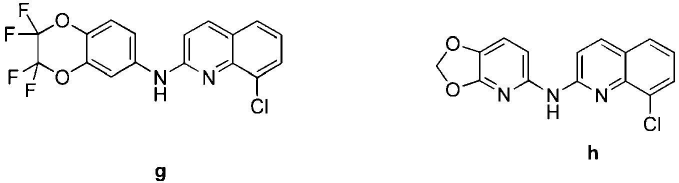

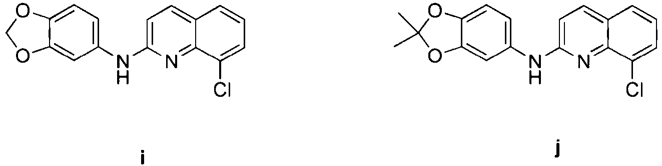

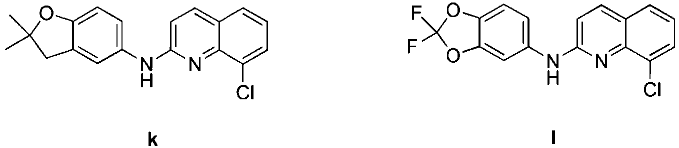

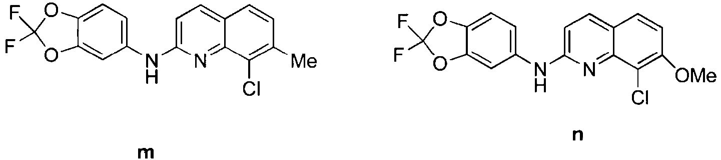

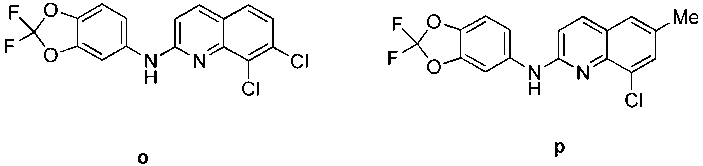

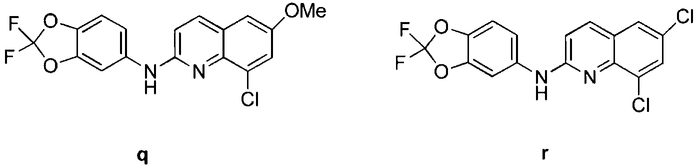

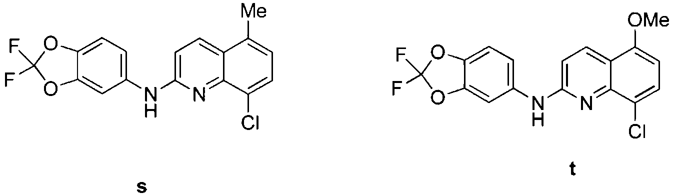

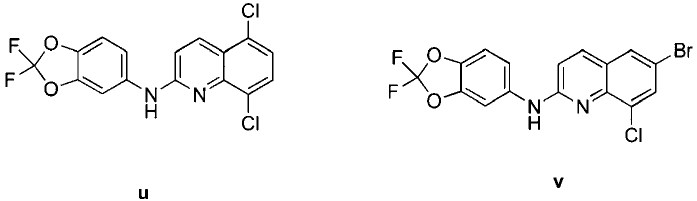

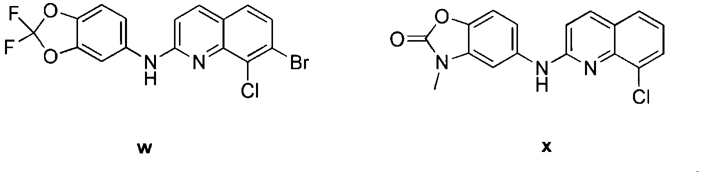

3. A compound of formula (II) or a pharmaceutically acceptable salt thereof, 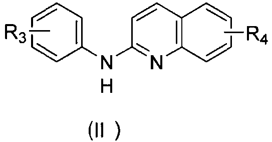
wherein R₃ is selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr²; R is C1-C6 alkyl;
HetAr² is substituted or unsubstituted 4- to 6-membered monocyclic heterocycle having 1 to 3 heteroatoms independently selected from N, O, or S; and is connected to a adjacent benzene ring to form a fused ring; wherein substituents are selected from hydrogen, halogen, cyano, hydroxyl, carboxyl, C1-C6 alkyl, C1-C6 haloalkyl, C1-C6 alkoxy, C1-C6 haloalkoxy, C1-C6 alkylthio, C1-C6 haloalkylthio, and C1-C6 hydroxyalkyl;
R₄ is selected from hydrogen, halogen, cyano, hydroxyl, carboxyl, COOR, C1-C6 alkyl, C1-C6 haloalkyl, C1-C6 alkoxy, C1-C6 haloalkoxy; R is C1-C6 alkyl;
the following compounds **A-U** are excluded: 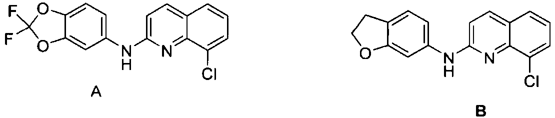 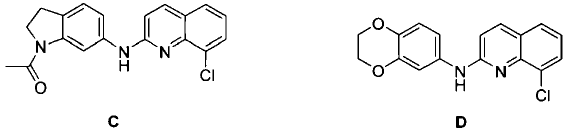 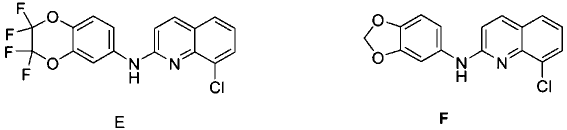 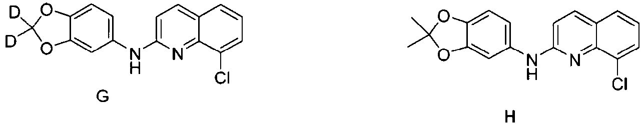 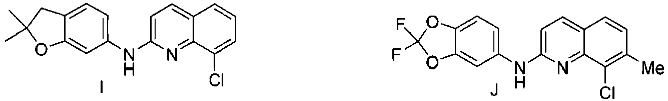 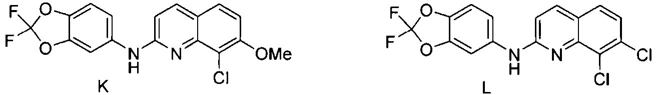 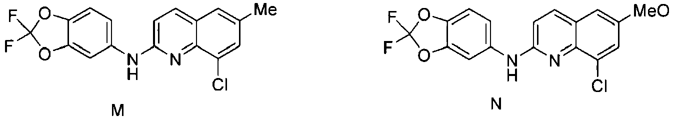 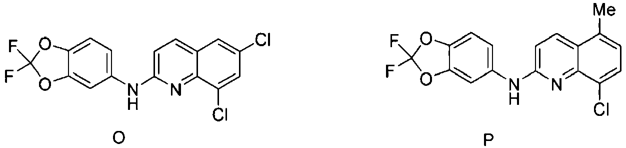 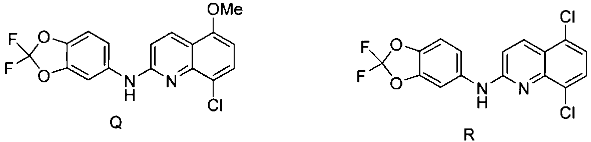 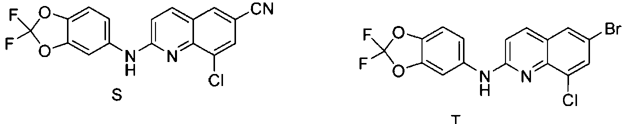 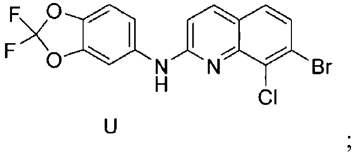
preferably, in the compound of formula (II), R₃ is selected from HetAr²; HetAr² is substituted or unsubstituted 4- to 6-membered monocyclic aromatic heterocycle having 1 to 3 heteroatoms independently selected from N, O, or S; and is connected to a adjacent benzene ring to form a fused ring; wherein substituents are selected from hydrogen, halogen, cyano, hydroxyl, carboxyl, C1-C6 alkyl, C1-C6 haloalkyl, C1-C6 alkoxy, C1-C6 haloalkoxy, C1-C6 alkylthio, C1-C6 haloalkylthio, and C1-C6 hydroxyalkyl; further substituents are selected from hydrogen, halogen, cyano, hydroxyl, carboxyl, C1-C3 alkyl, C1-C3 haloalkyl, C1-C3 alkoxy, C1-C3 haloalkoxy, C1-C3 alkylthio, C1-C3 haloalkylthio, and C1-C3 hydroxyalkyl; R₄ is halogen;
more preferably, in the compound of formula (II), R₃ is selected from HetAr²; HetAr² is substituted or unsubstituted 4- to 6-membered monocyclic saturated heterocycle containing one O; and is connected to a adjacent benzene ring to form a fused ring; wherein substituents are selected from hydrogen, halogen, cyano, hydroxyl, and carboxyl; R₄ is halogen.

4. A compound of formula (III) or a pharmaceutically acceptable salt thereof: 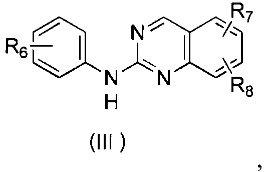
wherein R₆ is selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr³; R is C1-C6 alkyl;
HetAr³ is substituted or unsubstituted 4- to 6-membered monocyclic heterocycle having 1 to 3 heteroatoms independently selected from N, O, or S; and is connected to a adjacent benzene ring to form a fused ring; wherein substituents are selected from hydrogen, halogen, hydroxyl, and carboxyl;
R₇ and R₈ are independently selected from hydrogen, halogen, hydroxyl, carboxyl, COOR, C1-C6 alkyl, C1-C6 haloalkyl, C1-C6 alkoxy, and C1-C6 haloalkoxy; R is C1-C6 alkyl.

5. The compound or the pharmaceutically acceptable salt thereof according to claim 3, wherein R₆ is selected from C1-C6 haloalkoxy or C1-C6 haloalkylthio; R₇ and R₈ are independently selected from hydrogen, halogen, C1-C6 haloalkyl, and C1-C6 alkoxy; preferably, R₆ is selected from C1-C3 haloalkoxy or C1-C3 haloalkylthio; R₇ and R₈ are independently selected from hydrogen, halogen, C1-C3 haloalkyl, and C1-C3 alkoxy; more preferably, R₇ and R₈ are not both hydrogen, or preferably R₆ is selected from HetAr1, which is substituted or unsubstituted 4- to 6-membered monocyclic heterocycle having 1 to 3 heteroatoms independently selected from N, O, or S; and is connected to a adjacent benzene ring to form a fused ring; wherein substituents are selected from hydrogen and halogen; R₇ and R₈ are independently selected from hydrogen and halogen; more preferably, R₇ and R₈ are not both hydrogen.

6. A compound of formula (III-1) or a pharmaceutically acceptable salt thereof: 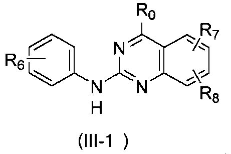
wherein R₆ is selected from hydrogen, halogen, cyano, hydroxyl, carboxyl, COOR, C1-C6 alkyl, C1-C6 haloalkyl, C1-C6 alkoxy, C1-C6 haloalkoxy, C1-C6 alkylthio, C1-C6 haloalkylthio, C1-C6 alkylamino, C1-C6 haloalkylamino, and HetAr³; R is C1-C6 alkyl;
HetAr³ is substituted or unsubstituted 4- to 6-membered monocyclic heterocycle having 1 to 3 heteroatoms independently selected from N, O, or S; and is connected to a adjacent benzene ring to form a fused ring; wherein substituents are selected from hydrogen, halogen, hydroxyl, and carboxyl;
R₀ is H or 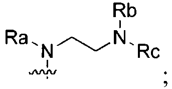
R₇ and R₈ are independently selected from halogen, C1-C6 haloalkylamino, HetAr⁴, and 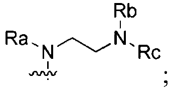
HetAr⁴ is optionally substituted 4- to 10-membered heterocycle or 5- to 10-membered aromatic ring having 1 to 3 heteroatoms independently selected from N, O, or S;
Rₐ is selected from hydrogen, halogen, hydroxyl, carboxyl, C1-C6 alkyl, C1-C6 haloalkyl; R_{b} and R_{c} are independently selected from hydrogen, halogen, hydroxyl, carboxyl, C1-C6 alkyl, and C1-C6 haloalkyl; or R_{b} and R_{c} together with the nitrogen atom to which they are attached further form optionally substituted saturated 5- to 6-membered heterocycle, wherein the 5- to 6-membered heterocycle optionally contains a heteroatom selected from N and O, and substituents of the optionally substituted saturated 5- to 6-membered heterocycle are independently selected from hydrogen, halogen, hydroxyl, carboxyl, COOR, C1-C6 alkyl, C1-C6 haloalkyl, C1-C6 alkoxy, C1-C6 haloalkoxy, C1-C6 alkylamino, and C1-C6 haloalkylamino.

7. The compound or the pharmaceutically acceptable salt thereof according to claim 6, wherein R₆ is selected from C1-C6 haloalkoxy or C1-C6 haloalkylthio; preferably, R₆ is selected from C1-C3 haloalkoxy or C1-C3 haloalkylthio; more preferably, R₆ is trifluoromethyloxy; preferably, R₇ is selected from F, Cl or Br; preferably, R₀ is H; preferably, R₇ is halogen; R₈ is selected from HetAr⁴ or 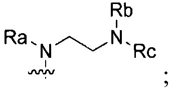 preferably, HetAr⁴ is optionally substituted 4- to 10-membered heterocycle having 1 to 3 heteroatoms independently selected from N, O or S, preferably substituents of HetAr⁴ is selected from hydrogen, halogen, hydroxyl, carboxyl, methyl, ethyl, propyl, butyl, and C1-C3 haloalkyl; preferably, Rₐ is selected from hydrogen, halogen, hydroxyl, carboxyl, methyl, ethyl, propyl, and butyl, R_{b} and R_{c} are independently selected from hydrogen, halogen, hydroxyl, carboxyl, methyl, ethyl, propyl, and butyl; or R_{b} and R_{c} together with the nitrogen atom to which they are attached further form optionally substituted saturated 5- to 6-membered heterocycle containing N and O, wherein substituents of the 5- to 6-membered heterocycle are independently selected from hydrogen, halogen, hydroxyl, carboxyl, C1-C6 alkyl, C1-C6 haloalkyl, C1-C6 alkoxy, C1-C6 haloalkoxy, C1-C6 alkylamino, and C1-C6 haloalkylamino; preferably, R_{b} and R_{c} are independently selected from methyl, ethyl, propyl, and butyl; or R_{b} and R_{c} together with the nitrogen atom to which they are attached further form saturated 5- to 6-membered heterocycle containing N and O.

8. The compound or the pharmaceutically acceptable salt thereof, wherein the compound is selected from:

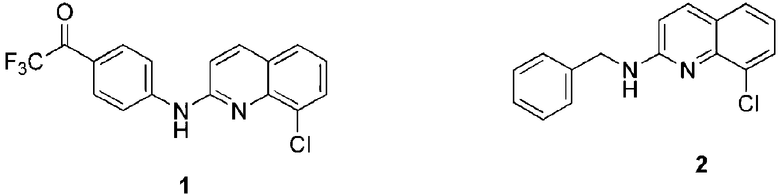

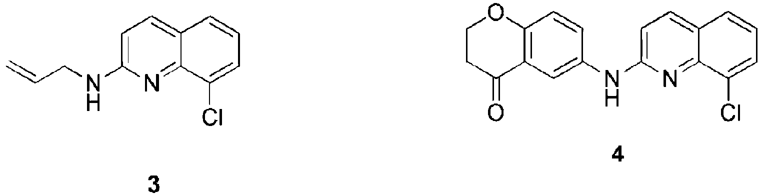

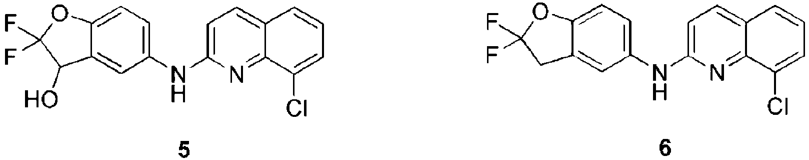

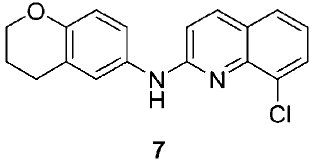

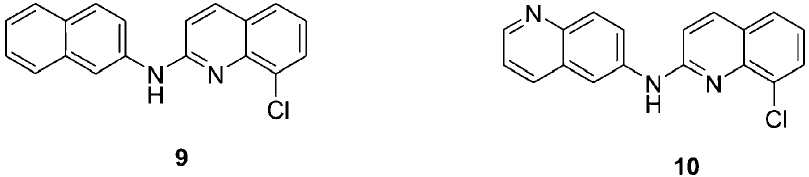

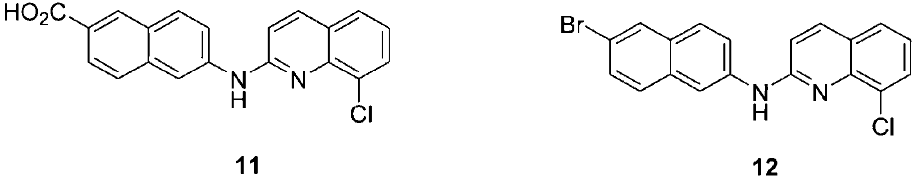

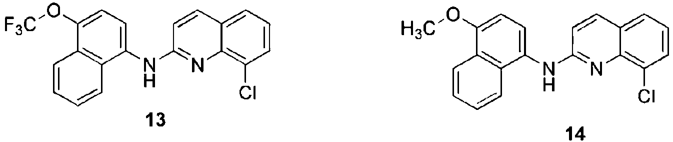

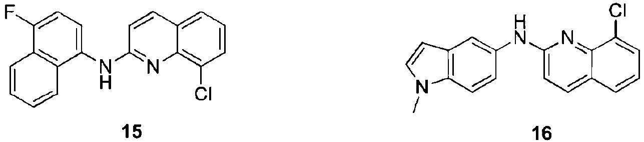

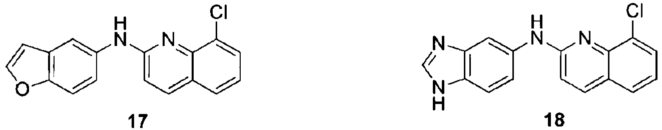

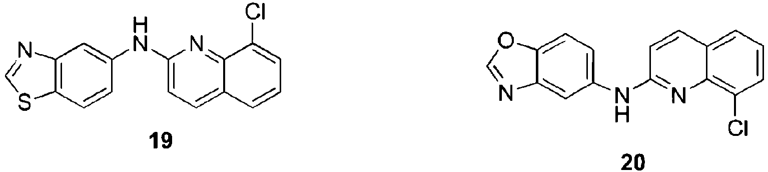

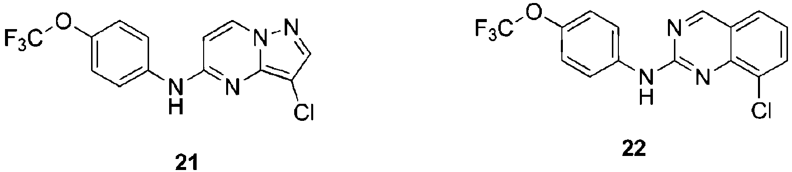

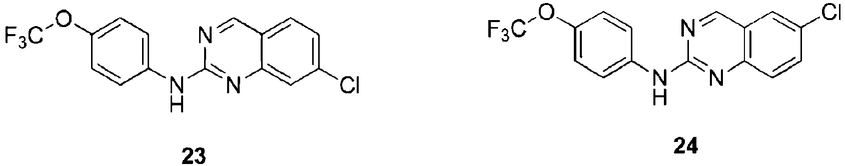

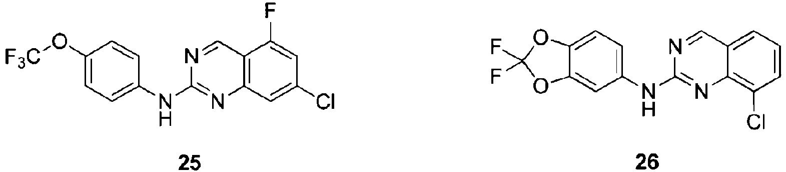

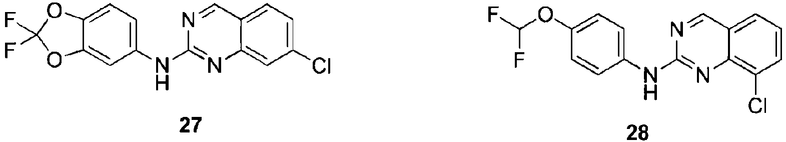

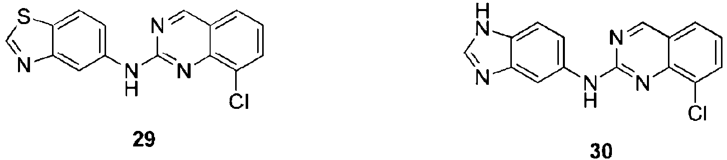

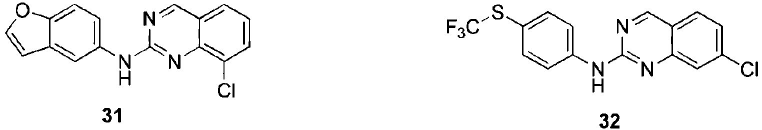

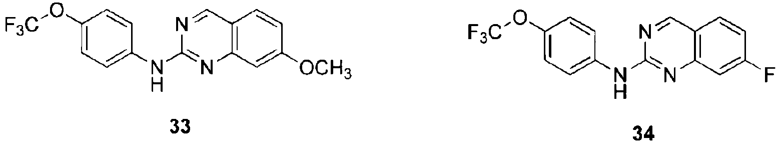

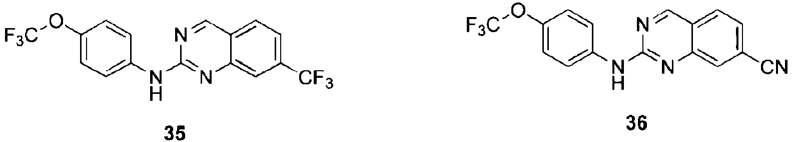

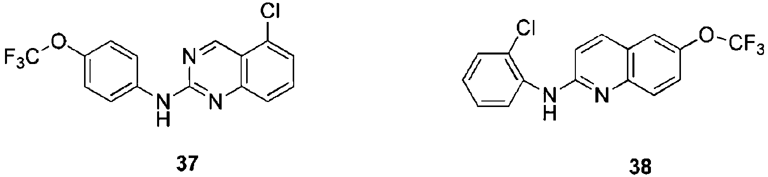

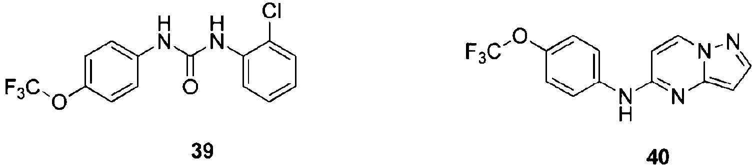

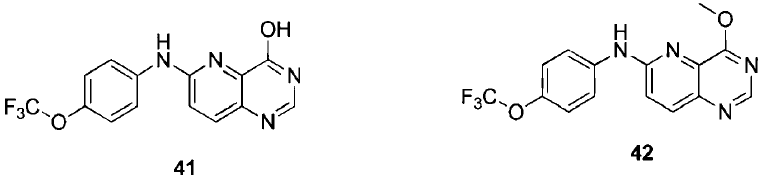

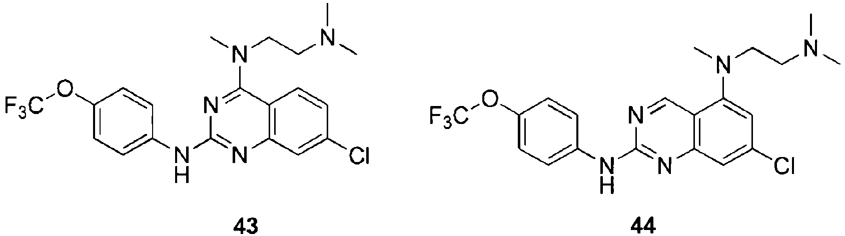

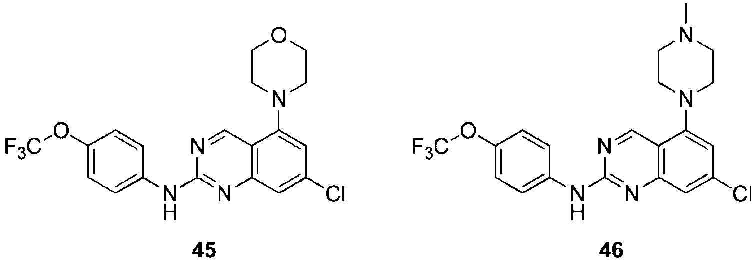

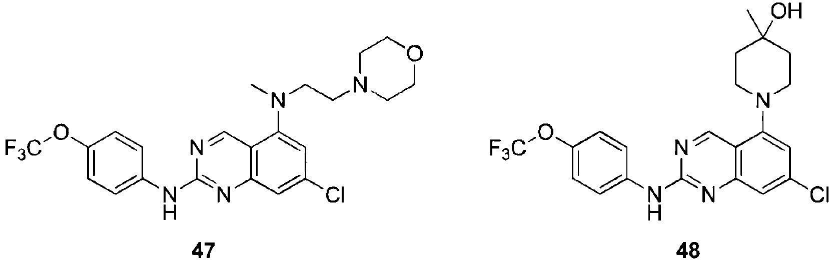

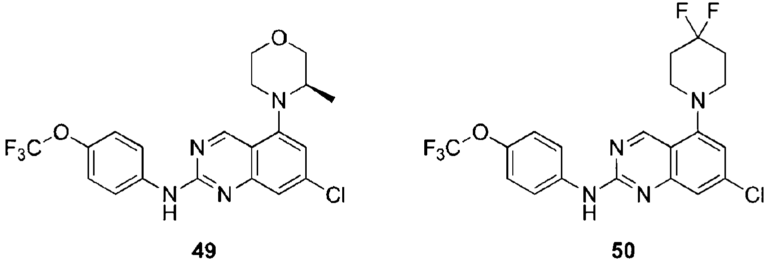

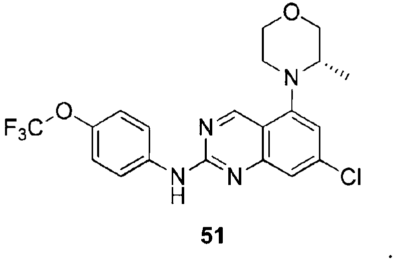

9. A method for preparing a compound, wherein the method is any one of the following (1) to (3):
(1) a method for preparing a compound of formula (I): 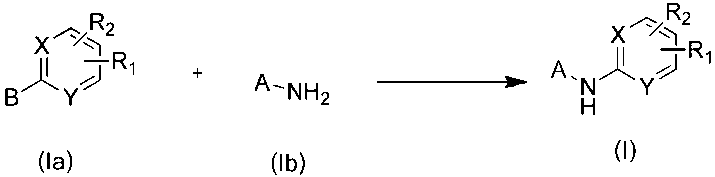 a compound of formula (Ia) or a pharmaceutically acceptable salt thereof and a compound of formula (Ib) or a pharmaceutically acceptable salt thereof undergo nucleophilic substitution to yield the compound of formula (I) or a pharmaceutically acceptable salt thereof; wherein B is halogen, preferably a chlorine atom; X, Y, R₁, and R₂ are as defined in claim 1;
(2) a method for preparing a compound of formula (II): 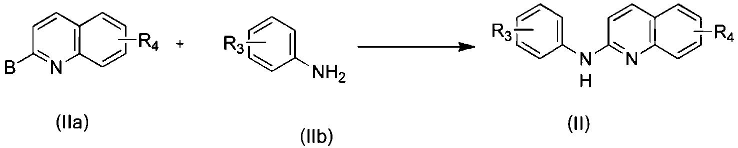 a compound of formula (IIa) or a pharmaceutically acceptable salt thereof and a compound of formula (IIb) or a pharmaceutically acceptable salt thereof undergo nucleophilic substitution to yield the compound of formula (II) or a pharmaceutically acceptable salt thereof; wherein B is halogen, preferably a chlorine atom; R₃ and R₄ are as defined in claim 2;
(3) a method for preparing a compound of formula (III): 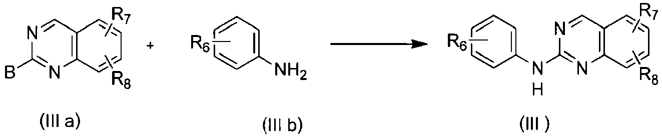
a compound of formula (IIIa) or a pharmaceutically acceptable salt thereof and a compound of formula (IIIb) or a pharmaceutically acceptable salt thereof undergo nucleophilic substitution to yield the compound of formula (III) or a pharmaceutically acceptable salt thereof; wherein B is halogen, preferably a chlorine atom; R₆, R₇, and R₈ are as defined in any one of claims 3 to 7.

10. A pharmaceutical composition comprising the compound or the pharmaceutically acceptable salt thereof according to any one of claims 1 to 8, and one or more pharmaceutically acceptable carriers.

11. Use of the compound or the pharmaceutically acceptable salt thereof according to any one of claims 1 to 8 as a microRNA-124 regulator.

12. Use of the compound or the pharmaceutically acceptable salt thereof according to any one of claims 1 to 8 in the preparation of a medicament for preventing or treating an inflammatory disease.

13. The use according to claim 12, wherein the inflammatory disease is selected from inflammatory bowel disease, rheumatoid arthritis, Crohn's disease, ulcerative colitis, multiple sclerosis, Alzheimer's disease, Parkinson's disease, osteoarthritis, atherosclerosis, ankylosing spondylitis, psoriasis, dermatitis, Sjogren's syndrome, bronchitis, asthma, and inflammation associated with colon cancer; preferably, the inflammatory disease is selected from inflammatory bowel disease, rheumatoid arthritis, Crohn's disease, ulcerative colitis, multiple sclerosis, osteoarthritis, ankylosing spondylitis, psoriasis, Sjogren's syndrome, bronchitis, and inflammation associated with colon cancer.
